(19) Europäisches Patentamt / European Patent Office / Office européen des brevets

(11) **EP 2 959 298 B1**

(12) **EUROPEAN PATENT SPECIFICATION**


(45) Date of publication and mention of the grant of the patent:
**05.09.2018 Bulletin 2018/36**

(21) Application number: **14706672.4**

(22) Date of filing: **19.02.2014**

(51) Int Cl.:
***G01N 33/574*** *(2006.01)*

(86) International application number:
**PCT/GB2014/050484**

(87) International publication number:
**WO 2014/128460 (28.08.2014 Gazette 2014/35)**


(54) **METHODS**

VERFAHREN

PROCÉDÉS


(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.02.2013 GB 201303078**

(43) Date of publication of application:
**30.12.2015 Bulletin 2015/53**



(73) Proprietors:
- **Cambridge Enterprise Limited
Cambridge
Cambridgeshire CB2 1TN (GB)**
- **United Kingdom Research and Innovation
Swindon SN2 1FL (GB)**

(72) Inventors:
- **FITZGERALD, Rebecca
Cambridge
Cambridgeshire CB2 2XZ (GB)**
- **ROSS-INNES, Caryn
Stapleford
Cambridgeshire CB22 5DT (GB)**

(74) Representative: **Script IP Limited
Turnpike House
18 Bridge Street
Frome Somerset BA11 1BB (GB)**



(56) References cited:
**WO-A2-2012/125807 US-A1- 2012 009 597**

- **CHIN-ANN J ONG: "Biomarkers in Barrett's esophagus and esophageal adenocarcinoma: Predictors of progression and prognosis", WORLD JOURNAL OF GASTROENTEROLOGY, vol. 16, no. 45, 7 December 2010 (2010-12-07), page 5669, XP055118587, ISSN: 1007-9327, DOI: 10.3748/wjg.v16.i45.5669**
- **LAO-SIRIEIX P ET AL: "Non-endoscopic screening biomarkers for Barrett's oesophagus: from microarray analysis to the clinic.", GUT NOV 2009, vol. 58, no. 11, November 2009 (2009-11), pages 1451-1459, XP009167512, ISSN: 1468-3288**
- **S. R. KADRI ET AL: "Acceptability and accuracy of a non-endoscopic screening test for Barrett's oesophagus in primary care: cohort study", BMJ, vol. 341, no. sep10 1, 25 January 2010 (2010-01-25), pages c4372-c4372, XP055118821, ISSN: 0959-8138, DOI: 10.1136/bmj.c4372**
- **TOKUMITSU Y ET AL: "Prognostic significance of polo-like kinase expression in esophageal carcinoma.", INTERNATIONAL JOURNAL OF ONCOLOGY OCT 1999, vol. 15, no. 4, October 1999 (1999-10), pages 687-692, XP008169491, ISSN: 1019-6439**
- **M. DI PIETRO ET AL: "The combination of autofluorescence endoscopy and molecular biomarkers is a novel diagnostic tool for dysplasia in Barrett's oesophagus", GUT, 10 April 2014 (2014-04-10), XP055118406, ISSN: 0017-5749, DOI: 10.1136/gutjnl-2013-305975**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

FIELD OF THE INVENTION

The invention is in the field of testing for, or aiding the detection of, surface abnormality in the scope of protection is defined by the appended claims.

BACKGROUND

Oesophageal cancer (OAC) is currently the eighth most common cancer type worldwide and its incidence has risen almost 5-fold over the past three decades.

Barrett's oesophagus is the first step in the pathway towards OAC and meta-analyses have demonstrated that Barrett's oesophagus confers a 0.12-0.5% increased risk of progression to adenocarcinoma per year. Barrett's oesophagus occurs when the normal oesophageal cells are replaced by glandular cells and this, with time, can progress to low-grade dysplasia (LGD), high-grade dysplasia (HGD) and then finally to adenocarcinoma.

Early diagnosis of OAC and/or its pre-malignant precursor Barrett's oesophagus can improve patient management and prognosis of OAC. In one known approach, the Cytosponge™ cell collection device has been developed, for example as published in WO2011/058316.

In addition, a test using TFF3 as a molecular marker has been developed by Fitzgerald *et al* as a clinical screening tool to detect Barrett's oesophagus, for example as published in US20120009597.

The first study using the Cytosponge™ (BEST1)(Kadri et al., 2010), demonstrated that the Cytosponge™ test is a feasible method of diagnosing Barrett's oesophagus in the primary care setting.

In the present system, symptomatic patients are sent for endoscopy. Endoscopy is an invasive procedure requiring highly trained clinicians. It is also an uncomfortable procedure for the patient, and can require sedation. When endoscopy is accompanied by biopsy, there is also a degree of risk to the patient undergoing the procedure. In the clinical setting, this is currently the only way of detecting Barrett's oesophagus, and/or Barrett's associated dysplasia or cancer.

Rugge et al (2010 Human Pathology vol 41 pages 1380-1386) disclose aurora kinase A (AURKA) in Barrett's carcinogenesis. It is noted that TP53 mutations are recognised as markers of an increased risk of Barrett's adenocarcinoma. Esophageal biopsy samples were obtained from long segments of Barrett's oesophagus. 9 of 10 Barrett's adenocarcinomas showed AURKA immunostaining. AURKA expression via mRNA analysis and microarray studies was examined. The authors concluded by attributing a significant role to AURKA overexpression in the progression of Barrett's mucosa to cancer. The authors concluded that further attempts were needed in larger and prospective studies to validate AURKA IHC expression as a potential prognostic marker in Barrett's mucosa patients.

Liu et al (2008 World Journal of Gastroenterology vol 14 pages 7199-7207) disclose a tissue array for Tp53, C-myc, CCND1 gene over-expression in different tumours. Seven different tumour types were examined. Analysis was nucleic acid based. Samples used were of known tumours. No detection method is taught. Samples were formalin fixed.

Agnese et al (2007 European Society for Medical Oncology vol 8 Suppl 6 vi110-vi115) disclose Aurora-A overexpression as an early marker of reflux-related columnar mucosa and Barrett's oesophagus. The authors could not find any statistically significant quantitative differences in AURKA mRNA expression between Barrett's mucosa (columnar lined oesophagus/CLO) and Barrett's oesophagus (BO) with or without dysplasia and p53 positive immunostaining.

Certain molecular markers have been studied in connection with Barrett's oesophagus. These markers have been studied in a purely research setting. These studies have been carried out on *in vitro* tissue samples. These markers have been studied singly. Currently, no such molecular markers are used in any clinical test for Barrett's associated abnormalities.

There is a need in the art for improved detection of Barrett's associated abnormalities. The prior art tests are expensive and labour-intensive, invasive and involve risks to the subject undergoing the test.

The present invention seeks to overcome problems associated with the prior art.

SUMMARY

Certain molecular markers have been shown to be associated with Barrett's associated abnormalities. These markers have been studied on tissue biopsies. Using a molecular marker on a tissue biopsy offers little practical advantage over the current clinical gold standard of morphological examination of the biopsy. This is because studying the markers in this manner still requires the biopsy to be collected, thereby still involving each of the drawbacks associated with that invasive procedure in the prior art. More importantly, the single markers which have been studied in the research setting have shown inadequate sensitivity and/or inadequate specificity to be regarded as robust markers contributing towards detection or diagnosis.

The present inventors studied a large range of candidate markers. They also studied these markers in different combinations. The present inventors have arrived at a small and defined panel of markers which, when tested in combination, yield clinically useful sensitivity and specificity scores. In addition, the inventors have studied the performance of these markers in surface sampled cells. For example, these combinations of markers can be employed in the analysis of cells collected from a surface sampling of the oesophagus, such as is obtained using cell collection devices, for example, a Cytosponge™.

The methods taught by the inventors involve novel combinations of markers which have not previously been used in clinical tests. In addition, the inventors demonstrate that these markers have application and produce reliable results when used on cells obtained from surface sampling of the oesophagus. Together, these various features of the methods of the invention provide advantages of robust and clinically useful risk assessment, coupled to advantageously avoiding the need for invasive tissue collection via biopsy. These and further advantages of the invention are described in more detail below. Disclosed is a method of aiding detection of a surface abnormality in the oesophagus of a subject, the method comprising:

a) providing a sample of cells from said subject, wherein said sample comprises cells collected from the surface of the subject's oesophagus;
b) assaying said cells for at least two markers selected from

(i) p53;
(ii) c-Myc;
(iii) AURKA or PLK1, preferably AURKA;
(iv) methylation of MyoD and Runx3; and
(v) atypia,

wherein detection of abnormal levels of at least two of said markers infers that the subject has an increased likelihood of a surface abnormality in the oesophagus. Further disclosed is a method of aiding detection of a surface abnormality in the oesophagus of a subject, wherein said surface abnormality is selected from the group consisting of low-grade dysplasia (LGD), high-grade dysplasia (HGD), asymptomatic oesophageal adenocarcinoma (OAC) and intra-mucosal cancer (IMC), the method comprising:

a) providing a sample of cells from said subject, wherein said sample comprises cells collected from the surface of the subject's oesophagus;
b) assaying said cells for at least two markers selected from

(i) p53;
(ii) c-Myc;
(iii) AURKA or PLK1, preferably AURKA; and
(iv) methylation of MyoD and Runx3;

wherein detection of abnormal levels of at least two of said markers infers that the subject has an increased likelihood of a surface abnormality in the oesophagus. Further disclosed is a method of aiding detection of a surface abnormality in the oesophagus of a subject, the method comprising:

a) providing a sample of cells from said subject, wherein said sample comprises cells collected from the surface of the subject's oesophagus;
b) assaying said cells for at least two markers selected from

(i) p53;
(ii) c-Myc;
(iii) AURKA or PLK1, preferably AURKA; and
(iv) methylation of MyoD and Runx3;

wherein detection of abnormal levels of at least two of said markers infers that the subject has an increased likelihood of a surface abnormality in the oesophagus.

The markers described herein are provided with guidance as to an absolute scoring for each marker. This has the advantage of incorporating the reference standard/comparison phase into an already analysed scoring system. However, if desired, the invention can instead be worked by comparison to reference standards eg. from healthy (having no oesophageal abnormalities) subject(s). Thus, in one aspect the disclosure provides a method of aiding detection of a surface abnormality in the oesophagus of a subject, the method comprising:

a) providing a sample of cells from said subject, wherein said sample comprises cells collected from the surface of the subject's oesophagus;

b) assaying said cells for at least two markers selected from

(i) p53;
(ii) c-Myc;
(iii) AURKA; and
(iv) methylation of MyoD and Runx3;

wherein detection of abnormal levels of at least two of said markers compared to a reference standard infers that the subject has an increased likelihood of a surface abnormality in the oesophagus.

Optionally step (b) comprises

(1) contacting said cells with reagents for detection of at least a first molecular marker selected from:

(i) p53;
(ii) c-Myc;
(iii) AURKA; and
(iv) methylation of MyoD and Runx3, and

(2) contacting said cells with reagents for detection of at least a second molecular marker selected from (i) to (iv) and/or assaying said cells for atypia.

More suitably step (b) comprises

(1) contacting said cells with reagents for detection of at least a first molecular marker selected from:

(i) p53;
(ii) c-Myc;
(iii) AURKA; and
(iv) methylation of MyoD and Runx3, and

(2) contacting said cells with reagents for detection of at least a second molecular marker selected from (i) to (iv).

Optionally said surface abnormality is selected from the group consisting of low-grade dysplasia (LGD), high-grade dysplasia (HGD), asymptomatic oesophageal adenocarcinoma (OAC) and intra-mucosal cancer (IMC). These all share the property of being 'glandular' ('columnar'). These all share the property of being 'Barrett's'. These are all dysplasia. None of these are squamous.

Suitably the invention is not concerned with squamous cell dysplasia.

Suitably the invention is not concerned with squamous cell cancer.

Suitably the surface abnormality is not a squamous cell abnormality.

Optionally said surface abnormality is selected from the group consisting of low-grade dysplasia (LGD), high-grade dysplasia (HGD), and intra-mucosal cancer (IMC). Optionally said surface abnormality is selected from the group consisting of low-grade dysplasia (LGD) and high-grade dysplasia (HGD).

Optionally said surface abnormality is selected from the group consisting of asymptomatic oesophageal adenocarcinoma (OAC) and intra-mucosal cancer (IMC).

Optionally said surface abnormality is low-grade dysplasia (LGD).

Optionally said surface abnormality is high-grade dysplasia (HGD).

Optionally said surface abnormality is asymptomatic oesophageal adenocarcinoma (OAC).

Optionally said surface abnormality is intra-mucosal cancer (IMC).

Optionally abnormal levels of at least three of said markers are assayed.

Optionally abnormal levels of at least four of said markers are assayed.

Optionally abnormal levels of each of said markers are assayed.

Optionally said cells are collected by unbiased sampling of the surface of the oesophagus.

Optionally said cells are collected using a capsule sponge.

Optionally the cells are prepared prior to being contacted with the reagents for detection of the molecular markers by the steps of (i) pelleting the cells by centrifuge, (ii) re-suspending the cells in plasma, and (iii) adding thrombin and incubating until a clot is formed. Optionally preparation further comprises the step of incubating said clot in formalin, processing into a paraffin block, and slicing into sections suitable for microscopic examination.

Optionally p53 is assessed by immunohistochemistry.

Optionally p53 is assessed at the nucleic acid level. Optionally p53 mutation status is assessed (e.g. detected). Optionally p53 mutations are assessed (e.g. detected) by sequencing. Suitably when p53 is detected at the nucleic acid level, 'detection of abnormal levels' means detection of a p53 mutation. In other words, detection of a p53 mutation is itself regarded as an abnormal p53 or abnormal level of p53. Assessing p53 at the nucleic acid level has the advantage of removing or ameliorating subjectivity which can be present when assessing staining levels e.g. at the protein level for p53.

Suitably p53 mutation(s) anywhere within the p53 gene are detected. This is advantageous since mutation(s) can be widespread throughout the gene. More suitably mutations in the DNA binding domain are detected. These are the most common mutations. Suitably the assay is capable of detecting mutations throughout the gene - see example 10 for more detail if further guidance is needed.

Suitably a p53 mutation is detected when a p53 nonsense mutation is detected. Suitably a p53 mutation is detected when a p53 missense mutation is detected. Suitably a p53 mutation is detected when a p53 deletion mutation is detected. Suitably a p53 mutation is detected when a p53 INDEL variant mutation is detected.
Suitably the p53 mutation is one mentioned in Example 10.
Suitably the p53 mutation is one in the DNA binding domain of p53.

Optionally p53 is assessed at both the nucleic acid and the protein level. This provides the advantage that any mutations which are not detected by protein assay are caught (E.g. p53 mutations which do not affect p53 expression/detection), and also any non-p53 changes (e.g. mutations in genes other than p53) which affect p53 expression are also caught (i.e. by the protein analysis).

Suitably p53 is assessed by detection of one or more p53 mutation(s).

Suitably p53 is assessed by immunohistochemistry and p53 is also assessed by detection of one or more p53 mutation(s).

Optionally cMyc is assessed by immunohistochemistry.

Optionally AURKA is assessed by immunohistochemistry.

It should be noted that AURKA is a preferred marker. However it will be appreciated that marker PLK1 also has a good sensitivity (91%) and a good specificity (88%). This biomarker was excluded in favour of AURKA as AURKA gave better sensitivity (93%) and specificity (94%) data (see examples). However, the inventors teach that AURKA or PLK1 overexpression detect essentially the same cases. Therefore PLK1 may be assayed instead of (or in addition to) AURKA. Thus suitably AURKA or PLK1 is assayed, preferably AURKA. Optionally methylation of MyoD/Runx3 is assessed by MethyLight analysis. Optionally atypia is assessed by scoring the cells for their morphology according to the Vienna Scale. Suitably the Vienna scale is as described in Schlemper et al 2007 Gut 2000;47:251-255.

In another aspect, the disclosure relates to a method as described above wherein step (b) of said method is preceded by the step of assaying said cells for TFF3.

In another aspect, the disclosure relates to an assay for selecting a treatment regimen, said assay comprising

a) providing a sample of cells from said subject, wherein said sample comprises cells collected from the surface of the subject's oesophagus;
b) assaying said cells for at least two markers selected from

(i) p53;
(ii) c-Myc;
(iii) AURKA; and
(iv) methylation of MyoD and Runx3;

wherein if abnormal levels of at least two of said markers are detected, then a treatment regimen of endoscopy and biopsy is selected.

In another aspect, the disclosure relates to an apparatus or system which is

(a) configured to analyse an oesophagal sample from a subject, wherein said analysis comprises
(b) assaying said cells for at least two markers selected from

(i) p53;
(ii) c-Myc;
(iii) AURKA; and
(iv) methylation of MyoD and Runx3;

said apparatus or system comprising an output module,
wherein if abnormal levels of at least two of said markers are detected, then said output module indicates an increased likelihood of a surface abnormality in the oesophagus for said subject.

In another aspect, the disclosure relates to use for applications relating to aiding detection of a surface abnormality in the oesophagus of a subject, of a material which recognises, binds to or has affinity for certain polypeptides, or methylation of certain nucleic acid sequences, wherein the polypeptides and/or nucleic acid sequences are as defined as above eg. p53, c-Myc, AURKA, methylation of Runx3/MyoD1. In another aspect, the disclosure relates to such a use of a combination of materials, each of which respectively recognises, binds to or has affinity for one or more of said polypeptide(s) or nucleic acid sequences.

In another aspect, the disclosure relates to an assay device for use in aiding detection of a surface abnormality in the oesophagus of a subject, which comprises a solid substrate having a location containing a material, which recognises, binds to or has affinity for certain polypeptides, or methylation of certain nucleic acid sequences, wherein the polypeptides and/or nucleic acid sequences are as defined above eg. p53, c-Myc, AURKA, methylation of Runx3/MyoD1.

In another aspect, the disclosure relates to a kit comprising reagents for determining the expression level of each of

(i) p53;
(ii) c-Myc;
(iii) AURKA;

in a biological sample, and optionally further comprising reagents for determining the methylation of MyoD and Runx3.

In another aspect, the disclosure relates to a method for aiding the detection of a surface abnormality in the oesophagus of a subject, the method comprising providing a sample of cells from said subject, wherein said sample comprises cells collected from the surface of the subject's oesophagus, assaying said cells for TFF3, wherein if TFF3 is detected in cell(s) of the sample, the method as described above carried out, wherein detection of abnormal levels of at least one marker in addition to detection of TFF3 indicates an increased likelihood of a surface abnormality in the oesophagus of said subject.

In another aspect, the disclosure relates to a method for aiding the detection of a surface abnormality in the oesophagus of a subject, the method comprising

(a) providing a sample of cells from said subject, wherein said sample comprises cells collected from the surface of the subject's oesophagus, assaying said cells for TFF3, wherein if TFF3 is detected in cell(s) of the sample, then the following additional steps are performed:
(b) assaying said cells for at least two markers selected from

(i) p53;
(ii) c-Myc;
(iii) AURKA; and
(iv) methylation of MyoD and Runx3;

wherein detection of abnormal levels of at least one marker in addition to detection of TFF3 indicates an increased likelihood of a surface abnormality in the oesophagus of said subject. Optionally detection of abnormal levels of at least two markers in addition to detection of TFF3, preferably least three markers in addition to detection of TFF3, preferably least four markers in addition to detection of TFF3, preferably each of the markers in addition to detection of TFF3, indicates an increased likelihood of a surface abnormality in the oesophagus of said subject. Optionally said cells are collected by unbiased sampling of the surface of the oesophagus. Optionally said cells are collected using a capsule sponge.

In another aspect, the disclosure relates to a method of collecting information useful for detecting oesophageal abnormalities comprising carrying out the steps as described above.
In another aspect, the disclosure relates to a method of collecting information useful for aiding diagnosis of oesophageal abnormalities comprising carrying out the steps as described above.
In another aspect, the disclosure relates to a method of diagnosis of oesophageal abnormalities comprising carrying out the steps as described above.
In another aspect, the disclosure relates to a method of aiding diagnosis of oesophageal abnormalities comprising carrying out the steps as described above.
In another aspect, the disclosure relates to a method of assessing the risk of oesophageal abnormalities comprising carrying out the steps as described above. In another aspect, the disclosure relates to a method of assessing the risk of an oesophageal abnormality comprising carrying out the steps as described above.

Optionally said abnormality is dysplasia. Optionally said abnormality is LGD, HGD, IMC or asymptomatic OAC.

In another aspect, the disclosure relates to a method for aiding the detection of a surface abnormality in the oesophagus of a subject, wherein said surface abnormality is oesophageal adenocarcinoma (OAC), the method comprising providing a sample of cells from said subject, wherein said sample comprises cells collected from the surface of the subject's oesophagus, assaying said cells for SMAD4, wherein if SMAD4 is detected in cell(s) of the sample an increased likelihood of oesophageal adenocarcinoma (OAC) in the oesophagus of said subject is indicated.

DETAILED DESCRIPTION OF THE INVENTION

The invention finds particular application in the assessment of the risk of a subject having dysplasia. Currently the assessment of dysplasia is only performed on biopsies collected from the subject. According to the present invention the subject can be assessed for their risk of having dysplasia (such as one or more of LGD, HGD, IMC; optionally also including asymptomatic OAC) by the methods described herein. These methods advantageously avoid biopsy. The methods of the invention suitably expressly exclude biopsy. The methods of the invention advantageously require only surface sampling of the oesophagus (or an in vitro sample from the surface of the oesophagus), thereby avoiding biopsy and/or endoscopy.

Thus a key part of the invention is the use of the panel of markers to assess the risk of the subject having dysplasia such as one or more of LGD, HGD, or IMC.

OAC is more typically regarded as an invasive form of disease; typically patients with OAC already display symptoms; typically the methods of the invention are used for screening or surveillance applications and for risk assessment applications rather than for express diagnosis of (e.g.) OAC. Invasive OAC is typically diagnosed using a different algorithm which is not part of this invention. However, asymptomatic OAC (or more precisely the elevated risk of asymptomatic OAC) can be detected by the methods of the present invention in the same manner as LGD/HGD/IMC (or more precisely the elevated risk of LGD/HGD/IMC). This has been carried out by the inventors. The invention was applied in the manner described herein. The result of that application of the method was an indication of higher risk of abnormality/dysplasia in that subject. The subject was recommended to undergo endoscopy/biopsy as a result of the finding of higher risk according to the present invention. The endoscopy/biopsy revealed asymptomatic OAC. The patient was then referred for appropriate treatment. Therefore the invention can be applied to the assessment of risk of abnormality/dysplasia which can include asymptomatic OAC, but the invention does not purport to be a diagnostic tool giving a definite diagnosis of OAC.

**Subject/Patient Groups**

Suitably the methods of the invention are applied to any subject. Suitably the methods of the invention are applied to any subject suspected of having Barrett's oesophagus. These applications might be useful in screening the population at large.

More suitably the methods/panel of the invention finds application in subjects or patients who are not known to have carcinoma but may be monitored or followed-up for Barrett's oesophagus.

More suitably the methods of the invention are applied to any subject having Barrett's oesophagus.
It is aiding the assessment of the risk of progression or the risk of having LGD/HGD/IMC in subjects which already have Barrett's oesophagus which is a key benefit of the invention.

The panel of the invention is not intended for detection of Barrett's oesophagus, but is intended for assessment of the risk of having dysplasia. Assessment of having Barrett's oesophagus is typically carried out using the established TFF3 marker of Barrett's oesophagus, or may be carried out by any suitable method for diagnosis of Barrett's oesophagus.

A key marker of Barrett's oesophagus is the TFF3 marker, (ie TFF3 positive on the surface sampled cells eg from a capsule sponge such as a Cytosponge™. Such subjects may turn out to have no dysplasia, low grade dysplasia, high grade dysplasia, or be indefinite for dysplasia. It is also possible that the patient could have an undiagnosed superficial intramucosal carcinoma. However the main benefit of the invention is in assessing risk of having dysplasia from a start point of already having Barrett's oesophagus. Of course the panel/method of the invention can be applied as a general screening tool to asymptomatic subjects, but this might not be economic (even though it would of course be very effective). Thus for economic and practical reasons the invention finds best application in screening those subjects already at risk of dysplasia, ie. those patients already having Barrett's oesophagus.

Suitably the subject has Barrett's oesophagus.
Suitably the subject tests positive for TFF3 in surface sampled oesophagus cells.

In one embodiment the test (panel) of the invention may be preceded by testing for TFF3. This serves as a useful internal control. If the subject is known to have Barrett's oesophagus, then their surface sampled cells should test positive for TFF3. Therefore if a surface sample of the oesophagus of a subject who is known to have Barrett's oesophagus tests negative for TFF3, this would indicate that the sample is inadequate (eg. insufficient cells, or lack of columnar cells, or some other issue). The recommendation then would be to resample the surface of the subject's oesophagus and retest for TFF3, and only proceed to test using the panel of the invention once a positive result for TFF3 is observed, indicating a reliable/robust sample from a patient with Barrett's oesophagus.

The inventors have, among other things, designed BEST2, a multicentre, prospective case and control study aiming to recruit 1,000 patients which is carried out to test the performance characteristics of the Cytosponge™ for diagnosing Barrett's oesophagus compared with endoscopy. Additionally, within BEST2, a panel of risk stratification biomarkers are evaluated on the Cytosponge™ to determine their ability to risk stratify patients according to the endoscopic grade of dysplasia. The panel of risk stratification biomarkers consists of four different biomarkers, namely p53 protein levels, c-MYC protein levels, Aurora kinase A (AURKA) protein levels and methylation of the promoter regions of the Runt-related transcription factor 3 (RUNX3) and myogenic differentiation 1 (MYOD1) genes. Optionally the panel may further comprise a fifth marker, atypia.

**Sample and Sample Collection**

Suitably the sample comprises cells from the subject of interest. Suitably the sample comprises oesophageal cells from the subject of interest. Suitably the sample is non-endoscopic ie. suitably the sample is obtained without the use of an endoscope. Endoscopic sampling is an invasive technique. Furthermore, endoscopic sampling is a targeted technique where biopsies are taken at intervals along the oesophagus, or where lesions are visually identified by the operator and specifically targeted for biopsy. Suitably the invention does not involve endoscopic samples such as endoscopic biopsies.

A key principle of the invention is to provide a test which is specific for oesophageal abnormalities. The test is specific for in the sense of not delivering problematic levels of false positives from cells of unrelated tissues such as normal squamous oesophagus, or gastric cardia (stomach). Thus, by providing a test with these specific characteristics, the invention advantageously provides a test targeted to detection of abnormal oesophagus cells. In this way, the invention advantageously avoids the need for targeted sample collection. Thus, the invention advantageously involves samples obtained by non-targeted sample collection such as sampling the entire surface of the oesophagus rather than only targeting areas of suspected lesions (Barrett's).

Thus, suitably the sample does not comprise an endoscopic biopsy.

Suitably the sample may comprise oesophageal brushings or surface cells. Oesophageal brushings may be obtained using an endoscope or by other means; suitably when the sample comprises oesophagal brushings they are obtained by non-endoscopic means.

Suitably the sample comprises cells from the surface of a subject's upper intestinal tract.

Suitably the sample consists of cells from the surface of a subject's upper intestinal tract.

Suitably the sample may comprise cells sampled from the entire oesophageal lumen. Suitably the sample may comprise both oesophageal and non-oesophageal cells. Suitably the sample may comprise oesophageal cells together with gastric cardia cells. Suitably the sample may consist of oesophageal cells.

Suitably the sample comprises cells from the surface of a subject's oesophagus. Suitably the sample consists of cells from the surface of a subject's oesophagus.

Most suitably, the sample may comprise cells collected using a capsule sponge type sampling technique.

Especially suitable sampling techniques are described in the examples section. Examples of suitable samples include oesophageal brushings (whether endoscopically or non-endoscopically obtained), samples obtained via balloon cytology, samples obtained via capsule sponge sampling. Most suitably, a sample comprises cells obtained via capsule sponge sampling.

The panel of markers are relevant to luminal surface cells. This means that the sample to be analysed need only be collected from the surface of the oesophageal lumen. This advantageously avoids the need for a biopsy such as an endoscopic biopsy. Moreover, this advantageously avoids the need to preserve tissue architecture in the sample being analysed.

A further advantage of the markers of the invention is that they have been selected to avoid false positives arising from cells collected from the gastric mucosa (e.g. gastric cardia/stomach). This has a specific advantage that if cells of the gastric mucosa are included in the sample, then the panel will still able to function as a mode of detection of oesophageal abnormalities. This is because the markers are not found in gastric mucosa cells, and therefore no false positives occur even when the sample comprises cells of the gastric mucosa.

Thus it can be appreciated that the choice of markers in the panel by the inventors provides a degree of specificity which has not yet been provided in any prior art approach to screening for oesophageal abnormalities. The present inventors were the first to actively seek, and to successfully provide, a panel capable of such focused discrimination.

A non-endoscopic capsule sponge device which has been used in a previous clinical study (for example Ref no: CI/2007/0053 in the UK) may be used for sample collection. A pilot study demonstrated that this device (the Cytosponge™) is acceptable to patients and could be used in primary care. The device consists of a polyurethane sponge, contained within a gelatin capsule, which is attached to a string. The capsule is swallowed and dissolves within the stomach after 3-5 minutes.
Suitably the cytological specimen collected is processed to a pellet which can then be embedded in paraffin thus preserving the tissue architecture. This can then undergo histological assessment and in addition, multiple molecular and/or morphological markers may be used on a single sample. Thus, this mode of sample collection is particularly suitable for use in the present invention.

The cells are suitably sampled from the surface of the oesophagus using a swallowable abrasive material, which material is retrieved from the patient and from which the cells are subsequently separated for analysis to determine the presence of the markers. Preferably substantially the entire surface of the oesophagus is sampled, preferably the entire surface.
By abrasive is meant that the material is capable of removing cells from the internal surface of the oesophagus. Clearly, since this is meant for use in a subject's oesophagus, 'abrasive' must be interpreted in the light of the application. In the context of the present invention the term 'abrasive' has the meaning given above, which can be tested by passing the material through the oesophagus in an appropriate amount/configuration and examining it to determine whether cells have been removed from the oesophagus.
The material used in the collection device must be sufficiently abrasive to sample any dysplastic cells present in the oesophagus. Preferably the material is sufficiently abrasive to sample any Barrett's or dysplastic or adenocarcinoma cells present. In a most preferred embodiment, preferably the material is sufficiently abrasive to be capable of sampling the whole oesophagus ie. so that some squamous cells are collected together with any Barrett's and/or columnar and/or adenocarcinoma cells which may be present. This is advantageous because squamous cells are more difficult to remove than dysplastic cells and so their sampling provides a control to the operator such that if normal squamous cells are removed by the material then the chances of having not sampled the cells of interest such as Barrett's or dysplastic cells (if present), which are easier to remove than normal squamous cells, is correspondingly small.
Preferably the swallowable abrasive material is expandable. In this embodiment, preferably the abrasive material is of a smaller size when swallowed than when withdrawn. An expandable material may be simply a resilient material compressed such that when released from compression it will expand again back to a size approximating its uncompressed size. Alternatively it may be a material which expands e.g. upon taking up aqueous fluid to a final size exceeding its original size.
In other words, preferably the material of the device expands, swells, inflates or otherwise increases in size between swallowing and withdrawal. Preferably the device is auto-expandable ie. does not require further intervention between swallowing and expansion. Preferably the device is not inflatable. Preferably the device expands by unfolding, unfurling, uncoiling or otherwise growing in size following removal of restraint after swallowing. Preferably the material of the device is compressible and reverts a size approximating its uncompressed size following swallowing. Preferably the device is constructed from a compressed material which is releasably restrained in a compressed state. Preferably the material is released from restraint after swallowing, allowing expansion of the device/material before withdrawal.
Preferably the device comprises compressible material which is compressed into capsule form. Preferably the compressible material is in the form of sponge material. Preferably the compressed sponge is at least partially surrounded by a soluble and/or digestible coat such as a capsule coat. Preferably the sponge is indigestible. Preferably the capsule coat is at least partially formed from gelatin. Preferably the capsule coat is fully formed from gelatin.
In one embodiment it may be desirable to make the whole device out of digestible material to increase safety in case of a device becoming lost in the subject. Naturally the abrasive material would need to be digested at a slower rate than the capsule and the cord would need to be similarly slowly digested. Preferably the abrasive material is non-digestible. Preferably the cord is non-digestible.
Preferably the abrasive material comprises polyurethane, preferably polyurethane sponge.
Suitably said abrasive material is compressible. Suitably said abrasive material comprises reticulated polyurethane.
Suitably the material has a uniform shape.
Suitably the material has a uniform diameter.
Suitably the uncompressed shape is round such as spherical.
Suitably the uncompressed diameter is 3cm.

Suitably said cord is attached to said abrasive material via a loop of cord arranged below the surface of the abrasive material, said loop being closed by a hitch knot.

Suitably said abrasive material is compressed and wherein said abrasive material is retained in a compressed state by a soluble capsule.
Suitably said soluble capsule comprises a gelatine capsule.
Suitably said capsule is capable of dissolution and the compressible abrasive material is capable of reverting to its uncompressed size within 5 minutes upon immersion in water at 30 degrees Celsius.

Preferably the device is a capsule sponge. As will be apparent from the specification, a capsule sponge is a device comprising compressible sponge as the abrasive material, which sponge is compressed into a capsule shape, which capsule shaped compressed sponge is preferably reversibly restrained in its compressed state by at least a partial coat of soluble and/or digestible material such as gelatine. Preferably the device is a capsule sponge as described in WO2011/058316.
Preferably the sample does not comprise endoscopically collected material. Preferably the sample does not comprise endoscopic biopsy. Preferably the sample does not comprise endoscopic brushings.
It is a feature of the invention that the sampling is not directed e.g. visually directed to any particular part of the oesophagus but rather the sponge is scraped along the entire surface of the oesophagus and obtains a heterogeneous sample of cells from the tract. It is a further advantage of the invention that a greater proportion of the surface of the oesophagus is sampled than is achieved by prior art techniques such as endoscopic biopsy (which samples approximately 1% of the surface) or endoscopic brushing.
Preferably at least 10% of the oesophageal surface is sampled, preferably at least 20%, preferably at least 30%, preferably at least 40%, preferably at least 50%, preferably at least 60%, preferably at least 70%, preferably at least 80%, preferably at least 90%. In a most preferred embodiment, preferably substantially the entire oesophagus is sampled, preferably the whole inner lumen of the oesophagus is sampled. This applies equally to the *in vitro* sample e.g. when the method of the invention does not include collection of the sample.
Suitably the sample is an *in vitro* sample.
Suitably the sample is an extracorporeal sample.
Suitably sampling the cellular surface of the upper intestinal tract such as the oesophagus comprises the steps of

(i) introducing a swallowable device comprising abrasive material capable of collecting cells from the surface of the oesophagus into the subject,
(ii) retrieving said device by withdrawal through the oesophagus, and
(iii) collecting the cells from the device.

Preferably step (i) comprises introducing a swallowable device comprising abrasive material capable of collecting cells from the surface of the oesophagus into the subject's stomach.
Suitably the sample is from a white Caucasian human subject.
Suitably the sample is from a subject with a history of reflux.
Suitably the sample is from a male subject.
Suitably the sample is from an obese subject.

**Methods of the Invention**

In one embodiment suitably the method is an *in vitro* method. In one embodiment suitably the method is an extracorporeal method. In one embodiment suitably the actual sampling of the cells is not part of the method of the invention. Suitably the method does not involve collection of the cells.
Suitably the sample is a sample previously collected. Suitably the method does not require the presence of the subject whose cells are being assayed. Suitably the sample is an *in vitro* sample. Suitably the method does not involve the actual medical decision, *stricto sensu*; such a decision *stricto sensu* would typically be taken by the physician.
Suitably the method of the invention is conducted *in vitro.* Suitably the method of the invention is conducted extracorporeally.

**Markers Used in the Invention**

| Marker | Abbreviation | Accession number / sequence | Comments |
|---|---|---|---|
| Trefoil factor 3 | TFF3 | NM_003226.2 | protein |
| p53 tumour suppressor protein | p53 | NM_000546 | protein |
| p53 tumour suppressor protein | p53 | NM_000546 (most suitably version NM_000546.5 - shown in full below) | nucleic acid |
| c-Myc oncogene | c-Myc | NM_002467 | protein |
| Aurora kinase A | AURKA | NM_198434 | protein |

(continued)

| Marker | Abbreviation | Accession number / sequence | Comments |
|---|---|---|---|
| | | most suitably the AURKA accession number/ sequence is NP_003591.2, which corresponds to the AURKA associated with the exemplary antibody used (see examples) | |
| serine/threonine-protein kinase PLK1 | PLK1 | NP_005021.2 | protein |
| myogenic differentiation 1 | MyoD1 | NM_002478 (see examples for primer sequences defining target) | methylation of nucleic acid |
| Runt-related transcription factor 3 | Runx3 | NM_001031680 (see examples for primer sequences defining target) | methylation of nucleic acid |

The Genbank accession numbers are provided with reference to the database as of the filing date of this application ie. 21 Feb 2013. In case any further assistance is needed, preferably the accession numbers provided should be taken to refer to Genbank release number 194.0 of 15 Feb 2013.

By way of illustration, the exemplary p53 sequence is provided below, as retrieved from GenBank:

**Homo sapiens tumor protein p53 (TP53), transcript variant 1, mRNA**

NCBI Reference Sequence: NM_000546.5

ACCESSION NM_000546

VERSION NM_000546.5

ORIGIN

```
1 gatgggattg gggttttccc ctcccatgtg ctcaagactg gcgctaaaag ttttgagctt
61 ctcaaaagtc tagagccacc gtccagggag caggtagctg ctgggctccg gggacacttt
121 gcgttcgggc tgggagcgtg ctttccacga cggtgacacg cttccctgga ttggcagcca
181 gactgccttc cgggtcactg ccatggagga gccgcagtca gatcctagcg tcgagccccc
241 tctgagtcag gaaacatttt cagacctatg gaaactactt cctgaaaaca acgttctgtc
301 ccccttgccg tcccaagcaa tggatgattt gatgctgtcc ccggacgata ttgaacaatg
361 gttcactgaa gacccaggtc cagatgaagc tcccagaatg ccagaggctg ctccccccgt
421 ggcccctgca ccagcagctc ctacaccggc ggcccctgca ccagcccccct cctggcccct
481 gtcatcttct gtcccttccc agaaaaccta ccagggcagc tacggtttcc gtctgggctt
541 cttgcattct gggacagcca agtctgtgac ttgcacgtac tcccctgccc tcaacaagat
601 gttttgccaa ctggccaaga cctgccctgt gcagctgtgg gttgattcca caccccccgcc
661 cggcacccgc gtccgcgcca tggccatcta caagcagtca cagcacatga cggaggttgt
721 gaggcgctgc ccccaccatg agcgctgctc agatagcgat ggtctggccc ctcctcagca
781 tcttatccga gtggaaggaa atttgcgtgt ggagtatttg gatgacagaa acacttttcg
841 acatagtgtg gtggtgccct atgagccgcc tgaggttggc tctgactgta ccaccatcca
901 ctacaactac atgtgtaaca gttcctgcat gggcggcatg aaccggaggc ccatcctcac
961 catcatcaca ctggaagact ccagtggtaa tctactggga cggaacagct ttgaggtgcg
1021 tgtttgtgcc tgtcctggga gagaccggcg cacagaggaa gagaatctcc gcaagaaagg
1081 ggagcctcac cacgagctgc ccccagggag cactaagcga gcactgccca acaacaccag
1141 ctcctctccc cagccaaaga agaaaccact ggatggagaa tatttcaccc ttcagatccg
1201 tgggcgtgag cgcttcgaga tgttccgaga gctgaatgag gccttggaac tcaaggatgc
1261 ccaggctggg aaggagccag gggggagcag ggctcactcc agccacctga agtccaaaaa
1321 gggtcagtct acctcccgcc ataaaaaact catgttcaag acagaagggc ctgactcaga
1381 ctgacattct ccacttcttg ttccccactg acagcctccc acccccatct ctccctcccc
1441 tgccattttg ggttttgggt ctttgaaccc ttgcttgcaa taggtgtgcg tcagaagcac
1501 ccaggacttc catttgcttt gtcccggggc tccactgaac aagttggcct gcactggtgt
1561 tttgttgtgg ggaggaggat ggggagtagg acataccagc ttagatttta aggtttttac
1621 tgtgagggat gtttgggaga tgtaagaaat gttcttgcag ttaagggtta gtttacaatc
1681 agccacattc taggtagggg cccacttcac cgtactaacc agggaagctg tccctcactg
1741 ttgaattttc tctaacttca aggcccatat ctgtgaaatg ctggcatttg cacctacctc

1801 acagagtgca ttgtgagggt taatgaaata atgtacatct ggccttgaaa ccacctttta
1861 ttacatgggg tctagaactt gacccccttg agggtgcttg ttccctctcc ctgttggtcg
1921 gtgggttggt agtttctaca gttgggcagc tggttaggta gagggagttg tcaagtctct
1981 gctggcccag ccaaaccctg tctgacaacc tcttggtgaa ccttagtacc taaaaggaaa
2041 tctcacccca tcccacaccc tggaggattt catctcttgt atatgatgat ctggatccac
2101 caagacttgt tttatgctca gggtcaattt cttttttctt tttttttttt tttttctttt
2161 ttctttgaga ctgggtctcg ctttgttgcc caggctggag tggagtggcg tgatcttggc
2221 ttactgcagc ctttgcctcc ccggctcgag cagtcctgcc tcagcctccg gagtagctgg
2281 gaccacaggt tcatgccacc atggccagcc aacttttgca tgttttgtag agatggggtc
2341 tcacagtgtt gcccaggctg gtctcaaact cctgggctca ggcgatccac ctgtctcagc
2401 ctcccagagt gctgggatta caattgtgag ccaccacgtc cagctggaag ggtcaacatc
2461 ttttacattc tgcaagcaca tctgcatttt caccccaccc ttcccctcct tctccctttt
2521 tatatcccat ttttatatcg atctcttatt ttacaataaa actttgctgc cacctgtgtg
2581 tctgaggggt g
```

## Atypia

Atypia is assessed by observation.

Suitably the cells are stained before observation. Suitably the cells are stained using haematoxylin and eosin (H&E) stain. This has the advantage of rendering the cells easily distinguished from one another according to conventional and long established histology.

Standard histology / cytology is used to tell the cells apart.

Scoring is carried out in accordance with the Vienna scale.

In the context of the invention, abnormal is judged according to the Vienna scale; therefore observing one or more of those abnormal categories of cells when assaying atypia as an optional extra marker in addition to the panel of markers of the invention would mean that a finding of 'abnormal' was recorded for the atypia marker in that analysis.

It is an advantage of optionally also assaying atypia in addition to the four markers of the panel of the invention that increased sensitivity and/or specificity may be obtained.

In case any further guidance is needed, reference is made to standard text books in this area such as Diagnostic Cytopathology by Winifred Gray 2nd edition. In addition, or alternatively, text books such as Gastrointestinal Pathology An Atlas and Textbook by Cecilia M. Fenoglio-Preiser, Amy E. Noffsinger, Grant N. Stemmermann, Patrick E. Lantz, Peter G. Isaacson Third edition may be used. These texts are specifically incorporated herein by reference for the sections showing the characteristics of the cell types mentioned herein. Any other conventional cytology/histology guides may be used if required.

Haematoxylin and Eosin (H&E)

The haematoxylin and eosin stain uses two separate dyes, one staining the nucleus and the other staining the cytoplasm and connective tissue. Haematoxylin is a dark purplish dye that will stain the chromatin (nuclear material) within the nucleus, leaving it a deep purplish-blue colour. Eosin is an orangish-pink to red dye that stains the cytoplasmic material including connective tissue and collagen, and leaves an orange-pink counterstain. This counterstain acts as a sharp contrast to the purplish-blue nuclear stain of the nucleus, and helps identify other entities in the tissues such as cell membrane (border), red blood cells, and fluid.

The staining process involves hydration of the sample (if necessary); staining with the nuclear dye (hematoxylin) and rinsing, then staining with the counterstain (eosin). They are then rinsed, and if necessary dehydrated (e.g. treated with water, then alcohol, and then xylene), and prepared for observation e.g. by addition of coverslips.

Progressive/Regressive Staining

There are two methods for performing the H&E stain: Progressive in which the slides are placed in haematoxylin then rinsed and placed in eosin; and the regressive method in which the slides are placed in a stronger type of haematoxylin, then differentiated in acid alcohol to take the haematoxylin back out of everything except the nucleus, and then placed in eosin. In both types of staining, a bluing solution (Scott's Tap Water or ammonia water) is optionally used to cause the nucleus to turn a deep purplish blue color.

In progressive staining, a milder form of haematoxylin is used that will only stain the nucleus of the cell and cause the nuclear material to turn a deeper blue when rinsed in water. With this method the technician can simply stain, rinse and move on to the next step. Its advantage is simplicity, fewer steps, and avoids the possibility of over/under differentiation in acid alcohols. The level or colour of staining is standardized and consistent. Progressive staining has the advantage of easier automation.

Haematoxylin products for progressive staining are commercially available such as from Sigma Inc. (Sigma Aldrich) and include: Gill's 1, Gill's 2, Gill's 3, and Mayer's haematoxylin. The difference in the three Gill stains is the haematoxylin strength. Gill's 1 is used primarily for cytology staining where a weaker haematoxylin is adequate because you are staining individual cells from a fluid suspension, not tissue. Gill's 2 and 3 are stronger and generally used for histology staining. They are developed for tissue structure. The choice of whether to use Gill's 2 or 3 is a matter of preference for the skilled worker.

In regressive staining, a stronger form of haematoxylin is used called Harris haematoxylin. Harris haematoxylin will stain everything on the slide and hold fast to the tissue when rinsed. Therefore after staining and rinsing with water, the next step is to differentiate or take out the excess haematoxylin from everything except the nucleus. The slides are agitated in a mild acid alcohol solution that slowly removes the excess haematoxylin. After differentiating the slides are rinsed and placed in a bluing solution (Scott's Tap Water or ammonia water), which will cause the nucleus to turn a deep purplish blue colour.

Haematoxylin products for regressive staining are commercially available such as from Sigma Inc. (Sigma Aldrich) and include Harris haematoxylin.

After haematoxylin staining the samples are rinsed, and stained in eosin. If necessary, they may be dehydrated with graded strengths of alcohols, cleared in xylene and finally prepared for observation e.g. with coverslips and/or permanent mounting media.

Eosin products are commercially available such as from Sigma Inc. (Sigma Aldrich) and include Eosin Y, Eosin Y Alcoholic, and Eosin Y with Phloxine. Similar to the three types of Gill's stain, the eosins are differentiated by their strength and depth in colour. Eosin Yis the weakest of the three and gives a pink stain to the cytoplasm and collagen. Eosin Y Alcoholic is a stronger stain and gives a more brilliant orangish red colour due to its alcohol ingredient. Eosin Y with Phloxine is the strongest stain and has an overwhelmingly red colour due to the addition of phloxine. While the selection of eosin is a matter for the skilled worker, Eosin Y with Phloxine is generally considered too red for standard histology. Thus suitably the eosin used is Eosin Y Alcoholic.

It is an advantage of haematoxylin and eosin (H&E) stain that use of molecular markers for specific cell types can be avoided.

**Reference Standard**

The invention requires determination of 'abnormal' levels of certain markers. 'Abnormal' may be defined by comparison to a reference standard.

Within the context of the present invention, a reference standard functions as an object of comparison to which the expression levels/methylation levels/atypia present in the sample of the subject can be compared to. The reference standard may comprise a sample from a healthy subject which is analysed in parallel with the sample of interest. Alternatively said reference standard may comprise expression level value(s) for said biomarkers previously determined from a sample taken from a healthy subject so as to give values of expression level of said biomarkers to compare with. This has the advantage of not requiring parallel analysis of the reference sample each time the method is carried out. Suitably the healthy person is an individual of similar demographic characteristics, such as age, sex, weight and any other relevant parameters, to the subject being considered.

The reference standard may also be a set of expression level values for said biomarkers determined over time as a mean. This has the advantage of eliminating the practical issues of taking and measuring a sample from a separate individual every time the method is performed. Suitably said set of expression level values for said biomarkers determined over time as a mean would be divided into different categories divided by medical characteristics, such as age, sex, weight and others, so as to provide a more directly comparable set of values for the particular subject being examined.

For the protein markers of the invention, their staining is scored as described herein. The scoring system already takes account of normal/abnormal. Therefore the need for direct reference standards for each analysis is advantageously made optional due to the absolute categorisation via scoring the staining.

For methylation, the MethyLight score is regarded as abnormal when assessed as described herein, such as in the examples section.

An exemplary methylation cut off for use is 0.02604. This may be varied according to need by the operator working the invention. For Methylight assays, exemplary cutoffs (methylation cut-offs) are in the range of 0.01-0.31. Again, these may be varied according to need by the operator working the invention.

**Reference Sequence**

When particular amino acid residues are referred to using numeric addresses, the numbering is taken using the full length amino acid sequence as the reference sequence. This is to be used as is well understood in the art to locate the residue of interest. This is not always a strict counting exercise - attention must be paid to the context. For example, if the protein of interest such as human p53 is of a slightly different length, then location of the correct residue in the p53 sequence corresponding to a particular residue may require the sequences to be aligned and the equivalent or corresponding residue picked, rather than simply taking the identically numbered residue of the sequence of interest. This is well within the ambit of the skilled reader.

Moreover, in the context of the present invention it is detection of particular polypeptide sequences corresponding to those described which is important. The techniques and/or reagents for such detection are widely available and/or straightforward to obtain or generate. Exemplary materials and techniques are provided in the examples section. Detection of a particular polypeptide e.g. the polypeptide product of a particular gene is suitably to be considered at the level of protein detection. It is a question of expression of the protein, rather than a determination of a specific or precise 100% identical amino acid sequence. Exemplary amino acid sequences are provided as guidance for the polypeptide being detected and are not intended to constrain the invention to the detection of only those precise full length 100% identical amino acid sequences. Thus, variants such as allelic variants; mutants such as point mutations or short additions or deletions which do not alter the fundamental identity of the polypeptide; or fragments such as splice variants, cleaved or mature proteins; post translationally modified proteins or other such common forms are to be considered within the remit of determining the presence/absence or expression level of the various biomarker proteins disclosed.

A fragment is suitably at least 10 amino acids in length, suitably at least 25 amino acids, suitably at least 50 amino acids, suitably at least 100 amino acids, suitably at least 200 amino acids, suitably the majority of the polypeptide of interest. Suitably a fragment comprises a whole motif or a whole domain of the polypeptide of interest.

## Sequence Homology/Identity

Although sequence homology can also be considered in terms of functional similarity (i.e., amino acid residues having similar chemical properties/functions), in the context of the present document it is preferred to express homology in terms of sequence identity.

Sequence comparisons can be conducted by eye or, more usually, with the aid of readily available sequence comparison programs. These publicly and commercially available computer programs can calculate percent homology (such as percent identity) between two or more sequences.

Percent identity may be calculated over contiguous sequences, i.e., one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues (for example less than 50 contiguous amino acids). For comparison over longer sequences, gap scoring is used to produce an optimal alignment to accurately reflect identity levels in related sequences having insertion(s) or deletion(s) relative to one another. A suitable computer program for carrying out such an alignment is the GCG Wisconsin Bestfit package (University of Wisconsin, U.S.A; Devereux et al., 1984, Nucleic Acids Research 12:387). Examples of other software than can perform sequence comparisons include, but are not limited to, the BLAST package, FASTA (Altschul et al., 1990, J. Mol. Biol. 215:403-410) and the GENEWORKS suite of comparison tools.

In the context of the present document, a homologous amino acid sequence is taken to include an amino acid sequence which is at least 40, 50, 60, 70, 80 or 90% identical. Most suitably a polypeptide having at least 90% sequence identity to the biomarker of interest will be taken as indicative of the presence of that biomarker; more suitably a polypeptide which is 95% or more suitably 98% identical at the amino acid level will be taken to indicate presence of that biomarker. Suitably said comparison is made over at least the length of the polypeptide or fragment which is being assayed to determine the presence or absence of the biomarker of interest. Most suitably the comparison is made across the full length of the polypeptide of interest. The same considerations apply to nucleic acid nucleotide sequences.

## Advantages

mRNA studies, such as are the main focus of Rugge et al 2010, suffer from difficulties in establishing a 'normal' level. It is challenging to define a cutoff value. Very large sample sizes are needed to render the results reliable. Wide ranges of expression levels are observed. mRNA expression levels may not correlate to protein expression levels. mRNA can degrade on Cytosponge collected samples. Protein is more stable. Rugge et al make no mention of the use of AURKA for diagnosis of dysplasia in Barrett's oesophagus. In a primary care setting the sample may be collected, stored in a fridge, posted in the mail and only then arrive at a laboratory for testing. It is an advantage of the invention that signal is not compromised during such sample treatments. Rugge et al measure AKA by IHC and do also correlate with p53. A main drawback with Rugge et al is that they report AKA as being a primarily cytoplasmic stain. This is highly problematic since AKA functions in the nucleus and so the cytoplasmic stain in Rugge et al does not seem reliable. The Ab Rugge et al use is from Epitomics and it appears likely that this is non-specific and is producing unreliable results. By contrast, we demonstrate nuclear staining. As an example, the inventors use an antibody from Millipore. The inventors have checked the antibody for specificity.

Suitably mRNA is not used for analysis in the present invention.

Suitably the antibodies used herein are specific for the protein(s) being assayed.

Liu et al 2008 study a panel of cancers from Chinese patients. In China, >90% of oesophageal cancers are squamous cell cancers. Therefore the authors have demonstrated expression of p53 in squamous cell cancers of the oesophagus, not along the progression from Barrett's to adenocarcinoma.

Agnese et al 2007 seek to assess whether Aurora Kinase A and p53 could help differentiate between Barrett's oesophagus with intestinal metaplasia and Barrett's oesophagus with gastric metaplasia. The conclusion of the abstract states that the study is too small to yield any significant results. In any case, by contrast the invention is concerned with detection of dysplasia/cancer for example in Barrett's oesophagus which is a separate question to Agnese et al's attempts to distinguish between gastric and intestinal metaplasia. Agnese et al measure RNA transcript levels and do not examine protein levels of Aurora Kinase A. RNA transcript levels do not necessarily translate to protein due to post-translational modifications. The inventors would not rely on RNA to say that it will be a good protein level biomarker. Numerous candidate markers fall out at this stage i.e. do not produce good protein biomarkers. Some aspects are now described by way of numbered paragraphs:

i. A method of aiding detection of a surface abnormality in the oesophagus of a subject, the method comprising:

a) providing a sample of cells from said subject, wherein said sample comprises cells collected from the surface of the subject's oesophagus;
b) assaying said cells for at least two markers selected from

(i) p53;
(ii) c-Myc;
(iii) AURKA or PLK1, preferably AURKA; and
(iv) methylation of MyoD and Runx3;

wherein detection of abnormal levels of at least two of said markers infers that the subject has an increased likelihood of a surface abnormality in the oesophagus.

ii. A method according to paragraph i wherein step (b) comprises

(1) contacting said cells with reagents for detection of at least a first molecular marker selected from:

(i) p53;
(ii) c-Myc;
(iii) AURKA or PLK1, preferably AURKA; and
(iv) methylation of MyoD and Runx3, and

(2) contacting said cells with reagents for detection of at least a second molecular marker selected from (i) to (iv).

iii. A method according to paragraph i or paragraph ii, wherein said surface abnormality is selected from the group consisting of low-grade dysplasia (LGD), high-grade dysplasia (HGD), asymptomatic oesophageal adenocarcinoma (OAC) and intra-mucosal cancer (IMC).

iv. A method according to paragraph i or paragraph ii, wherein abnormal levels of at least three of said markers are assayed.

v. A method according to any preceding paragraph, wherein abnormal levels of at least four of said markers are assayed.

vi. A method according to any preceding paragraph, further comprising assaying said cells for atypia.

vii. A method according to any preceding paragraph, wherein said cells are collected by unbiased sampling of the surface of the oesophagus.

viii. A method according to paragraph vii, wherein said cells are collected using a capsule sponge.

ix. A method according to any preceding paragraph, wherein the cells are prepared prior to being contacted with the reagents for detection of the molecular markers by the steps of (i) pelleting the cells by centrifuge, (ii) re-suspending the cells in plasma, and (iii) adding thrombin and incubating until a clot is formed.

x. A method according to paragraph ix, further comprising the step of incubating said clot in formalin, processing into a paraffin block, and slicing into sections suitable for microscopic examination.

xi. A method according to any preceding paragraph, wherein p53 is assessed by immunohistochemistry.

xii. A method according to any preceding paragraph, wherein cMyc is assessed by immunohistochemistry.

xiii. A method according to any preceding paragraph, wherein AURKA is assessed by immunohistochemistry.

xiv. A method according to any preceding paragraph, wherein methylation of MyoD/Runx3 is assessed by MethyLight analysis.

xv. A method according to paragraph vi, wherein atypia is assessed by scoring the cells for their morphology according to the Vienna Scale.

xvi. A method according to any preceding paragraph, wherein step (b) of said method is preceded by the step of assaying said cells for TFF3.

xvii. An assay for selecting a treatment regimen, said assay comprising

a) providing a sample of cells from said subject, wherein said sample comprises cells collected from the surface of the subject's oesophagus;
b) assaying said cells for at least two markers selected from

(i) p53;
(ii) c-Myc;
(iii) AURKA; and
(iv) methylation of MyoD and Runx3;

wherein if abnormal levels of at least two of said markers are detected, then a treatment regimen of endoscopy and biopsy is selected.

xviii. An apparatus or system which is

(a) configured to analyse an oesophagal sample from a subject, wherein said analysis comprises
(b) assaying said cells for at least two markers selected from

(i) p53;
(ii) c-Myc;
(iii) AURKA; and
(iv) methylation of MyoD and Runx3;

said apparatus or system comprising an output module,
wherein if abnormal levels of at least two of said markers are detected, then said output module indicates an increased likelihood of a surface abnormality in the oesophagus for said subject.

xix. Use for applications relating to aiding detection of a surface abnormality in the oesophagus of a subject, of a material which recognises, binds to or has affinity for certain polypeptides, or methylation of certain nucleic acid sequences, wherein the polypeptides and/or nucleic acid sequences are as defined in any of paragraphs i to xv.

xx. Use according to paragraph xix of a combination of materials, each of which respectively recognises, binds to or has affinity for one or more of said polypeptide(s) or nucleic acid sequences.

xxi. An assay device for use in aiding detection of a surface abnormality in the oesophagus of a subject, which comprises a solid substrate having a location containing a material, which recognises, binds to or has affinity for certain polypeptides, or methylation of certain nucleic acid sequences, wherein the polypeptides and/or nucleic acid sequences are as defined in any of paragraphs i to xv.

xxii. A kit comprising reagents for determining the expression level of each of

(i) p53;
(ii) c-Myc;
(iii) AURKA;

in a biological sample, and optionally further comprising reagents for determining the methylation of MyoD and Runx3.

xxiii. A method for aiding the detection of a surface abnormality in the oesophagus of a subject, the method comprising providing a sample of cells from said subject, wherein said sample comprises cells collected from the surface of the subject's oesophagus, assaying said cells for TFF3, wherein if TFF3 is detected in cell(s) of the sample, the method according to any of paragraphs i to xv is carried out, wherein detection of abnormal levels of at least one marker in addition to detection of TFF3 indicates an increased likelihood of a surface abnormality in the oesophagus of said subject.

xxiv. A method according to paragraph xxiii wherein detection of abnormal levels of at least two markers in addition to detection of TFF3, preferably least three markers in addition to detection of TFF3, preferably least four markers in addition to detection of TFF3, preferably all five markers in addition to detection of TFF3, indicates an increased likelihood of a surface abnormality in the oesophagus of said subject.

xxv. A method according to paragraph xxiii or paragraph xxiv, wherein said cells are collected by unbiased sampling of the surface of the oesophagus.

xxvi. A method according to paragraph xxv, wherein said cells are collected using a capsule sponge.

## BRIEF DESCRIPTION OF THE DRAWINGS

Embodiments of the present invention will now be described further, with reference to the accompanying drawings, in which:

Figure 1 shows examples of c-MYC staining on cells obtained from the Cytosponge™ at the four different staining intensities (0, 1, 2, 3).
Figure 2 shows photographs and a graph.
Figure 3 shows a flow diagram.
Figure 4 shows a flow diagram.
Figure 5 shows a flow diagram.
Figure 6 shows photographs.
**Figure 7 shows a flow chart illustrating the study outline.** The number of samples used at each stage is given. The methodology used for each study phase is shown on the left hand side. EAC, Esophageal adenocarcinoma, BE, Barrett's esophagus, HGD, high grade dysplasia.
**Figure 8 shows mutation in esophageal adenocarcinoma.** The bar graph on the top indicates the percentage of samples with aberrations for a given gene. The number in bold denotes the total number of mutations for each gene. Genes with four or more mutations in our EAC discovery and validation cohort (combined total of 112 patients) were included. The proportion of missense, nonsense/splice and indel mutations are shown. The matrix below shows the number of samples with mutations in both genes for each possible pairing of genes. The red highlighted box indicates significantly co-occurring mutations (Benjamini-Hochberg adjusted p-value <0.05).
**Figure 9 shows *TP53* and *SMAD4* mutations accurately define the boundaries in the progression towards cancer whilst other mutations appear to occur independent of disease stage.** A. Bar graph showing the number of never-dysplastic BE patients (NDBE), BE patients with high grade dysplasia (HGD) and EAC patients with at least one mutation in our panel consisting of 26 genes. B. Percentage of never-dysplastic BE, BE with HGD and EAC samples with mutations in recurrently-mutated genes (mutated in ≥4 samples) identified in the EAC discovery cohort and EAC Validation cohort. *TP53* and *SMAD4* are the only genes for which mutations separate the boundaries between never-dysplastic and dysplastic BE (*TP53*) or cancer (*SMAD4*) (* p<0.05). C. Proposed model for the boundary-defining mutations in BE carcinogenesis. The hashed box depicts multiple other mutations which may occur and provide selective advantage at any stage of disease.
**Figure 10 shows *TP53* mutations can be used to diagnose BE with prevalent high-grade dysplasia on the Cytosponge™.** A. Schematic demonstrating Cytosponge™ sampling of cells from the top of the stomach, full length of the esophagus and oropharynx. B. Allele fractions for known *TP53* mutations, previously identified by sequencing *TP53* on diagnostic biopsies. For these four patients the mutation can also be detected in material collected using the Cytosponge™. Patient 4 swallowed the Cytosponge™ on two different occasions, 8 months apart, and the data for both Cytosponge™ samples is shown. N/A = Not applicable as no sample was taken, AF= allele fraction. C. The allele fraction of *TP53* mutations identified in Cytosponge™ samples is shown for the three patients groups: no BE, BE with no dysplasia and BE with high grade dysplasia (HGD). D. The positions of the *TP53* mutations identified on the Cytosponge™ samples are shown above the gene diagram compared with those found in the EAC and BE HGD biopsy cohorts. The dotted line on the gene outline denotes the two small areas not covered by the multiplex PCR assay (amino acids 1-27 and 361-393). TA, transcription activation domain; OD, oligomerization domain.

Further particular and preferred aspects are set out in the accompanying independent and dependent claims. Features of the dependent claims may be combined with features of the independent claims as appropriate, and in combinations other than those explicitly set out in the claims.
Where an apparatus feature is described as being operable to provide a function, it will be appreciated that this includes an apparatus feature which provides that function or which is adapted or configured to provide that function.

EXAMPLES

**Example 1: Selection of Markers**

There were a number of reasons for selecting the four molecular risk stratification biomarkers, (optionally plus a fifth marker atypia), examples of which are set out below:
p53 protein accumulation was selected as a biomarker as p53 is one of the best characterised tumour suppressor proteins and has been shown to be associated with dysplasia in Barrett's oesophagus (Bian et al., 2001) as well as with increased risk of progression to OAC (Kastelein et al., 2012; Sikkema et al., 2009).

c-MYC, a well characterised oncogene, was included as it is recurrently amplified in OAC (Miller et al., 2003; Rygiel et al., 2008) and displays increased gene expression in Barrett's with high grade dysplasia in our in-house gene expression arrays.

Aurora kinase A (AURKA) was selected as a surrogate marker of aneuploidy as AURKA overexpression, centrosome amplification and aneuploidy have been shown to be associated. AURKA is a key regulator of mitotic entry, centrosome maturation and spindle assembly and overexpression of AURKA has been shown to cause centrosome amplification and chromosomal instability (Zhou et al., 1998). AURKA protein expression has also been shown to be significantly upregulated in Barrett's with high grade dysplasia and OAC compared to Barrett's with no dysplasia (Rugge et al., 2010). For the methylation biomarkers, five genes that have previously been shown to be methylated with increasing grade of dysplasia were tested. These genes were p16, ESR1, MYOD1, HPP1 and RUNX3 (Eads et al., 2001; Schulmann et al., 2005). The best two were selected (MYOD1 and RUNX3).

**Example 2: Exclusion of Markers**

There are robust reasons for excluding other potential biomarkers for use on the Cytosponge™. Some examples of markers excluded from the design of the panel are discussed below:
Eleven other potential biomarkers were evaluated to determine whether these could be used in conjunction with the Cytosponge™ to detect Barrett's with dysplasia. The 11 biomarkers were EGFR, CDNK2A, FGFR2, CCNA1, DDX21, MSLN, PLK1, HER2, DNMT1, MYHFD2 and VNN2. EGFR, HER2, CDNK2A, CCNA1 and FGFR2 were selected from published literature and DDX21, MSLN, PLK1, DNMT1, MTHFD2 and VNN2 were selected from in-house gene expression array data. VNN2 was eliminated as there are no antibodies available for staining formalin fixed paraffin embedded (FFPE) slides for this protein. FGFR2 and CDKN2A were eliminated as expression of both these proteins was detected in gastric glandular tissue which would also be sampled by the Cytosponge™. MTHFD2 was excluded as the staining was only cytoplasmic and too faint overall.

CCNA1 was initially tested directly on the Cytosponge™ as Cyclin A has been used as a successful biomarker in the inventors' laboratory (Lao-Sirieix et al., 2004; Lao-Sirieix et al., 2007). Unfortunately CCNA1 did not perform well on the Cytosponge™ in the pilot analysis (TFF3+ positive controls with no Barrett's esophagus = 26, NDBE= 44, Indefinite for dysplasia = 12, LGD=7, HGD=7) and was therefore discontinued for the BEST2 study. It is most likely that CCNA1 did not perform well due to the proliferation within normal tissues and the inability to determine compartment specific proliferation (surface versus deeper glands) from the architecture of the Cytosponge™ collected cells.

HER2 staining was tested on some Cytosponge™ samples but as HER2 is known to be amplified or overexpressed in only about 15% of Barrett's with high grade dysplasia the staining was discontinued as it would not be a sensitive enough biomarker.

The remaining five biomarkers were excluded as they were either not sensitive or specific enough - see table:

**Table:** Sensitivity and specificity of the five biomarkers that were stained on our in-house TMAs but did not make it through to the final panel. The TMAs comprised of 54 Barrett's biopsies with no dysplasia, 32 Barrett's biopsies with low grade dysplasia and 18 Barrett's biopsies with high grade dysplasia.

| Protein biomarker | Sensitivity (%) | Specificity (%) |
|---|---|---|
| DDX21 | 77 | 84 |
| DNMT1 | 41 | 98 |
| EGFR | 72 | 77 |
| MSLN | 61 | 45 |
| PLK1 | 91 | 88 |

MSLN was excluded as it is expressed in Barrett's with no dysplasia and is therefore not specific enough for detecting Barrett's with dysplasia. DNMT1 looked promising as it was very specific (98%), however when we tried to verify the data using a different DNMT1 antibody the data did not agree. We therefore did not continue with DNMT1 as a biomarker as we lost confidence in the antibodies.

EGFR was excluded as overall the sensitivity (72%) and the specificity (77%) were too low. DDX21 was excluded as even though the sensitivity and specificity were acceptable, there were lots of Barrett's with no dysplasia cases that had low DDX21 expression and we were looking for a cleaner biomarker that had no staining versus staining. PLK1 had a good sensitivity (91%) and a good specificity (88%) but this biomarker was excluded as AURKA gave better sensitivity (93%) and specificity (94%) data and as AURKA and PLK1 overexpression would detect essentially the same cases we only chose one of the markers and chose AURKA as this gave better data.

**Example 3: Sample processing and preparation**

**Processing of the capsule sponge specimens**

Cytosponge™ capsules were swallowed by patients and then placed directly into preservative solution at 4°C until processed further. The samples were vortexed extensively and shaken vigorously to remove any cells from the sponge material. The preservative liquid containing the cells was centrifuged at 1000 RPM for 5 minutes to pellet the cells. The resulting pellet was re-suspended in 500 μL of plasma and thrombin (Diagnostic reagents, Oxford, UK) was then added in 10 μL increments until a clot formed. The clot was then placed in formalin for 24h prior to processing into a paraffin block. The sample was cut into 3.5 μm sections to provide 15 slides, named slides 1 to 15, with two sections placed on slide 1 and 2.

**Example 4: Assay of optional further marker - Atypia**

Assessing atypia on samples derived from the Cytosponge is carried out by microscopic examination and scoring.

The first slide containing two sections was stained with H&E and atypia was assessed by an expert pathologist (Dr Maria O'Donovan).

The scoring is carried out in accordance with the Vienna scale.

**Example 5: Assay of marker - Protein Biomarkers**

Each of the three protein biomarkers on/in cells in the samples obtained using the Cytosponge™ were assayed by immunohistochemical staining.

For each of the protein biomarkers one slide was stained using immunohistochemistry (IHC) to assess the protein expression in each of the samples. Slide 4 was used for p53, slide 8 for c-MYC and slide 10 for AURKA.

All slides were stained using the BondMax autostainer with the Leica Bond Polymer Detection kit. The conditions and antibodies used can be found in the following Table:

**Table: IHC staining conditions and antibodies used:**

| Antigen | Protocol | Antigen retrieval | Antibody | Antibody dilution |
|---|---|---|---|---|
| p53 | Protocol F | H1(30) | Novocastra™ Mouse Monoclonal Antibody<br>p53 Protein (DO-7)<br>Product Code: NCL-p53-DO7 | 1:50 |
| c-MYC | MRC+E* | H2(20) | Epitomics c-MYC antibody, clone Y69, Rabbit monoclonal<br>Cat#: 1472-1 | 1:50 |
| AURKA | MRC+E | H2(30) | Millipore Anti-Aurora-A (C-term), clone EP1008Y, Rabbit Monoclonal<br>Cat #: 04-1037 | 1:1000 |
| * For c-MYC staining, the primary antibody was incubated with 60 minutes | | | | |

Staining Protocol MRC+E

Leica MICROSYSTEMS

**Protocol: MRC+E60**

**Bond-max and Bond-x**

**Full name:** MRC & Enh 60'
**ID:** 10033
**Version:** 0(current)
**Type:** IHC staining
**Created by:** BondPowerUser
**Creation time:** 28/11/2011 15:32
**Facility:** MRC
**Staining status:** Single

---

**Step Reagent** *Supplier: Leica Microsystems*
1 *Peroxide Block
Step type: Reagent | Incubation time: 5min0s | Temperature: Ambient | Dispense type: Selected vol.

---

**Step Reagent** *Supplier: Leica Microsystems*
2 *Bond Wash Solution
Step type: Wash | Incubation time: 0s | Temperature: Ambient | Dispense type: Selected vol.

---

**Step Reagent** *Supplier: Leica Microsystems*
3 *Bond Wash Solution
Step type: Wash | Incubation time: 0s | Temperature: Ambient | Dispense type: Open

---

**Step Reagent** *Supplier: Leica Microsystems*
4 *Bond Wash Solution
Step type: Wash | Incubation time: 0s | Temperature: Ambient | Dispense type: Selected vol.

---

**Step Reagent** *Supplier: Not applicable*
5 Primary
Step type: Reagent | Incubation time: 60min0s | Temperature: Ambient | Dispense type: Selected vol.

---

**Step Reagent** *Supplier: Leica Microsystems*
6 *Bond Wash Solution
Step type: Wash | Incubation time: 0s | Temperature: Ambient | Dispense type: Selected vol.

---

**Step Reagent** *Supplier: Leica Microsystems*
7 *Bond Wash Solution
Step type: Wash | Incubation time: 0s | Temperature: Ambient | Dispense type: Selected vol.

---

**Step Reagent** *Supplier: Leica Microsystems*
8 *Bond Wash Solution
Step type: Wash | Incubation time: 0s | Temperature: Ambient | Dispense type: Selected vol.

---

12/02/2014 11:23 1/4
Leica BOND™

Leica MICROSYSTEMS

Full name: MRC & Enh 60'
ID: 10033
Version: 0(current)
Type: IHC staining
Created by: BondPowerUser
Creation time: 28/11/2011 13:32
Facility: MRC
Staining status: Single

**Protocol: MRC+E60**

**Bond-max and Bond-x**

**Step Reagent** — *Supplier: Leica Microsystems*

9 *Post Primary

Step type: Reagent | Incubation time: 8min0s | Temperature: Ambient | Dispense type: Selected vol.

**Step Reagent** — *Supplier: Leica Microsystems*

10 *Bond Wash Solution

Step type: Wash | Incubation time: 2min0s | Temperature: Ambient | Dispense type: Selected vol.

**Step Reagent** — *Supplier: Leica Microsystems*

11 *Bond Wash Solution

Step type: Wash | Incubation time: 2min0s | Temperature: Ambient | Dispense type: Selected vol.

**Step Reagent** — *Supplier: Leica Microsystems*

12 *Bond Wash Solution

Step type: Wash | Incubation time: 2min0s | Temperature: Ambient | Dispense type: Selected vol.

**Step Reagent** — *Supplier: Leica Microsystems*

13 *Polymer

Step type: Reagent | Incubation time: 8min0s | Temperature: Ambient | Dispense type: Selected vol.

**Step Reagent** — *Supplier: Leica Microsystems*

14 *Bond Wash Solution

Step type: Wash | Incubation time: 2min0s | Temperature: Ambient | Dispense type: Selected vol.

**Step Reagent** — *Supplier: Leica Microsystems*

15 *Bond Wash Solution

Step type: Wash | Incubation time: 2min0s | Temperature: Ambient | Dispense type: Selected vol.

**Step Reagent** — *Supplier:*

16 *Deionized Water

Step type: Wash | Incubation time: 0s | Temperature: Ambient | Dispense type: Selected vol.

12/02/2014 11:23 Leica BOND™ 2/4

Leica MICROSYSTEMS

Full name: MRC & Enh 60'
ID: 10033
Version: 0(current)
Type: IHC staining
Created by: BondPowerUser
Creation time: 28/11/2011 13:32
Facility: MRC
Staining status: Single

## Protocol: MRC+E60

### Bond-max and Bond-x

**Step Reagent** *Supplier: Leica Microsystems*
17 *Mixed DAB Refine
Step type: Reagent | Incubation time: 0s | Temperature: Ambient | Dispense type: Selected vol.

**Step Reagent** *Supplier: Leica Microsystems*
18 *Mixed DAB Refine
Step type: Reagent | Incubation time: 10min0s | Temperature: Ambient | Dispense type: Selected vol.

**Step Reagent** *Supplier:*
19 *Deionized Water
Step type: Wash | Incubation time: 0s | Temperature: Ambient | Dispense type: Selected vol.

**Step Reagent** *Supplier:*
20 *Deionized Water
Step type: Wash | Incubation time: 0s | Temperature: Ambient | Dispense type: Selected vol.

**Step Reagent** *Supplier:*
21 *Deionized Water
Step type: Wash | Incubation time: 0s | Temperature: Ambient | Dispense type: Selected vol.

**Step Reagent** *Supplier: Leica Microsystems*
22 *Bond DAB Enhancer
Step type: Reagent | Incubation time: 10min0s | Temperature: Ambient | Dispense type: Selected vol.

**Step Reagent** *Supplier: Leica Microsystems*
23 *Bond Wash Solution
Step type: Wash | Incubation time: 0s | Temperature: Ambient | Dispense type: Selected vol.

**Step Reagent** *Supplier: Leica Microsystems*
24 *Bond Wash Solution
Step type: Wash | Incubation time: 0s | Temperature: Ambient | Dispense type: Selected vol.

12/02/2014 11:23 3/4
Leica BOND™

Leica MICROSYSTEMS

| | |
|---|---|
| **Full name:** | MRC & Enh 60' |
| **ID:** | 10033 |
| **Version:** | 0(current) |
| **Type:** | IHC staining |
| **Created by:** | BondPowerUser |
| **Creation time:** | 28/11/2011 13:32 |
| **Facility:** | MRC |
| **Staining status:** | Single |

# Protocol: MRC+E60

## Bond-max and Bond-x

**Step Reagent** *Supplier: Leica Microsystems*

25 *Bond Wash Solution

Step type: Wash | Incubation time: 0s | Temperature: Ambient | Dispense type: Selected vol.

**Step Reagent** *Supplier: Leica Microsystems*

26 *Hematoxylin

Step type: Reagent | Incubation time: 5min0s | Temperature: Ambient | Dispense type: Selected vol.

**Step Reagent** *Supplier:*

27 *Deionized Water

Step type: Wash | Incubation time: 0s | Temperature: Ambient | Dispense type: Selected vol.

**Step Reagent** *Supplier: Leica Microsystems*

28 *Bond Wash Solution

Step type: Wash | Incubation time: 0s | Temperature: Ambient | Dispense type: Selected vol.

**Step Reagent** *Supplier:*

29 *Deionized Water

Step type: Wash | Incubation time: 0s | Temperature: Ambient | Dispense type: Selected vol.

12/02/2014 11:23 Leica BOND™ 4/4

Staining Protocol F

Leica MICROSYSTEMS

**Full name:** *IHC Protocol F
**ID:** 242
**Version:** 3(current)
**Type:** IHC staining
**Created by:** Leica
**Creation time:** 01/09/2010 14:13
**Facility:** MRC
**Staining status:** Single

**Protocol: *IHC F**

**Bond-max and Bond-x**

**Step Reagent** — *Supplier: Leica Microsystems*
1 *Peroxide Block
Step type: Reagent | Incubation time: 5min0s | Temperature: Ambient | Dispense type: Selected vol.

**Step Reagent** — *Supplier: Leica Microsystems*
2 *Bond Wash Solution
Step type: Wash | Incubation time: 0s | Temperature: Ambient | Dispense type: Selected vol.

**Step Reagent** — *Supplier: Leica Microsystems*
3 *Bond Wash Solution
Step type: Wash | Incubation time: 0s | Temperature: Ambient | Dispense type: Open

**Step Reagent** — *Supplier: Leica Microsystems*
4 *Bond Wash Solution
Step type: Wash | Incubation time: 0s | Temperature: Ambient | Dispense type: Selected vol.

**Step Reagent** — *Supplier: Not applicable*
5 Primary
Step type: Reagent | Incubation time: 15min0s | Temperature: Ambient | Dispense type: Selected vol.

**Step Reagent** — *Supplier: Leica Microsystems*
6 *Bond Wash Solution
Step type: Wash | Incubation time: 0s | Temperature: Ambient | Dispense type: Selected vol.

**Step Reagent** — *Supplier: Leica Microsystems*
7 *Bond Wash Solution
Step type: Wash | Incubation time: 0s | Temperature: Ambient | Dispense type: Selected vol.

**Step Reagent** — *Supplier: Leica Microsystems*
8 *Bond Wash Solution
Step type: Wash | Incubation time: 0s | Temperature: Ambient | Dispense type: Selected vol.

12/02/2014 11:22 — 1/4

Leica BOND™

Leica MICROSYSTEMS

Full name: *IHC Protocol F
ID: 242
Version: 3(current)
Type: IHC staining
Created by: Leica
Creation time: 01/09/2010 14:13
Facility: MRC
Staining status: Single

# Protocol: *IHC F

## Bond-max and Bond-x

**Step Reagent** *Supplier: Leica Microsystems*
9 *Post Primary
Step type: Reagent | Incubation time: 8min0s | Temperature: Ambient | Dispense type: Selected vol.

**Step Reagent** *Supplier: Leica Microsystems*
10 *Bond Wash Solution
Step type: Wash | Incubation time: 2min0s | Temperature: Ambient | Dispense type: Selected vol.

**Step Reagent** *Supplier: Leica Microsystems*
11 *Bond Wash Solution
Step type: Wash | Incubation time: 2min0s | Temperature: Ambient | Dispense type: Selected vol.

**Step Reagent** *Supplier: Leica Microsystems*
12 *Bond Wash Solution
Step type: Wash | Incubation time: 2min0s | Temperature: Ambient | Dispense type: Selected vol.

**Step Reagent** *Supplier: Leica Microsystems*
13 *Polymer
Step type: Reagent | Incubation time: 8min0s | Temperature: Ambient | Dispense type: Selected vol.

**Step Reagent** *Supplier: Leica Microsystems*
14 *Bond Wash Solution
Step type: Wash | Incubation time: 2min0s | Temperature: Ambient | Dispense type: Selected vol.

**Step Reagent** *Supplier: Leica Microsystems*
15 *Bond Wash Solution
Step type: Wash | Incubation time: 2min0s | Temperature: Ambient | Dispense type: Selected vol.

**Step Reagent** *Supplier:*
16 *Deionized Water
Step type: Wash | Incubation time: 0s | Temperature: Ambient | Dispense type: Selected vol.

12/02/2014 11:22 Leica BOND™ 2/4

Leica MICROSYSTEMS

Full name: *IHC Protocol F
ID: 242
Version: 5(current)
Type: IHC staining
Created by: Leica
Creation time: 01/09/2010 14:13
Facility: MRC
Staining status: Single

## Protocol: *IHC F

## Bond-max and Bond-x

**Step Reagent** *Supplier: Leica Microsystems*
17 *Mixed DAB Refine
Step type: Reagent Incubation time: 0s Temperature: Ambient Dispense type: Selected vol.

**Step Reagent** *Supplier: Leica Microsystems*
18 *Mixed DAB Refine
Step type: Reagent Incubation time: 10min0s Temperature: Ambient Dispense type: Selected vol.

**Step Reagent** *Supplier:*
19 *Deionized Water
Step type: Wash Incubation time: 0s Temperature: Ambient Dispense type: Selected vol.

**Step Reagent** *Supplier:*
20 *Deionized Water
Step type: Wash Incubation time: 0s Temperature: Ambient Dispense type: Selected vol.

**Step Reagent** *Supplier:*
21 *Deionized Water
Step type: Wash Incubation time: 0s Temperature: Ambient Dispense type: Selected vol.

**Step Reagent** *Supplier: Leica Microsystems*
22 *Hematoxylin
Step type: Reagent Incubation time: 5min0s Temperature: Ambient Dispense type: Selected vol.

**Step Reagent** *Supplier:*
23 *Deionized Water
Step type: Wash Incubation time: 0s Temperature: Ambient Dispense type: Selected vol.

**Step Reagent** *Supplier: Leica Microsystems*
24 *Bond Wash Solution
Step type: Wash Incubation time: 0s Temperature: Ambient Dispense type: Selected vol.

12/02/2014 11:22 3/4
Leica BOND™

Leica
MICROSYSTEMS

Full name: *IHC Protocol F
ID: 242
Version: 5(current)
Type: IHC staining
Created by: Leica
Creation time: 01/09/2010 14:13
Facility: MRC
Staining status: Single

**Protocol: *IHC F**

**Bond-max and Bond-x**

| Step | Reagent | | Supplier: |
|---|---|---|---|
| 25 | *Deionized Water | | |
| Step type: Wash | Incubation time: 0s | Temperature: Ambient | Dispense type: Selected vol. |

It should be noted that suitably cMYC is stained using MRC+E protocol but the antibody is incubated for 60 minutes; suitably AURKA is stained using the MRC+E protocol but the primary antibody is only incubated for 15 minutes.

**Scoring of immunohistochemical staining**

**p53** was scored as 0 - 3 (intensity of staining) with 3 being considered significant staining and 0, 1 or 2 non-significant staining. Only strong (intensity = 3) p53 staining was considered significant. p53 accumulation has been shown to correlate with Barrett's with dysplasia and also predict progression (Kaye et al., 2009; Skacel et al., 2002). The absent pattern (Kaye et al., 2010) was not counted as significant as the epithelial cells in Barrett's oesophagus frequently do not stain for p53.

**c-MYC** was scored as 0 - 3 (intensity of staining) with 0 and 1 being considered non-significant staining and 2 and 3 being considered significant staining. This cut off was selected as it was the most useful to discriminate between Barrett's with no dysplasia and Barrett's with any dysplasia. An example of c-MYC staining at the different intensities is found in Figure 1.

**AURKA** was scored as 0 or 1, with 0 being no staining and 1 being any positive staining. Examples of no staining and of positive staining are shown in Figure 2.

Suitably AURKA staining is nuclear. Suitably only nuclear staining is assessed in scoring AURKA. Suitably cytoplasmic staining (if any) is disregarded. Suitably AURKA staining according to the present invention is not cytoplasmic.

**Initial testing of the three protein biomarkers**

To further screen and ensure that the three potential protein biomarkers would perform successfully in our hands, p53, c-MYC and AURKA staining was performed on our in-house Barrett's tissue microarrays (TMAs). These TMAs consisted of 54 Barrett's biopsies with no dysplasia, 32 Barrett's biopsies with low grade dysplasia and 18 Barrett's biopsies with high grade dysplasia. As these TMAs were comprised of Barrett's biopsies, only the surface staining was scored as these are the cells that the Cytosponge™ would sample. In this dataset all three biomarkers performed well, as can be seen from the following table:

Table of sensitivity and specificity of p53, c-MYC and AURKA on our in-house Barrett's TMAs. The TMAs comprised of 54 Barrett's biopsies with no dysplasia, 32 Barrett's biopsies with low grade dysplasia and 18 Barrett's biopsies with high grade dysplasia:

| **Protein biomarker** | **Sensitivity (%)** | **Specificity (%)** |
|---|---|---|
| p53 | 54 | 100 |
| c-MYC | 79 | 96 |
| AURKA | 93 | 94 |

These confirmed markers were therefore taken forward to evaluate on the cell samples collected using the Cytosponge™.

**Example 6: Assay of marker - Methylation markers**

Methylation analysis on cells collected using the Cytosponge™ is carried out as follows: Genomic DNA was extracted from 8 x 10μm sections of the processed Cytosponge™ FFPE clot using Deparaffinization Buffer (Qiagen) and the QIAamp FFPE DNA Tissue Kit (Qiagen). The protocol was followed as described by the manufacturer with the exception that samples were incubated at 56°C for 24 hours instead of the described 1 hour, and 10 μl of extra Proteinase K was added to the samples roughly half way through the 24 hour incubation. After extraction, DNA was quantified using the Qubit™ dsDNA HS Assay Kits (Invitrogen) and 75 ng was bisulphite converted using the EZ DNA Methylation-Gold™ kit (as described by the manufacturer). Samples were eluted in 25 μl of water and 2 μl was used per MethyLight reaction as described in (Eads et al., 2000). β actin was used as an internal control to normalise for the amount of input DNA. The sequences of the primers and probes used were: MYOD1 forward primer: 5'-gagcgcgcgtagttagcg-3', MYOD1 reverse primer: 5'-tccgacacgccctttcc-3', MYOD1 probe: 5'-6FAM-ctccaacacccgactactatatccgcgaaa-TAMRA-3', ACTB forward primer: 5'-tggtgatggaggaggtttagtaagt-3', ACTB reverse primer: 5'-aaccaataaaacctactcctcccttaa-3', ACTB probe: 5'-6FAM-accaccacccaacacacaataacaaacaca-TAMRA-3' (from (Eads et al., 2001)), RUNX3 forward primer: 5'-ggcttttggcgagtagtggtc-3', RUNX3 reverse primer: 5'-acgaccgacgcgaacg-3', RUNX3 protein: 5'-6FAM-cgttttgaggttcgggtttcgtcgtt-TAMRA-3' from the Meltzer laboratory. Universally methylated DNA (D5010-1, Zymo Research) that had been bisulphite converted was used to derive standard curves for each of the primer and probe sets and a calibrator was used in all experiments to allow absolute quantification of the methylation levels in all samples. Amplification conditions used for all reactions were: 95°C for 10 mins followed by 50 cycles of 95°C for 15 seconds and 60°C for 1 minute.

The degree of methylation of each gene was calculated using the following formula:

$$\%\ methylation = (A/B)/(C/D)$$

A= value of methylation of gene of interest
B= value of methylation of the gene of interest in the fully methylated control
C= level of amplification of β actin in the sample
D= level of amplification of β actin in the fully methylated control

The % methylation of the two genes was then added together to give a methylation value.

**Initial testing of the methylated regions**

In a pilot experiment consisting of 113 Cytosponge™ samples (15 TFF3+ controls with no Barrett's esophagus, 54 Barrett's with no dysplasia, 20 Barrett's with LGD and 24 Barrett's with HGD), all five methylated regions (p16, HPP1, RUNX3, ESR1 and MYOD1) were assessed to see which subset of methylated regions performed the best and had the best sensitivity and specificity to detect dysplasia on the Cytosponge™, with the data presented in the following Table:

Table showing the area under the curve (AUC) for the five different methylation biomarkers. (15 TFF3+ controls with no Barrett's esophagus, 54 Barrett's with no dysplasia, 20 Barrett's with LGD and 24 Barrett's with HGD):

| Methylated gene | AUC |
|---|---|
| ESR1 | 0.739 |
| HPP1 | 0.754 |
| MYOD1 | 0.771 |
| p16 | 0.673 |
| RUNX3 | 0.754 |

Together RUNX3 and MYOD1 gave the best area under the curve when comparing any dysplasia with no dysplasia and were therefore taken forward to evaluate further on the Cytosponge™ samples.

**Example 7: Detection Of Surface Abnormality**

In this example we demonstrate a method of aiding detection of a surface abnormality in the oesophagus of a subject.

A sample of cells from the subject is provided. The sample comprises cells collected from the surface of the subject's oesophagus. In this example, the cells were collected by swallowing and retrieval of an abrasive cell collection device. In this example, the device is the Cytosponge™. Thus the cells were sampled from the surface of the subject's oesophagus.

The cells are assayed for at least two markers selected from

(i) p53;
(ii) c-Myc;
(iii) AURKA; and
(iv) methylation of MyoD and Runx3;

Performance of the risk stratification biomarkers on the BEST2 Cytosponge™ samples is demonstrated. In this example, after selecting the three protein biomarkers and the two-gene methylation panel as in the earlier examples, all four risk stratification biomarkers, optionally including fifth marker atypia, were tested on the BEST2 Cytosponge™ samples.

The data presented in this example are from 18 control patients, 95 Barrett's patients with no dysplasia, 25 Barrett's patients with LGD and 30 Barrett's patients with HGD.

Examples of lack of p53, c-MYC and AURKA staining in Barrett's with no dysplasia and significant, dark staining in Barrett's with dysplasia are shown in Figure 2. Figure 2 shows the panel of markers for detecting dysplasia on samples collected from the surface of the oesophagus such as by using the Cytosponge™. The panel of markers includes three protein biomarkers (p53, c-MYC and Aurora kinase A) and a two-gene methylation panel consisting of RUNX3 and MYOD1.

When comparing Barrett's with no dysplasia to Barrett's with HGD, p53, c-MYC and AURKA give a sensitivity of 57, 60 and 73%, respectively, and a specificity of 97, 89 and 85%, respectively. The percentage methylation of RUNX3 and MYOD1 when added together gave an area under the curve of 0.815 and a sensitivity and specificity of 83% and 80%, respectively, which is shown in the table under 'MethyLight':

Table of sensitivity and specificity values for the panel of risk stratification markers when comparing Barrett's with high grade dysplasia to Barrett's with no dysplasia:

| | **p53** | **c-MYC** | **AURKA** | **MethyLight** |
|---|---|---|---|---|
| **Sensitivity** | 57 | 60 | 73 | 83 |
| **Specificity** | 97 | 89 | 85 | 80 |

To assess the ability of this panel of risk stratification markers to detect dysplasia on the Cytosponge™ samples, a cut off of at least two positive biomarkers was used. Using these criteria, 27/30 (90%) of the patients with high grade dysplasia were detected and 16/25 (64%) of the patients with low grade dysplasia were detected (see table A below).

Table A shows how each of the risk stratification biomarkers perform individually as well as when the panel is used together to detect dysplasia on the Cytosponge™:

| Table A | **# patients** | **p53** | **c-MYC** | **AURKA** | **MethyLight** | **≥1 biomarker +** | **≥2 biomarkers+** |
|---|---|---|---|---|---|---|---|
| Controls | 16 | 0 | 1 | 4 | 0 | 6 (38%) | 0 (0%) |
| NDBE | 97 | 4 | 11 | 13 | 23 | 40 (41%) | 16 (16%) |
| LGD | 25 | 6 | 13 | 16 | 12 | 22 (88%) | 16 (64%) |
| HGD/IMC | 30 | 17 | 18 | 22 | 25 | 29 (97%) | 27 (90%) |

These data gave a specificity of 87% when comparing high grade dysplasia to no dysplasia (i.e. Barrett's with no dysplasia and controls).

Thus it is demonstrated that detection of abnormal levels of at least two of said markers infers that the subject has an increased likelihood of a surface abnormality in the oesophagus.

**Example 8: Alternate Approach - Cytosponge plus single marker**

In this example a method of aiding detection of a surface abnormality in the oesophagus of a subject, the method comprising:

a) providing a sample of cells from said subject, wherein said sample comprises cells collected from the surface of the subject's oesophagus;
wherein said sample of cells is collected using a swallowable abrasive device (such as a Cytosponge™) to sample the surface of the subject's oesophagus
b) assaying said cells for at least one marker selected from

(i) p53;
(ii) c-Myc;
(iii) AURKA; and
(iv) methylation of MyoD and Runx3;

wherein detection of abnormal levels of at least two of said marker infers that the subject has an increased likelihood of a surface abnormality in the oesophagus.

In this example, the method steps are performed as in example 7, but only one marker in the panel is required to be abnormal. Thus this represents a combination of the abrasive device/ Cytosponge approach with the panel of markers disclosed.

When relaxing the criteria and using a cut off of one positive biomarker, 29/30 (97%) of the high grade patients were detected and 23/35 (92%) of the low grade patients were detected (see Table A above). At this cut off the specificity is 59%. High sensitivity is essential for a biomarker panel used for surveillance so that patients at risk of invasive cancer are not missed. Even if the specificity is lower this is still useful since a significant proportion of patients are saved unnecessary endoscopy.

**Example 9: Further Applications**

These data indicate that the abrasive surface sampling (such as using Cytosponge™) together with a panel of biomarkers can be used to risk stratify BE patients. This has the advantage of enabling a decrease in the number of endoscopies required by BE patients. This also has the advantage of avoiding the sampling bias associated with biopsies. We propose that the abrasive surface sampling (such as using Cytosponge™) test together with the panel of risk biomarkers will alter (and provide technical benefits over) the current clinical practice (Figure 3 - flow diagram showing the current clinical pathway for patients with persistent dyspepsia or reflux). Currently patients who are symptomatic and experience persistent dyspepsia or reflux will be offered an endoscopy. If these patients are identified to have Barrett's oesophagus multiple biopsies will be taken for pathology. Depending on the diagnosis, the patients will be entered into the surveillance program and will be offered endoscopy coupled with biopsies at a determined time interval. If Barrett's with high grade dysplasia is detected they will be offered treatment.

As the majority of patients with Barrett's will never progress and will never develop Barrett's with dysplasia, these patients will have to tolerate an endoscopy every two years even though their risk of progressing is so low.

We propose that according to the invention these surveillance endoscopies coupled with biopsies can be advantageously **replaced** by a surveillance regime using the abrasive surface sampling (such as using Cytosponge™) test together with the panel of risk biomarkers described herein. For example, this is explained with reference to figure 4.

Figure 4 shows a flow diagram showing the proposed clinical/ screening pathway which includes the abrasive surface sampling (such as using Cytosponge™) together with the panel of biomarkers. Included are modelled numbers to demonstrate the number of endoscopies that will be avoided by using the Cytosponge™ as a screening and/or risk stratification tool according to the invention. These numbers are based on a screening population of 10,000 people and assume that 6.5% of this at risk population will have Barrett's oesophagus. The numbers also assume that 10% of the patients with Barrett's will have dysplasia. The number of patients at each stage depends on the marker's accuracy (sensitivity and specificity).

Figure 5 shows a flow diagram showing the proposed Barrett's surveillance pathway which includes the abrasive surface sampling (such as using Cytosponge™) together with the panel of biomarkers. Included are modelled numbers to demonstrate the number of endoscopies that will be avoided by using the Cytosponge™ as a risk stratification tool. The numbers also assume that 10% of the patients with Barrett's will have dysplasia. The number of patients at each stage depends on the marker's accuracy (sensitivity and specificity).

Figure 6 shows examples of p53 staining intensities.

Patients who are at high risk of having Barrett's oesophagus (i.e. patients with persistent reflux or dyspepsia) will be offered to undergo the tests described herein (eg. by surface sampling such as via swallowing the Cytosponge™) as part of a screening programme.

In one aspect, the sample may be pre-tested for the marker TFF3. If the test is negative for TFF3 (the Barrett's biomarker) the patient will be offered to take the pre-test again at a defined interval. Two negative Cytosponges™ means that the subject's risk of having Barrett's oesophagus is extremely low (- 0.2%) and therefore there is no clinical reason for the patient to have an endoscopy. In this case then optionally no risk biomarkers (ie. the test/panel of the invention) would be assayed for the patient's Cytosponge™ sample. The patient may be re-tested at a future date.

However, in another aspect, if either of the pretests are positive for TFF3 then the panel of risk biomarkers will be performed (assayed) using the abrasive surface sample (such as obtained using Cytosponge™) according to the invention. If none of the risk biomarkers in the described panel are positive the chance that the patient has any dysplasia is very low (0.6%) so they may be offered a retest in 2-5 years' time as part of a surveillance programme. If 1 or more of the biomarkers are positive the chance that the patient has dysplasia is much higher (11.3%, relative risk 19 times higher than if there are no positive biomarker) and they may be offered an endoscopy coupled with biopsies. These numbers show that the abrasive surface sampling (such as using Cytosponge™) together with the panel of risk biomarkers saves 56% (665/1184) of unnecessary endoscopies.

Thus the invention provides numerous technical and economic benefits as set out herein.

**Example 10 - Ordering of mutations in preinvasive disease stages of esophageal carcinogenesis**

In this example, the application of p53 mutation analysis at the nucleic acid level is demonstrated. In addition, the use of SMAD4 as a marker of EAC is demonstrated.

**Summary**

Cancer genome sequencing studies have identified numerous putative driver genes but the relative timing of mutations in carcinogenesis remains unclear. The gradual progression from pre-malignant Barrett's esophagus (BE) to esophageal adenocarcinoma (EAC) provides an ideal model to study the ordering of somatic mutations. We identified recurrently-mutated genes and assessed clonal structure using whole-genome sequencing and amplicon resequencing of 112 EACs. We next screened a cohort of 109 biopsies from two key transition points in the development of malignancy; benign metaplastic never-dysplastic Barrett's esophagus (NDBE, n=66), and high-grade dysplasia (HGD, n=43). Unexpectedly, the majority of recurrently mutated genes in EAC were also mutated in NDBE. Only *TP53* and *SMAD4* were stage-specific, confined to HGD and EAC, respectively. Finally, we applied this knowledge to identify high-risk BE in a novel non-endoscopic test. In conclusion, mutations in EAC driver genes generally occur exceptionally early in disease development with profound implications for diagnostic and therapeutic strategies.

**INTRODUCTION**

Most epithelial cancers develop gradually from pre-invasive lesions, in some instances after an initial metaplastic conversion. Research to characterize the genomic landscape of cancer has focused on established invasive disease with the goal of developing biomarkers for personalised therapy[1]. However, it is becoming increasingly clear that extensive genomic heterogeneity is present in the majority of advanced cancers[2]. The most appropriate therapeutic targets are therefore those mutations that occur early in the development of disease and are thus clonal in the resulting malignancy. The identification of causative mutations occurring early in pathogenesis is also pivotal to developing clinically useful biomarkers. In this context mutations occurring at disease-stage boundaries, for example, the transition from non-dysplastic epithelium to dysplasia, and then to cancer would be most informative. The evidence to date on the genetic evolution of cancer from pre-malignant lesions suggests that the accumulation of mutations is step-wise[3-5]. In the most well-studied example, the adenoma-dysplasia-colorectal adenocarcinoma progression sequence, it has been possible to assign timings for a limited number of candidate genes by comparative lesion sequencing[3]. More recent studies have sought to utilize statistical algorithms to infer the life history[4,5] of a tumor from single samples.

Esophageal adenocarcinoma (EAC) arises from metaplastic Barrett's esophagus (BE) in the context of chronic inflammation secondary to exposure to acid and bile[6,7]. BE lends itself well to studies of genetic evolution due to the repeated sampling of the mucosa during clinical surveillance prior to therapeutic intervention[8]. Previous studies of EAC and BE have generally used candidate gene approaches with the goal of identifying clinical biomarkers to complement histological examination, which is an approach fraught with difficulties[8,9]. Data from high-density single nucleotide polymorphism (SNP) arrays and exome-sequencing studies are now accumulating with a plethora of mutations identified in many different genes[10,11]. However, little work has yet focused on the precise ordering of these alterations in large cohorts of patients with pre-malignant disease and associated clinical follow-up data.

Recently Agrawal *et al.* performed exome sequencing on 11 EAC samples and two samples of BE adjacent to the cancer. Intriguingly, the majority of mutations were found to be present even in apparently normal BE[12] similar to the observation in colorectal adenocarcinoma. This raises the possibility that prior to the progression to malignancy mutations that predict the risk of progression may be detectable within cytologically benign tissue. However it is unclear to what extent the same mutations may be present in BE tissue from patients that have not progressed to cancer. This question is important as the majority of patients with BE will not progress to cancer, and somatic alterations occurring early, prior to dysplasia, are unlikely to provide clinically discriminatory biomarkers. Biomarker research in this area is critical since the current endoscopic surveillance strategies are increasingly recognized to be ineffective[13] and therefore novel approaches are required[14,15].

The aims of this study were: 1) identify a list of candidate, novel, recurrently-mutated genes in EAC; 2) to accurately resolve the stage of disease at which mutation occurs therefore providing insight as to the role of these recurrent mutations in cancer progression, and 3) test their utility in clinical applications, i.e. using the non-invasive, non-endoscopic, cell sampling device, the Cytosponge™.

## *RESULTS*

### *HIGH MUTATION BURDEN AND UNUSUAL MUTATIONAL SIGNATURE IN EAC*

The discovery cohort (22 EACs subject to WGS) reflected the known clinico-demographic features of the disease: male predominance (M:F, 4.5:1), a mean age of 68 years (range 53 to 82), and a majority with advanced disease (81.8% (18/22) > stage I). Of the 22 cases, 17 (77.3%) had evidence of BE in the resection specimen (Table 1).

**Table 1:** Demographics of the patient cohorts

| | | EAC cohorts | | BE cohorts | | *TP53* analysis on Cytosponge™ | | |
|---|---|---|---|---|---|---|---|---|
| | | Discovery | Validation | Never-dysplastic BE | BE with HGD | No BE Controls | Never-dysplastic BE | BE with HGD |
| Number | | 22 | 90 | 40 | 39 | 23 | 44 | 22 |
| Age (years) | | 68 (53-82) | 66 (32-83) | 63 (32-81) | 71 (50-87) | 53 (28-74) | 61 (41-85) | 66 (41-82) |
| Sex (M:F) | | 5:1 | 5 : 1 | 2 : 1 | 12:1 | 1 : 2 | 4 : 1 | 10 : 1 |
| Stage (%) | I | 4 (18.2) | 14 (15.6) | | | | | |
| | II | 6 (27.3) | 14 (15.6) | | | | | |
| | III | 11 (50.0) | 49 (54.4) | | | | | |
| | IV | 1 (4.5) | 4 (4.4) | | | | | |
| | n/a | 0 (0.0) | 9 (10.0) | | | | | |
| BE length (cm) | | | | 4.8 (1-9) | 8.6 (2-16) | | 5.8 (1-12) | 8.5 (4-16) |
| Follow up from EAC diagn osis (months) | | 28.5 (5-63) | 18 (1-134) | | | | | |
| Total BE surveillance (months) | | | | 58 (4-132) | 1 (0-45) | | 56 (0-175) | 24 (0-180) |

* Data shown reflect mean (range) for age and BE length, number (percentage) for stage and median (range) for follow up from EAC diagnosis and total BE surveillance. Sex ratio rounded to the nearest whole number.

Samples were sequenced to a mean coverage of 63- and 67-fold in tumor and normal samples, respectively.

We identified a median of 16,994 somatic SNVs (range: 4,518-56,528) and 994 small indels per sample (range 262-3,573). From this final dataset a total of 1,086 coding region mutations were subject to verification as part of a larger pipeline bench marking study. We used ultra-deep targeted re-sequencing, achieving a median coverage of >13,000 fold, and confirmed 1,081 (99.5%) as somatic. Using Sanger sequencing, 23/25 (92%) indels were verified as real and somatic. As observed by Dulak *et al* in the intervening time since our study commenced", the most frequent mutation type across the discovery cohort was T:A>G:C transversions with a striking enrichment at CTT trinucleotides. This enrichment for T:A>G:C transversions differentiates EAC from other cancers that have been studied by WGS, including

breast, colorectal and hepatocellular[16-18].

## TARGETED AMPLICON RESEQUENCING IN A VALIDATION COHORT OF EACs

To identify novel genes involved in the development of EAC in BE, we sought to identify recurrently mutated targets in our discovery cohort (n=22 cases). A final list of 26 genes that were either mutated significantly above the background rate or in pathways of interest were selected and tested in a larger cohort (90 additional EACs, Table 1), using targeted amplicon re-sequencing. The findings confirmed and extended those of our discovery cohort and previous work from others[11,12,19], including the identification of recurrent mutations in the SWI/SNF complex, such as *ARID1A*. Analysis of *ARID1A* protein expression loss by immunohistochemistry in a cohort of 298 additional EACs identified absent or decreased expression in 41% (122/298). This suggests alternative mechanisms of down regulation may be present though we did not identify any large-scale structural variants within the WGS data from our discovery cohort (data not shown).

We next combined the data from both the discovery and validation cohorts and identified 15 genes that were mutated in four or more samples (Figure 8). These included those previously identified as EAC candidate genes, and several novel candidates: *MYO18B, SEMA5A* and *ABCB1. TP53* was mutated in the majority of cases; however 31% of cases are wild type for *TP53.* Although we do not have enough power to detect mutually-exclusive mutations in our cohort, we can detect significantly co-occurring mutations. *SEMA5A* and *ABCB1* mutations occurred more commonly in the same tumor than would be expected by chance (Benjamini-Hochberg-adjusted p-value = 0.0021) although the reason for this association remains unclear.

## SIMILAR MUTATION FREQUENCY ACROSS BARRETT'S ESOPHAGUS DISEASE STAGES

The stage specificity of mutations can be derived from patients at discrete stages of BE carcinogenesis. Mutations occurring at disease-stage boundaries would be candidate biomarkers of malignant progression. In addition, mutations occurring early in the development of disease should represent ideal targets for novel therapeutic interventions due to their presence in the majority of cells in more advanced lesions due to clonal expansion early in the natural history. We therefore sought to identify the mutation status of the 26 genes in our panel in BE samples obtained from a prospective cohort of patients undergoing endoscopic surveillance. This included 109 BE biopsies from 79 patients (Figure 7). We selected 66 never-dysplastic BE samples from 40 BE patients for whom there was no evidence for progression to dysplasia or malignancy (median follow-up time 58 months, range 4-132), and 43 BE biopsy samples (from 39 patients) of histopathologically confirmed high grade dysplasia (HGD), the stage just prior to the development of invasive EAC (Table 1). We did not include low-grade dysplasia due to the poor agreement on the histopathological grading of this lesion[20].

The findings were striking and unexpected. For the never-dysplastic BE cohort, 21/40 (53%) patients were found to have mutations within their BE segment (Figure 9a), with several biopsies containing multiple mutations. In total, we identified 29 SNVs and 7 indels within this cohort. Importantly, the mutations identified in never-dysplastic BE occurred in several genes previously identified as drivers in EAC[11,19] and other cancers[21,22], including *SMARCA4, ARID1A,* and *CNTNAP5* (Figure 9b). Of interest, seven of these 29 SNVs were mutations at T:A base pairs. Of these, 5/7 (71%) occurred at TT dinucleotide sequences, the mutational context identified as highly enriched in the EAC WGS data. Thus, this mutational process may well be active at the earliest stages of disease. Of the 43 HGD biopsy samples, 39 (91%) were found to have mutations in at least one of the genes in our panel with a total of 67 SNVs and 7 indels.

Hence, rather than the frequency of mutation in a given gene increasing across disease stages, we observed that for the vast majority of genes the mutational frequency was not significantly different between never-dysplastic BE, HGD and EAC (Fisher's exact test with Benjamini-Hochberg correction for multiple testing, Figure 9b). Only *TP53* (p<0.0001) and *SMAD4 p=0.0061*) (Figure 9b and c) exhibited mutational frequencies that would distinguish between disease stages and thus identify progression towards malignancy. *TP53* was found to be recurrently mutated in both HGD (72%) and EAC (69%) samples, but only in a single case (2.5%) of never-dysplastic BE. *SMAD4* was mutated at a lower frequency (13%) and intriguingly was only found in EAC, the invasive stage of disease.

## Clonal analysis of recurrent mutations

Having identified the occurrence of mutations in the earliest stages of disease development we next sought to identify whether these mutations were fully-clonal or sub-clonal in our original discovery cohort of 22 EACs. For each of the 15 genes mutated in ≥4 samples from our expanded cohort we combined our high-depth resequencing of SNVs, copy number variant data and LOH analysis to determine the fraction of tumor cells containing the mutation. If mutation occurs at the earliest stage of disease development, prior to the clonal expansion of the malignancy, we would expect that the mutation would be present in all cells of the tumor. For 7/15 genes; *SMAD4, TP53, ARID1A, SMARCA4, TLR4,*

*CDKN2A* and *PNLIPRP3* this was the case. Mutation in the other 8 genes (*MYO18B, TRIM58, CNTNAP5, ABCB1, PCDH9, UNC13C* and *CCDC102B*) was not always present in the major clone, suggesting that mutation of these genes may be selected for at multiple stages of tumorigenesis (Figure 9d)

## APPLICATION OF KNOWLEDGE ON MUTATIONAL ORDERING TO A DIAGNOSTIC TEST

The current clinical strategy for patients with BE involves regular endoscopic examinations to try and identify patients with dysplasia who are at high risk of progression to adenocarcinoma. This approach is highly controversial due to the inherent difficulties in accurate identification of dysplastic lesions, and recent data suggest that endoscopic surveillance of BE is not effective[13,23]. The difficulties involved in endoscopic surveillance for BE include sampling bias inherent in random biopsies protocols and the subjective and time-consuming histopathological diagnosis of dysplasia. We therefore developed a novel approach which has the potential to overcome these limitations of BE surveillance. The strategy comprises a non-endoscopic device called the Cytosponge™ which can be provided to patients in the primary care setting. This device collects cells from the entire esophageal mucosa, thus avoiding sampling bias and can be combined with objective biomarkers for diagnosis[24,25]. To date our focus has been on a biomarker for diagnosing BE, however, since most BE patients will not progress to EAC, this BE biomarker needs to be combined with a biomarker (or a panel of biomarkers) to identify the high-risk dysplastic patients. From the aforementioned sequencing data, TP53 mutations fit the criteria of a good risk stratification candidate marker, since *TP53* mutations discriminate between patients with and without high grade dysplasia, the key point of therapeutic intervention. Though the device samples abnormal tissue, the majority of cells collected are from normal gastric glandular tissue at the top of the stomach as well as normal squamous areas of the esophagus, and therefore any mutant DNA would theoretically be in the minority, requiring a very sensitive assay (Figure 10a). This situation is analogous to the detection of tumor cell-free DNA in blood as a biomarker in advanced malignant disease: sensitive assays have been developed to detect extremely low levels of mutant DNA against normal background[26,27]. We therefore took an analogous approach to detecting mutations in Cytosponge™ material.

To determine whether mutations within BE lesions could be detected in material collected from the Cytosponge™, we first tested mutations previously identified in endoscopic BE biopsies. Four patients with HGD dysplasia had *TP53* mutations and had also swallowed the Cytosponge™ (twice in patient 4). For all four patients, the specific *TP53* mutations were detected at an allele fraction (proportion of variant reads) of between 0.04 and 0.24 (Figure 10b).

We then tested whether we could detect unknown *TP53* mutations within material collected using the Cytosponge™ as this would be required for a clinical test. We amplified the majority of the coding region of TP53 (1019/1182 bp (86%)) by multiplexed PCR and sequenced the amplified DNA by massively-parallel sequencing. *TP53* mutations were called *de novo* using TAm-Seq[26] on samples from control patients (no BE), BE patients with no dysplasia as well as BE patients with high grade dysplasia. As we expected, no *TP53* mutations were identified in samples from control patients or BE patients with no dysplasia (Figure 10c), demonstrating 100% specificity in differentiating between patients with HGD and no dysplasia. In contrast, *TP53* mutations were identified in 19/22 (86%) HGD patients. The allele fractions of the *TP53* mutations varied widely (between 0.006 to 0.357) but anything in this range can be called successfully and mutations were mostly clustered in the DNA binding domain, as expected (Figure 10d).

## DISCUSSION

BE is the only known precursor lesion of EAC, co-occurring in >80% of cases presenting *de novo*[28], however the majority of BE patients will never progress to invasive disease[29]. There is therefore a need for sensitive and specific biomarkers that can identify those patients at risk of progression. As long ago as the Nowell hypothesis, a stepwise selection of genomic mutations has been assumed necessary for cancer development[30]. Somatic genomic variants should therefore be highly sensitive and specific markers of disease stage. By screening for our panel of recurrently-mutated genes in a cohort of patients with BE who had never developed dysplasia, and a cohort of those with HGD, we hoped to identify a step-wise accumulation of mutations across these disease stages. Surprisingly we identified numerous mutations occurring in never-dysplastic BE at detectable allele fractions (>10%). Intriguingly the most prevalent gene mutations in EAC were also present at a similar frequency in BE and HGD samples, including mutations within cancer-associated genes, for example *ARID1A* and *SMARCA4,* members of the SWI/SNF complex.. These data demonstrate the complex mutational landscape that may be present even within tissue with a very low risk of malignant progression which has an entirely benign histopathological appearance. The exact role of these mutations at such an early stage of disease development remains unclear. However, it is known that clonal expansions occur frequently in BE and it is possible that these mutations provide an increase in fitness of a clone without leading to disruption of the epithelial architecture or providing the necessary cellular characteristics for invasion. A similar observation has been reported in endometrial cancer. In the normal population -35% of women harbour PTEN mutant glands in their endometrial tissue yet the lifetime risk of endometrial cancer is ~2.5%[31].

Our result has substantial implications for the specificity of tests aiming to use highly sensitive detection of mutations for the early diagnosis of malignancy[32]. Biomarkers predicting individuals at risk for cancer need to have substantial predictive power to distinguish between those who will and will not develop cancer. In our study almost all recurrently mutated genes in EAC, including *ABCB1, CNTNAP5, MYO18B* amongst others, are ruled out for use as surveillance tools for progression risk. Only mutation in *TP53* and *SMAD4* accurately defined the boundaries of disease states. The fact that mutation of *SMAD4* was only found in EAC provides a clear genetic distinction between EAC and HGD. However, the low frequency of SMAD4 mutation (13%) makes it a sub-optimal candidate for biomarker development. Furthermore, HGD, rather than EAC, is now the ideal point of clinical intervention due to the advent of endoscopic therapy. We therefore focused on *TP53* for the proof-of-principle Cytosponge™ study. Sequencing technologies are now being introduced to routine clinical use, and genes of interest can be sequenced rapidly and with exquisite sensitivity, providing a quantitative read-out[26]. We detected mutations in 86% of HGD Cytosponge™ samples using a simple, clinically applicable test. To improve the sensitivity of any early detection programme, it will also be key to identify the genetic or epigenetic changes that drive HGD and EAC in the minority of patients without a detectable *TP53* mutation. In addition, since genetic diversity has been shown to predict progression to BE it maybe possible to perform somatic mutation testing looking at both presence and relative proportions of mutations in a panel of genes, to identify patients with high-risk disease[33]..

In conclusion, never-dysplastic BE harbours frequent mutations affecting recurrently-mutated genes in EAC. Given the low rate of progression to malignant disease in never-dysplastic BE, the role of these mutations on the road to malignancy is unclear. It is generally accepted that the mutations observed in a tumor are accrued in a linear progression with each step bringing the clone closer to the invasive endpoint. Our observation of mutations in almost all of the recurrently-mutated genes in the tissue of patients who have not gone on to develop malignancy argues against a major role of these mutations in the progression towards cancer. Though their recurrent nature suggests a role in clonal expansion at the pre-malignant stage they do not seem to provide any long term increase in the likelihood of malignant progression.

From a clinical perspective, because the vast majority of recurrently-mutated genes in EAC do not differentiate between the pre-malignant and malignant stages of disease, they therefore cannot be applied in a simple binary test, i.e. mutant or non-mutant, as biomarkers of malignant progression. The Cytosponge™ provides a representative sample of the entire esophageal mucosa and coupled with high-throughput sequencing is capable of sensitive and objective detection of HGD. This approach could be readily adapted as our understanding of the genetic basis for this disease evolves. Furthermore, our systematic molecular approach to identify key mutations involved in the steps distinguishing pre-inasive from invasive disease has applicability to other epithelial cancers amenable to early detection.

**Methods**

**Sample Collection, Pathology Review and Extraction.**

The study was approved by the Institutional Ethics Committees (REC Ns 07/H0305/52 and 10/H0305/1) and all patients gave individual informed consent. For the discovery cohort, esophageal adenocarcinoma (EAC) patients were recruited prospectively and samples were obtained either from surgical resection or endoscopic ultrasound (EUS). Blood or normal squamous oesophageal samples, distant at least 5cm from the tumor, were used as germline reference. All tissue samples were snap-frozen in liquid nitrogen immediately after collection and stored at -80°C. Prior to DNA extraction, one section was cut from each oesophageal tissue sample and H&E staining was performed. Cancer samples were deemed suitable for DNA extraction only after consensus review by two expert pathologists had confirmed tumor cellularity ≥70%. Where blood was not available the same review process was applied to the normal esophageal samples to ensure that only squamous epithelium was present. For the Discovery cohort 127 cases were screened from two centers (Cambridge and Southampton). 63 cases had 70% cellularity required to meet ICGC criteria and of these 22 tumor:normal pairs had sufficient quality and quantity of DNA extracted (total yield ≥ 5μg), and were submitted for whole genome sequencing. From the remaining 105 cases available, 90 had >50% cellularity and all of these had sufficient DNA for the amplicon sequencing. For all cases in the discovery and validation cohort there was a 260/280 ratio 1.8-2.1. For the pre-invasive disease cohort we screened our entire 10 year prospective Barrett's cohort of >500 patients and selected cases in which there was frozen material available and for which review of the frozen section revealed a homogeneous grade of dysplasia following expert histopathological review. The Cytosponge™ samples were all those available as part of an interim analysis from an ongoing prospective case-control study (BEST2).

DNA was extracted from frozen esophageal tissue using the DNeasy kit (Qiagen) and from blood samples using the Nucleon™ Genomic Extraction kit (Gen-Probe) according to the manufacturer's instructions. For validation DNA was extracted using the AllPrepDNA/RNA Mini Kit (Qiagen) according to the manufacturer's instructions.

**Whole Genome Sequencing**

A single library was created for each sample, and 100bp paired-end sequencing was performed under contract by Illumina to a typical depth of at least 50x, with 94% of the known genome being sequenced to at least 8x coverage while achieving a PHRED quality of at least 30 for at least 80% of mapping bases. Typically, 5 lanes of a HiSeq-2000 (Illumina) flow cell achieved this, but samples were not multiplexed, so some exceeded these minimum standards by a large margin. Filtered read sequences were mapped to the human reference genome (GRCh37) using Burrows-Wheeler Alignment (BWA)[1], and duplicates marked using Picard (http://picard.sourceforge.net). As part of an extensive quality assurance process, QC metrics and alignment statistics were computed on a per lane basis. Aggregated QC for each discovery cohort sample was determined. Details of any tiles within flow cells that were removed post-QC was determined.

The FastQCpackage(http://www.bioinformatics.babraham.ac.uk/projects/fastqc/) was used to assess the quality score distribution of the sequencing reads, and enabled the identification of three lanes of sequencing that required trimming due to a drop in quality in the later cycles of sequencing.

**WGS Mutation Calling**

Somatic single nucleotide variants (SNVs) were predicted using SomaticSniper V1.0.2[2] run with the following command:

somaticsniper -q 1-Q 15 -F vcf -J -r 0.001000 -T 0.850000 -N 2 -s 0.01 -f

The output from SomaticSniper was then filtered using the following criteria derived from comparison of heuristic filters applied to SomaticSniper and VarScan 2[3] and implemented using scripts provided in Koboldt *et al*[3] and custom scripts (homopolymer filter).

1. Germline and Tumor sample coverage ≥10
2. Average variant position in read between positions 10 and 90
3. Percentage of supporting reads from each strand ≥1% and ≤99%
4. Total supporting reads ≥4
5. Average distance of variant base from effective 3' end of supporting reads ≥20 bp
6. Average mapping quality difference between reference and variant supporting reads <30
7. Average difference in length of trimmed sequences between reference and variant reads <25bp
8. Mismatch quality sum difference <100 between reference and variant reads
9. Adjacent homopolymer<5bp
10. Nearest indel≥ 40bp

In addition all variants were compared to dbSNP129 and removed if overlapping with predicted germline SNPs.A median of 99.7% of the mappable genome was covered to at least 10-fold coverage in the tumor and matched germline sample and so was defined as callable.

Candidate somatic indels were taken as the consensus between SAMtools[4] and Pindel[5], filtered to exclude those indels present in the matched normal genome of any of the 22 samples (including non-consensus indel calls). Indels falling within coding regions and splice sites were manually inspected to generate a final list of calls. Variants were annotated with sequence ontology terms to describe consequence and position relative to Ensembl gene annotations. SNVs and indels were also annotated with matching or nearest features in UniSNP.

**Verification of indel variants by PCR**

A total of 25 coding indels, confirmed by manual review, were randomly selected for verification. Primers (sequences available on request) were designed to amplify the predicted variant location. PCR was performed on both the tumor and normal DNA and the resulting products were Sanger sequenced. All traces were visualized using Chromas lite 2.01 and were manually reviewed for presence of the variant. An indel was considered somatic if it was present only in the tumor trace.

**Verification of single nucleotide variants by targeted re-sequencing**

As part of a larger benchmarking exercise of our SNV calling pipeline we selected 2007 SNVs to be verified. These SNVs included those that had failed filters and those that had been predicted using the Illumina pipeline, ELAND alignment plus STRELKA. The complete analysis of these data is ongoing with the overall aim of optimizing the sensitivity of our SNV calling pipeline. Following a preliminary analysis and comparison to the ICGC benchmarking exercise we chose to increase the stringency of our filters for this pilot dataset (detailed above). The verification data in this manuscript is for only those SNVs passing these additional filters. Putative non-synonymous SNVs (1330 in total) underwent ultra-high-depth targeted sequencing. For eight samples all non-synonymous variants were sent for verification. In the remaining 14 cases, the selected SNVs were restricted to non-synonymous variants in genes mutated in more than one sample. Amplicons were generated, indexed and pooled, and libraries constructed as per Shah *et al*[6]. Samples were pooled separately and a single lane of HiSeq-2000 data was generated for each, leading to a typical depth of coverage of 13,855 (IQR:3,408 to 39,059 for the amplicons). For 1086 of these ≥50-fold coverage was generated for both tumor and normal. An SNV was confirmed as somatic if the variant allele frequency was ≤ 1% in the matched normal and ≥ 2% in the tumor, and 108 1 SNVs met these criteria giving a verification rate of 108 1/1086 (99.5%).

**Mutation validation in independent samples**

Mutation validation was performed in a cohort of 90 additional EACs and 109 BEbiopsies, including 43BE biopsies with histopathologically confirmed HGD and 66 with no dysplasia. The Access Array microfluidics PCR platform (Fluidigm) together with high-throughput sequencing (Illumina) was used for the targeted re-sequencing.

Amplicons with a median size of 180bp (range 100-200bp) were designed using Fluidigm in-house software (primers available on request)[7]. After two iterations of primer design, one gene remained uncovered by suitable amplicons (*DIRC3*) and this was removed from further analysis. Hence, in total 26 genes were selected. All primers were synthesised with universal sequences (termed CS1 and CS2) appended at the 5'-end.

Target amplification and sample barcoding was performed using the manufacturer's standard multiplex protocol (Fluidigm, Access Array User Guide). Primers were combined into multiplex pools ranging from 1 to 12 primer pairs. The Access Array system was used to combine PCR reagents (FastStart High Fidelity PCR System, Roche) with 47 DNA samples (50ng) plus a single negative control and 48 sets of multiplexed primers into 2,304 unique 35nL PCR reactions. Thermal cycling was then applied to amplify all selected targets by PCR. Post-PCR, a harvesting reagent was used to collect the amplified products of the 48-multiplex reactions, per sample, through the sample inlets, for subsequent sequencing. Illumina sequencing adaptors and a 10bp sample specific barcode were attached through an additional 15 cycles of PCR. After the PCR products were barcoded, the PCR products from a small number of samples, as well as the water controls, were analyzed using the Agilent 2100 BioAnalyzer to ensure the expected amplicon size was obtained and that there was no contamination across the PCR reactions. They were then pooled together and purified using AMPure XP beads using a bead to amplicon ratio of 1.8:1.0. The library was quantified using the Agilent BioAnalyzer and subjected to Illumina cluster generation. One-hundred to 150bp paired-end sequencing was performed on aHiSeq 2000 or MiSeq with a 10-base indexing (barcode) read, using custom sequencing primers targeted to the CS1 and CS2 tags for both read1, read 2 (index read) and read 3, according to manufacturer's recommendations.

Methods used for analysis of targeted sequencing data generated using TAm-Seq have been reported previously[7]. Reads were de-multiplexed using a known list of barcodes allowing zero mismatches. Each set of reads was aligned independently to the hg19 reference genome using BWA in the paired-end mode[1]. Using expected genomic positions, each set of aligned reads was separated further into its constituent amplicons. A pileup was generated for each amplicon using SAMtools v1.17[4]. Using a base quality and a mapping quality cut-off of 30, observed frequencies of non-reference alleles for every sequenced locus across all amplicons and barcodes were calculated. For each locus and base, the distribution of non-reference background allele frequencies/reads was modeled and the probability of obtaining the observed frequency/number of reads (or greater) was calculated. Putative substitutions were identified based on a probability cut-off (confidence margin) of 0.9995. Known SNPs obtained from the 1000 Genomes project, dbSNP version 135 and regions covering amplification primers were discarded. Any substitutions observed at >5% allele frequency in more than half of the sequenced samples were discarded. Small insertions and deletions of sequence were predicted using GATK. All remaining putative mutations were annotated with sequence ontology terms to describe consequence and position relative to Ensembl gene annotations. In the final list, all nonsense or missense exonic mutations and splicing mutations with an allele frequency of 10% or greater at loci covered at least 100-fold were retained. Three genes were removed at this stage due to poor sequence coverage in all samples, *TLR1, TLR7* and *TLR9,* leaving a total of 23 genes for further analysis.

In order to verify the called mutations, all nonsynonymous mutations identified from the Fluidigm Access Array sequencing were re-amplified using the CS1-/CS2-tagged primer pair targeting the region and DNA from the original sample. Where available, DNA from a matched normal sample (blood, duodenum or normal squamous epithelium)was also amplified using the identical, tagged primer pair. Amplification was performed in 5 μl reactions (0.1 Phusion® High-Fidelity DNA Polymerase(New England BioLabs), 1x Phusion Buffer, 4.5 mM $MgCl_2$, 5% DMSO, 0.2 mM dNTPs, 1 μM forward and reverse primer, 25 ng genomic DNA. The PCR cycling conditions were as follows; 50°C for 2 minutes, 70°C for 20 minutes, 95°C for 10 minutes followed by 10 cycles of 95°C for 15 seconds, 60°C for 30 seconds and 72°C for 1 minute, followed by 2 cycles of 95°C for 15 seconds, 80°C for 30 seconds, 60°C for 30 seconds and 72°C for 1 minute, followed by 8 cycles of 95°C for 15 seconds, 60°C for 30 seconds and 72°C for 1 minute followed by 2 cycles of 95°C for 15 seconds, 80°C for 30 seconds, 60°C for 30 seconds and 72°C for 1 minute, and 8 cycles of 95°C for 15 seconds, 60°C for 30 seconds and 72°C for 1 minute followed by 5 cycles of 95°C for 15 seconds, 80°C for 30seconds, 60°C for 30seconds and 72°C 1minute. Following amplification, 2μl of each PCR reaction were collected and pooled in batches of 12 reactions such that only unique amplicons were contained within each pool. Thereafter, 5μl of the pooled reaction mix was added to 2μlof ExoSAP-IT® (Affymetrix). The samples were incubated at 37°C for 15 minutes followed by 80°C for 15 minute. The resulting product was diluted 1:100 in sterile water and Illumina sequencing adaptors and a 10bp barcode was attached to each pool using an additional 15 cycles of PCR (0.1 unit Phusion® High-Fidelity DNA Polymerase(New England BioLabs), 1x Phusion Buffer, 4.5mM $MgCl_2$, 5% DMSO, 0.2mMdNTPs, 1 μM forward and reverse barcoding primers, 1μl ExoSAP-IT®-treated PCR product (1:100 dilution). Cycling conditions were as follows: heat activation at 95°C for 2 minutes, followed by 15 cycles of 95°C for 15 seconds, 60°C for 30 seconds and 72°C for 1 minute, followed by a final elongation step of 72°C for 3 minutes.

As previously, PCR products following barcoding were first analyzed using an Agilent 2100 BioAnalyzer to ensure the expected amplicon size was obtained. They were then pooled together and purified using AMPure XP beads using a bead to amplicon ratio of 1.8 to 1.0. The library was quantified using the KAPA-Library Quantification Kit (KAPA Biosystems) on a Lightcycler® 480 (Roche), diluted to 2nM and subjected to Illumina cluster generation and sequencing on the Illumina MiSeq (150bp paired-end). Reads were de-multiplexed using a known list of barcodes allowing zero mismatches. Each set of reads was aligned independently to the hg19 reference genome using BWA in the paired-end mode[1]. Samtoolsmpileupv1.17[4] was used to generate counts for each nucleotide at the position of the putative somatic mutation. Samples with a mutant allele frequency ≥3% and a depth of coverage ≥ 50 were considered as verified mutations. In addition, mutant allele frequency in the matched normal was required to be <1%. We additionally removed all mutations from those samples without a matched normal that were confirmed as germline in the cohort of samples with sequenced matched normal.

**Processing of the capsule sponge specimens**

Cytosponge™ capsules were swallowed by patients and then placed directly into preservative solution at 4°C until processed further. The samples were vortexed extensively and shaken vigorously to remove any cells from the sponge material. The preservative liquid containing the cells was centrifuged at 1000 RPM for 5 minutes to pellet the cells. The resulting pellet was re-suspended in 500 μL of plasma and thrombin (Diagnostic reagents, Oxford, UK) was then added in 10 μL increments until a clot formed. The clot was then placed in formalin for 24h prior to processing into a paraffin block. Eight times ten micrometer sections were cut and placed in a tube for DNA extraction.

**DNA extraction from the Cytosponge samples**

Genomic DNA was extracted from 8 x 10μm sections of the processed Cytosponge™ FFPE clot using Deparaffinization Buffer (Qiagen) and the QIAamp FFPE DNA Tissue Kit (Qiagen). The protocol was followed as described by the manufacturer with the exception that samples were incubated at 56°C for 24 hours instead of the described 1 hour, and 10 μl of extra Proteinase K was added to the samples roughly half way through the 24 hour incubation. After extraction, DNA was quantified using the Qubit™ dsDNA HS Assay Kits (Invitrogen)

**Sequencing for TP53 mutations**

A multiplex TP53 PCR assay was used to sequence the coding region of the TP53 gene. The multiplex consisted of 14 primer pairs[7] and these 14 primer pairs were divided into two different pools. The sequences of each of the primers, the genomic region that they amplify (co-ordinates are correct for the hg19 version of the human genome) as well as which pool they were part of are described in Table 12 and 13.

All p53 multiplex PCRs were performed in duplicate using Q5 Hot Start High-Fidelity 2X Master Mix (New England Biolabs). The coding region of the TP53 gene was first amplified using a PCR mix consisting of: 1 x Q5 master mix, 5% DMSO, final concentration of 50 nM of each primer pair and up to 70 ng of FFPE DNA extracted from Cytosponge samples. The cycling conditions for the PCR were: Initial denaturation at 95°C for 30 seconds followed by 30 cycles of 95°C for 10 seconds, 60°C for 10 seconds and 72°C for 15 seconds. A final extension at 72°C for 2 minutes was also included to ensure elongation of all PCR products.

After the first round of PCR, 2.5 ul of Pool 1 and 2.5ul of Pool 2 were pooled together. Two microlitres of IllustraExostar 1-Step (GE Healthcare UK Ltd) was added to the 5 ul of pooled PCR products and the Exostar reaction was performed (15 minutes at 37°C followed by 15 minutes at 80°C) to degrade the primers from the first reaction. One microlitre of the pooled, Exostar-treated products was then added to the barcode PCR in order to add a unique barcode as well as add the sequencing primers onto the PCR products. The barcodes used for this second PCR were taken from Forshew et al[7] and the core sequence of the barcode primers can be found in Table 14. The Fluidigm barcode primers were used as they contain a sequence that binds to the CS1 and CS2 sequences present in the first p53 primers as well as the Illumina adapters. The barcode PCR mix consisted of 1 x Q5 master mix, 5% DMSO, final concentration of 400 nM of each barcode primer pair and 1 ul of undiluted, Exostar-treated DNA. The cycling conditions for the PCR were: Initial denaturation at 98°C for 30 seconds followed by 14 cycles of 98°C for 10 seconds, 60°C for 10 seconds and 72°C for 30 seconds. A final extension at 72°C for 2 minutes was also included to ensure elongation of all PCR products.

**TAm-seq SNV and indel calling for detecting TP53 mutations on Cytosponge™ samples**

Indels were called by selecting outliers from locus-specific distributions of background mutation rates. Candidate insertions and deletions in each sample were compared with insertion and deletion rates at the same locus in samples from every other patient, and scored by means of z-scores. Indels with a z-score greater than or equal to 10, at least 200x coverage and at least 5 supporting reads were retained.

**REFERENCES FOR METHODS OF EXAMPLE 10**

1. Li, H. & Durbin, R. Fast and accurate short read alignment with Burrows-Wheeler transform. Bioinformatics 25, 1754-60 (2009).
2. Larson, D.E. et al. SomaticSniper: identification of somatic point mutations in whole genome sequencing data. Bioinformatics 28, 311-7 (2012).
3. Koboldt, D.C. et al. VarScan 2: somatic mutation and copy number alteration discovery in cancer by exome sequencing. Genome Res 22, 568-76 (2012).
4. Li, H. et al. The Sequence Alignment/Map format and SAMtools. Bioinformatics 25, 2078-9 (2009).
5. Ye, K., Schulz, M.H., Long, Q., Apweiler, R. & Ning, Z. Pindel: a pattern growth approach to detect break points of large deletions and medium sized insertions from paired-end short reads. Bioinformatics 25, 2865-71 (2009).
6. Shah, S.P. et al. The clonal and mutational evolution spectrum of primary triple-negative breast cancers. Nature 486, 395-9 (2012).
7. Forshew, T. et al. Noninvasive identification and monitoring of cancer mutations by targeted deep sequencing of plasma DNA. Sci Transl Med 4, 136ra68 (2012).

References for Example 10

1. Chin, L., Andersen, J.N. & Futreal, P.A. Cancer genomics: from discovery science to personalized medicine. Nat Med 17, 297-303 (2011).
2. Gerlinger, M. et al. Intratumor heterogeneity and branched evolution revealed by multiregion sequencing. N Engl J Med 366, 883-92 (2012).
3. Jones, S. et al. Comparative lesion sequencing provides insights into tumor evolution. Proc Natl Acad Sci U S A 105, 4283-8 (2008).
4. Nik-Zainal, S. et al. The life history of 21 breast cancers. Cell 149, 994-1007 (2012).
5. Vogelstein, B. et al. Genetic alterations during colorectal-tumor development. N Engl J Med 319, 525-32 (1988).
6. Goh, X.Y. et al. Integrative analysis of array-comparative genomic hybridisation and matched gene expression profiling data reveals novel genes with prognostic significance in oesophageal adenocarcinoma. Gut 60, 1317-26 (2011).
7. Quante, M. et al. Bile acid and inflammation activate gastric cardia stem cells in a mouse model of Barrett-like metaplasia. Cancer Cell 21, 36-51 (2012).
8. Greaves, M. & Maley, C.C. Clonal evolution in cancer. Nature 481, 306-13 (2012).
9. Varghese, S., Lao-Sirieix, P. & Fitzgerald, R.C. Identification and clinical implementation of biomarkers for Barrett's esophagus. Gastroenterology 142, 435-441 e2 (2012).
10. Dulak, A.M. et al. Gastrointestinal Adenocarcinomas of the Esophagus, Stomach, and Colon Exhibit Distinct Patterns of Genome Instability and Oncogenesis. Cancer Res (2012).
11. Dulak, A.M. et al. Exome and whole-genome sequencing of esophageal adenocarcinoma identifies recurrent driver events and mutational complexity. Nat Genet 45, 478-86 (2013).
12. Agrawal, N. et al. Comparative genomic analysis of esophageal adenocarcinoma and squamous cell carcinoma. Cancer Discov (2012).
13. Corley, D.A. et al. Impact of Endoscopic Surveillance on Mortality From Barrett's Esophagus-Associated Esophageal Adenocarcinomas. Gastroenterology 145, 312-319 e1 (2013).
14. Shaheen, N.J. & Hur, C. Garlic, Silver Bullets, and Surveillance Upper Endoscopy for Barrett's Esophagus. Gastroenterology 145, 273-6 (2013).
15. Hayes, D.F. et al. Breaking a vicious cycle. Sci Transl Med 5, 196cm6 (2013).
16. Nik-Zainal, S. et al. Mutational processes molding the genomes of 21 breast cancers. Cell 149, 979-93 (2012).
17. Fujimoto, A. et al. Whole-genome sequencing of liver cancers identifies etiological influences on mutation patterns and recurrent mutations in chromatin regulators. Nat Genet 44, 760-4 (2012).
18. Bass, A.J. et al. Genomic sequencing of colorectal adenocarcinomas identifies a recurrent VTI1A-TCF7L2 fusion. Nat Genet 43, 964-8 (2011).
19. Streppel, M.M. et al. Next-generation sequencing of endoscopic biopsies identifies ARID1A as a tumor-suppressor gene in Barrett's esophagus. Oncogene (2013).
20. Curvers, W.L. et al. Low-grade dysplasia in Barrett's esophagus: overdiagnosed and underestimated. Am J Gastroenterol 105, 1523-30 (2010).
21. Wang, K. et al. Exome sequencing identifies frequent mutation of ARID1A in molecular subtypes of gastric cancer. Nat Genet 43, 1219-23 (2011).
22. Jones, S. et al. Frequent mutations of chromatin remodeling gene ARID1A in ovarian clear cell carcinoma. Science 330, 228-31 (2010).
23. Reid, B.J., Li, X., Galipeau, P.C. & Vaughan, T.L. Barrett's oesophagus and oesophageal adenocarcinoma: time for a new synthesis. Nat Rev Cancer 10, 87-101 (2010).
24. Kadri, S.R. et al. Acceptability and accuracy of a non-endoscopic screening test for Barrett's oesophagus in primary care: cohort study. BMJ 341, c4372 (2010).
25. Lao-Sirieix, P. et al. Non-endoscopic screening biomarkers for Barrett's oesophagus: from microarray analysis to the clinic. Gut 58, 1451-9 (2009).
26. Forshew, T. et al. Noninvasive identification and monitoring of cancer mutations by targeted deep sequencing of plasma DNA. Sci Transl Med 4, 136ra68 (2012).
27. Dawson, S.J. et al. Analysis of circulating tumor DNA to monitor metastatic breast cancer. N Engl J Med 368, 1199-209 (2013).
28. Theisen, J. et al. Preoperative chemotherapy unmasks underlying Barrett's mucosa in patients with adenocarcinoma of the distal esophagus. Surg Endosc 16, 671-3 (2002).
29. Bhat, S. et al. Risk of malignant progression in Barrett's esophagus patients: results from a large population-based study. J Natl Cancer Inst 103, 1049-57 (2011).
30. Nowell, P.C. The clonal evolution of tumor cell populations. Science 194, 23-8 (1976).
31. Mutter, G.L. et al. Molecular identification of latent precancers in histologically normal endometrium. Cancer Res 61, 4311-4 (2001).
32. Kinde, I. et al. Evaluation of DNA from the Papanicolaou test to detect ovarian and endometrial cancers. Sci Transl Med 5, 167ra4 (2013).
33. Maley, C.C. et al. Genetic clonal diversity predicts progression to esophageal adenocarcinoma. Nat Genet 38, 468-73 (2006).

**Example 11: Statistical Analysis**

Here we show detailed, statistical analysis showing the effect of different biomarker combinations on sensitivity and specificity. The data look very good and assaying 4 biomarkers is certainly an advantage. There are 2 tables below: one for high grade dysplasia only and one for any dysplasia.

Table showing results from any dysplasia (low grade, high grade and indefinite):

| ID | EXPLANATORY | SPECIFICITY (MEAN) | SPECIFICITY (SD) | SENSITIVITY (MEAN) | SENSITIVITY (SD) | naivescore |
|---|---|---|---|---|---|---|
| 31 | **Atypia, p53, MYC, Methylation, Aurka** | 0.8409 | 0.0532 | 0.7546 | 0.1137 | 1.5955 |
| 30 | **p53, MYC, Methylation, Aurka** | 0.8546 | 0.0459 | 0.7395 | 0.1028 | 1.5942 |
| 27 | **Atypia, p53, MYC, Aurka** | 0.8768 | 0.0542 | 0.7067 | 0.1135 | 1.5836 |
| 16 | **Atypia, p53, MYC** | 0.8169 | 0.0469 | 0.7583 | 0.0997 | 1.5752 |
| 10 | **p53, MYC** | 0.8702 | 0.0413 | 0.6966 | 0.1015 | 1.5668 |
| 29 | **Atypia, MYC, Methylation, Aurka** | 0.8303 | 0.0498 | 0.7301 | 0.0989 | 1.5604 |
| 7 | **Atypia, MYC** | 0.8385 | 0.0447 | 0.7213 | 0.1045 | 1.5597 |
| 28 | **Atypia, p53, Methylation, Aurka** | 0.8488 | 0.0509 | 0.7081 | 0.1098 | 1.5568 |
| 24 | **p53, Methylation, Aurka** | 0.8825 | 0.0525 | 0.6629 | 0.1099 | 1.5454 |
| 23 | **p53, MYC, Aurka** | 0.8469 | 0.0978 | 0.6923 | 0.1179 | 1.5392 |
| 22 | **p53, MYC, Methylation** | 0.8498 | 0.0829 | 0.6879 | 0.1225 | 1.5377 |
| 20 | **Atypia, MYC, Aurka** | 0.8523 | 0.0757 | 0.6843 | 0.1180 | 1.5366 |
| 26 | **Atypia, p53, MYC, Methylation** | 0.8630 | 0.0515 | 0.6706 | 0.1176 | 1.5336 |
| 6 | **Atypia, p53** | 0.9045 | 0.0362 | 0.6243 | 0.1055 | 1.5288 |
| 19 | **Atypia, MYC, Methylation** | 0.8460 | 0.0600 | 0.6784 | 0.1198 | 1.5244 |
| 12 | **Atypia, Aurka** | 0.7258 | 0.0544 | 0.7945 | 0.0894 | 1.5203 |

| | | | | | | |
|---|---|---|---|---|---|---|
| 21 | **Atypia, Methylation, Aurka** | 0.8445 | 0.0484 | 0.6707 | 0.1109 | 1.5152 |
| 17 | **Atypia, p53, Methylation** | 0.8732 | 0.0924 | 0.6376 | 0.1325 | 1.5108 |
| 25 | **MYC, Methylation, Aurka** | 0.8568 | 0.0500 | 0.6509 | 0.1079 | 1.5077 |
| 18 | **Atypia, p53, Aurka** | 0.8615 | 0.1082 | 0.6447 | 0.1367 | 1.5062 |
| 11 | **p53, Methylation** | 0.7305 | 0.0549 | 0.7749 | 0.0932 | 1.5054 |
| 8 | **Atypia, Methylation** | 0.7041 | 0.0622 | 0.7903 | 0.1083 | 1.4943 |
| 13 | **MYC, Methylation** | 0.6966 | 0.0629 | 0.7953 | 0.1077 | 1.4920 |
| 14 | **MYC, Aurka** | 0.6802 | 0.0556 | 0.7760 | 0.0928 | 1.4562 |
| 9 | **Atypia, Aurka** | 0.6838 | 0.0588 | 0.7715 | 0.0979 | 1.4553 |
| 5 | **Aurka** | 0.7372 | 0.0522 | 0.7179 | 0.1026 | 1.4550 |
| 1 | **Atypia** | 0.9365 | 0.0297 | 0.5097 | 0.1110 | 1.4462 |
| 3 | **MYC** | 0.8919 | 0.0383 | 0.5450 | 0.1156 | 1.4369 |
| 4 | **Methylation** | 0.7366 | 0.0549 | 0.6984 | 0.1038 | 1.4350 |
| 15 | **Methylation, Aurka** | 0.6776 | 0.1117 | 0.7232 | 0.1621 | 1.4008 |
| 2 | **p53** | 0.9469 | 0.0218 | 0.4319 | 0.1172 | 1.3788 |

Table showing results from high grade dysplasia only:

| ID | EXPLANATORY | SPECIFICITY (MEAN) | SPECIFICITY (SD) | SENSITIVITY (MEAN) | SENSITIVITY (SD) | naivescore |
|---|---|---|---|---|---|---|
| 31 | **Atypia, p53, MYC, Methylation, Aurka** | 0.8918 | 0.0486 | 0.8061 | 0.1019 | 1.6979 |
| 27 | **Atypia, p53, MYC, Aurka** | 0.8894 | 0.0410 | 0.8010 | 0.0937 | 1.6904 |
| 28 | **Atypia, p53, Methylation, Aurka** | 0.8610 | 0.0416 | 0.8287 | 0.0930 | 1.6897 |
| 30 | **p53, MYC, Methylation, Aurka** | 0.8616 | 0.0355 | 0.8261 | 0.0894 | 1.6877 |
| 26 | **Atypia, p53, MYC, Methylation** | 0.8917 | 0.0379 | 0.7751 | 0.0882 | 1.6668 |
| 24 | **p53, Methylation, Aurka** | 0.8873 | 0.0327 | 0.7709 | 0.0840 | 1.6582 |
| 21 | **Atypia, Methylation, Aurka** | 0.8619 | 0.0417 | 0.7890 | 0.0809 | 1.6509 |

| | | | | | | |
|---|---|---|---|---|---|---|
| 10 | **p53, MYC** | 0.8724 | 0.0338 | 0.7779 | 0.0866 | 1.6503 |
| 20 | **Atypia, MYC, Aurka** | 0.8846 | 0.0545 | 0.7641 | 0.0939 | 1.6487 |
| 23 | **p53, MYC, Aurka** | 0.8957 | 0.0658 | 0.7492 | 0.1036 | 1.6449 |
| 6 | **Atypia, p53** | 0.9047 | 0.0282 | 0.7333 | 0.0866 | 1.6379 |
| 29 | **Atypia, MYC, Methylation, Aurka** | 0.8388 | 0.0416 | 0.7967 | 0.0890 | 1.6355 |
| 22 | **p53, MYC, Methylation** | 0.8936 | 0.0662 | 0.7343 | 0.1019 | 1.6278 |
| 17 | **Atypia, p53, Methylation** | 0.9103 | 0.0557 | 0.7146 | 0.1042 | 1.6249 |
| 18 | **Atypia, p53, Aurka** | 0.9089 | 0.0469 | 0.7135 | 0.1052 | 1.6224 |
| 16 | **Atypia, p53, MYC** | 0.8559 | 0.0605 | 0.7641 | 0.1069 | 1.6199 |
| 7 | **Atypia, MYC** | 0.8423 | 0.0355 | 0.7714 | 0.0927 | 1.6137 |
| 19 | **Atypia, MYC, Methylation** | 0.8829 | 0.0510 | 0.7216 | 0.1014 | 1.6045 |
| 25 | **MYC, Methylation, Aurka** | 0.8609 | 0.0391 | 0.7353 | 0.0956 | 1.5962 |
| 11 | **p53, Methylation** | 0.7378 | 0.0623 | 0.8497 | 0.1186 | 1.5875 |
| 8 | **Atypia, Methylation** | 0.7403 | 0.0997 | 0.8355 | 0.1704 | 1.5759 |
| 1 | **Atypia** | 0.9352 | 0.0232 | 0.6293 | 0.0974 | 1.5646 |
| 4 | **Methylation** | 0.7392 | 0.0445 | 0.8227 | 0.0791 | 1.5619 |
| 12 | **Atypia, Aurka** | 0.7339 | 0.0681 | 0.8258 | 0.1230 | 1.5597 |
| 13 | **MYC, Methylation** | 0.7108 | 0.0609 | 0.8478 | 0.1263 | 1.5586 |
| 2 | **p53** | 0.9680 | 0.0169 | 0.5566 | 0.1017 | 1.5246 |
| 5 | **Aurka** | 0.7375 | 0.0416 | 0.7720 | 0.0858 | 1.5095 |
| 15 | **Methylation, Aurka** | 0.8010 | 0.1103 | 0.7080 | 0.1336 | 1.5090 |
| 9 | **Atypia, Aurka** | 0.7360 | 0.1153 | 0.7642 | 0.1472 | 1.5002 |
| 14 | **MYC, Aurka** | 0.6855 | 0.0574 | 0.7861 | 0.1072 | 1.4715 |
| 3 | **MYC** | 0.8915 | 0.0302 | 0.5743 | 0.1029 | 1.4658 |

The score in the final column is the sum of sensitivity and specificity. It is still important to look at them separately and take into account the variance.

Thus marker combinations may be chosen to maximise sensitivity whilst minimising loss of specificity.

**Example 12**

In this example we show Performance of the risk stratification biomarkers to detect dysplasia on the Cytosponge™ LGD = low grade dysplasia HGD/IMC = high grade dysplasia/intramucosal cancer

| | # patients | Atypia | p53 | c-MYC | AURKA | MethyLight | ≥1 biomarker+ | ≥2 biomarkers+ |
|---|---|---|---|---|---|---|---|---|
| Non-dysplastic controls | 144 | 7 | 4 | 38 | 38 | 32 | 68 (47%) | 19 (13%) |
| LGD | 32 | 11 | 5 | 17 | 17 | 15 | 28 (88%) | 18 (56%) |
| HGD/IMC | 42 | 26 | 25 | 34 | 34 | 34 | 40 (95%) | 38 (90%) |

| **# Biomarkers positive** | **≥1** | **≥2** |
|---|---|---|
| **Sensitivity** | 95 | 90 |
| **Specificity** | 53 | 87 |

**Example 13**

In this example we show data on p53 IHC and nucleic acid (by sequencing) either separately or together.

| | # | TP53 mut detected | p53 significant stain (intensity = 3) | Both (mut and significant stain) | Either mut/sig stain/both | None (i.e. no mut or sig stain) |
|---|---|---|---|---|---|---|
| NDBE | 44 | 0 | 0 | 0 | 0 | 44 |
| HGD | 22 | 19 (86%) | 14 (64%) | 12 (55%) | 21 (95%) | 1 (5%) |

Although illustrative embodiments of the invention have been disclosed in detail herein, with reference to the accompanying drawings, it is understood that the invention is not limited to those precise embodiments and that various changes and modifications can be effected therein by one skilled in the art without departing from the scope of the invention as defined by the appended claims and their equivalents.

**References**

Bian, Y.S., Osterheld, M.C., Bosman, F.T., Benhattar, J., and Fontolliet, C. (2001). p53 gene mutation and protein accumulation during neoplastic progression in Barrett's esophagus. Mod Pathol 14, 397-403.

Eads, C.A., Danenberg, K.D., Kawakami, K., Saltz, L.B., Blake, C., Shibata, D., Danenberg, P.V., and Laird, P.W. (2000). MethyLight: a high-throughput assay to measure DNA methylation. Nucleic Acids Res 28, E32.

Eads, C.A., Lord, R.V., Wickramasinghe, K., Long, T.I., Kurumboor, S.K., Bernstein, L., Peters, J.H., DeMeester, S.R., DeMeester, T.R., Skinner, K.A., et al. (2001). Epigenetic patterns in the progression of esophageal adenocarcinoma. Cancer Res 61, 3410-3418. Kadri, S.R., Lao-Sirieix, P., O'Donovan, M., Debiram, I., Das, M., Blazeby, J.M., Emery, J., Boussioutas, A., Morris, H., Walter, F.M., et al. (2010). Acceptability and accuracy of a non-endoscopic screening test for Barrett's oesophagus in primary care: cohort study. BMJ 341, c4372.

Kastelein, F., Biermann, K., Steyerberg, E.W., Verheij, J., Kalisvaart, M., Looijenga, L.H., Stoop, H.A., Walter, L., Kuipers, E.J., Spaander, M.C., et al. (2012). Aberrant p53 protein expression is associated with an increased risk of neoplastic progression in patients with Barrett's oesophagus. Gut.

Kaye, P.V., Haider, S.A., Ilyas, M., James, P.D., Soomro, I., Faisal, W., Catton, J., Parsons, S.L., and Ragunath, K. (2009). Barrett's dysplasia and the Vienna classification: reproducibility, prediction of progression and impact of consensus reporting and p53 immunohistochemistry. Histopathology 54, 699-712.
Kaye, P.V., Haider, S.A., James, P.D., Soomro, I., Catton, J., Parsons, S.L., Ragunath, K., and Ilyas, M. (2010). Novel staining pattern of p53 in Barrett's dysplasia--the absent pattern. Histopathology 57, 933-935.
Lao-Sirieix, P., Brais, R., Lovat, L., Coleman, N., and Fitzgerald, R.C. (2004). Cell cycle phase abnormalities do not account for disordered proliferation in Barrett's carcinogenesis. Neoplasia 6, 751-760.
Lao-Sirieix, P., Lovat, L., and Fitzgerald, R.C. (2007). Cyclin A immunocytology as a risk stratification tool for Barrett's esophagus surveillance. Clin Cancer Res 13, 659-665.
Miller, C.T., Moy, J.R., Lin, L., Schipper, M., Normolle, D., Brenner, D.E., Iannettoni, M.D., Orringer, M.B., and Beer, D.G. (2003). Gene amplification in esophageal adenocarcinomas and Barrett's with high-grade dysplasia. Clin Cancer Res 9, 4819-4825.
Rugge, M., Fassan, M., Zaninotto, G., Pizzi, M., Giacomelli, L., Battaglia, G., Rizzetto, C., Parente, P., and Ancona, E. (2010). Aurora kinase A in Barrett's carcinogenesis. Hum Pathol 41, 1380-1386.
Rygiel, A.M., Milano, F., Ten Kate, F.J., Schaap, A., Wang, K.K., Peppelenbosch, M.P., Bergman, J.J., and Krishnadath, K.K. (2008). Gains and amplifications of c-myc, EGFR, and 20.q13 loci in the no dysplasia-dysplasia-adenocarcinoma sequence of Barrett's esophagus. Cancer Epidemiol Biomarkers Prev 17, 1380-1385.
Schulmann, K., Sterian, A., Berki, A., Yin, J., Sato, F., Xu, Y., Olaru, A., Wang, S., Mori, Y., Deacu, E., et al. (2005). Inactivation of p16, RUNX3, and HPP1 occurs early in Barrett's-associated neoplastic progression and predicts progression risk. Oncogene 24, 4138-4148.
Sikkema, M., Kerkhof, M., Steyerberg, E.W., Kusters, J.G., van Strien, P.M., Looman, C.W., van Dekken, H., Siersema, P.D., and Kuipers, E.J. (2009). Aneuploidy and overexpression of Ki67 and p53 as markers for neoplastic progression in Barrett's esophagus: a case-control study. Am J Gastroenterol 104, 2673-2680.
Skacel, M., Petras, R.E., Rybicki, L.A., Gramlich, T.L., Richter, J.E., Falk, G.W., and Goldblum, J.R. (2002). p53 expression in low grade dysplasia in Barrett's esophagus: correlation with interobserver agreement and disease progression. Am J Gastroenterol 97, 2508-2513.
Zhou, H., Kuang, J., Zhong, L., Kuo, W.L., Gray, J.W., Sahin, A., Brinkley, B.R., and Sen, S. (1998). Tumour amplified kinase STK15/BTAK induces centrosome amplification, aneuploidy and transformation. Nat Genet 20, 189-193.

**Claims**

**1.** An *in vitro* method of aiding detection of a surface abnormality in the oesophagus of a subject, wherein said surface abnormality is selected from the group consisting of low-grade dysplasia (LGD), high-grade dysplasia (HGD), asymptomatic oesophageal adenocarcinoma (OAC) and intra-mucosal cancer (IMC), the method comprising:

a) providing a sample of cells from said subject, wherein said sample comprises cells collected from the surface of the subject's oesophagus;
b) assaying said cells for at least four markers, at least one marker from each of subsections (i) to (iv), selected from

(i) p53;
(ii) c-Myc;
(iii) AURKA or PLK1, preferably AURKA; and
(iv) methylation of MyoD and Runx3;

wherein detection of abnormal levels of at least four of said markers infers that the subject has an increased likelihood of a surface abnormality in the oesophagus.

**2.** An *in vitro* method of aiding detection of a surface abnormality in the oesophagus of a subject, wherein said surface abnormality is selected from the group consisting of low-grade dysplasia (LGD), high-grade dysplasia (HGD), asymptomatic oesophageal adenocarcinoma (OAC) and intra-mucosal cancer (IMC), the method comprising:

a) providing a sample of cells from said subject, wherein said sample comprises cells collected from the surface of the subject's oesophagus;
b) assaying said cells for at least four markers selected from

(i) p53;
(ii) c-Myc;
(iii) AURKA; and

(iv) methylation of MyoD and Runx3;

wherein detection of abnormal levels of at least four of said markers infers that the subject has an increased likelihood of a surface abnormality in the oesophagus.

**3.** An *in vitro* method according to any preceding claim, further comprising assaying said cells for atypia, wherein atypia is assessed by scoring the cells for their morphology according to the Vienna Scale.

**4.** An *in vitro* method according to any preceding claim, wherein said cells are collected by unbiased sampling of the surface of the oesophagus.

**5.** An *in vitro* method according to claim 4, wherein said cells are collected using a capsule sponge.

**6.** An *in vitro* method according to any preceding claim, wherein the cells are prepared prior to being contacted with the reagents for detection of the molecular markers by the steps of (i) pelleting the cells by centrifuge, (ii) re-suspending the cells in plasma, and (iii) adding thrombin and incubating until a clot is formed, incubating said clot in formalin, processing into a paraffin block, and slicing into sections suitable for microscopic examination.

**7.** An *in vitro* method according to any of claims 1 to 6, wherein p53 is assessed by immunohistochemistry.

**8.** An *in vitro* method according to any of claims 1 to 6, wherein p53 is assessed by detection of one or more p53 mutation(s).

**9.** An *in vitro* method according to any of claims 1 to 6, wherein p53 is assessed by immunohistochemistry and wherein p53 is also assessed by detection of one or more p53 mutation(s).

**10.** An *in vitro* assay for selecting a treatment regimen, said assay comprising

a) providing a sample of cells from said subject, wherein said sample comprises cells collected from the surface of the subject's oesophagus;
b) assaying said cells for at least four markers selected from

(i) p53;
(ii) c-Myc;
(iii) AURKA; and
(iv) methylation of MyoD and Runx3;

wherein if abnormal levels of at least four of said markers are detected, then a treatment regimen of endoscopy and biopsy is selected.

**11.** An apparatus or system which is

(a) specifically adapted to analyse an oesophagal sample from a subject, wherein said analysis comprises
(b) assaying said cells for at least four markers selected from

(i) p53;
(ii) c-Myc;
(iii) AURKA; and
(iv) methylation of MyoD and Runx3;

said apparatus or system comprising an output module,
wherein if abnormal levels of at least four of said markers are detected, then said output module indicates an increased likelihood of a surface abnormality in the oesophagus for said subject, wherein said surface abnormality is selected from the group consisting of low-grade dysplasia (LGD), high-grade dysplasia (HGD), asymptomatic oesophageal adenocarcinoma (OAC) and intra-mucosal cancer (IMC).

**12.** Use of an assay device according to the method of any of claims 1-9 for use in aiding detection of a surface abnormality in the oesophagus of a subject, wherein said surface abnormality is selected from the group consisting of low-grade dysplasia (LGD), high-grade dysplasia (HGD), asymptomatic oesophageal adenocarcinoma (OAC) and intra-mucosal cancer (IMC), which comprises a solid substrate having a location containing a material, which recognises, binds to or has affinity for certain polypeptides, or methylation of certain nucleic acid sequences, wherein the polypeptides and/or nucleic acid sequences are:

(i) p53;
(ii) c-Myc;
(iii) AURKA or PLK1, preferably AURKA; and
(iv) methylation of MyoD and Runx3.

**Patentansprüche**

1. *In vitro* Verfahren zur Unterstützung des Feststellens einer Oberflächenanomalie im Ösophagus eines Subjekts, wobei die Oberflächenanomalie ausgewählt ist aus der Gruppe, bestehend aus niedrig gradiger Dysplasie (LGD), hochgradiger Dysplasie (HGD), asymptomatischem Adenokarzinom des Ösophagus (OAC) und intramukosalem Krebs (IMC), das Verfahren umfasst:

   a) Bereitstellen einer Probe von Zellen von dem Subjekt, wobei die Probe Zellen umfasst, welche der Oberfläche des Ösophagus des Subjekts entnommen sind;
   b) Testen der Zellen auf mindestens vier Marker, mindestens einem Marker von jedem der folgenden Absätze (i) bis (iv), ausgewählt aus:

      (i) p53;
      (ii) c-Myc;
      (iii) AURKA oder PLK1, vorzugsweise AURKA; und
      (iv) Methylierung von MyoD und Runx3;

   wobei aus dem Feststellen von abnormalen Spiegeln von mindestens vier Markern erfolgt, dass bei dem Subjekt eine erhöhte Wahrscheinlichkeit für eine Oberflächenanomalie im Ösophagus vorliegt.

2. *In vitro* Verfahren zur Unterstützung des Feststellens einer Oberflächenanomalie im Ösophagus eines Subjekts, wobei die Oberflächenanomalie ausgewählt ist aus der Gruppe, bestehend aus niedrig gradiger Dysplasie (LGD), hochgradiger Dysplasie (HGD), asymptomatischem Adenokarzinom des Ösophagus (OAC) und intramukosalem Krebs (IMC), das Verfahren umfasst:

   a) Bereitstellen einer Probe von Zellen von dem Subjekt, wobei die Probe Zellen umfasst, welche der Oberfläche des Ösophagus des Subjekts entnommen sind;
   b) Testen der Zellen auf mindestens vier Marker, ausgewählt aus:

      (i) p53;
      (ii) c-Myc;
      (iii) AURKA; und
      (iv) Methylierung von MyoD und Runx3;

   wobei aus dem Feststellen von abnormalen Spiegeln von mindestens vier Markern erfolgt, dass bei dem Subjekt eine erhöhte Wahrscheinlichkeit für eine Oberflächenanomalie im Ösophagus vorliegt.

3. *In vitro* Verfahren gemäß einem der vorstehenden Ansprüche, welche des Weiteren das Testen der Zellen auf Atypien umfasst, wobei die Atypien durch Bewerten der Zellen hinsichtlich ihre Morphologie gemäß Wiener Skala beurteilt werden.

4. *In vitro* Verfahren gemäß einem der vorstehenden Ansprüche, wobei die Zellen durch unvoreingenommene Probenentnahme von der Oberfläche des Ösophagus gewonnen werden.

5. *In vitro* Verfahren gemäß Anspruch 4, wobei die Zellen unter Verwendung eines Kapselschwamms gewonnen werden.

**6.** *In vitro* Verfahren gemäß einem der vorstehenden Ansprüche, wobei die Zellen vor dem in Kontakt bringen mit den Reagenzien zum Feststellen der molekularen Marker durch folgende Schritte vorbereitet werden (i) Pelletieren der Zellen in der Zentrifuge; (ii) Resuspendieren der Zellen in Plasma und (iii) Hinzufügen von Thrombin und Inkubieren bis sich ein Klumpen bildet, Inkubieren des Klumpens in Formalin, Verarbeiten zu einem Paraffinblock und Anfertigen von Schnitten, welche zur mikroskopischen Untersuchung geeignet sind.

**7.** *In vitro* Verfahren gemäß einem der Ansprüche 1 bis 6, wobei p53 immunhistochemisch beurteilt wird.

**8.** *In vitro* Verfahren gemäß einem der Ansprüche 1 bis 6, wobei p53 durch Feststellen einer oder mehrerer p53-Mutation(en) beurteilt wird.

**9.** *In vitro* Verfahren gemäß einem der Ansprüche 1 bis 6, wobei p53 immunhistochemisch beurteilt wird und wobei p53 auch durch Feststellen einer oder mehrerer p53-Mutation(en) beurteilt wird.

**10.** In vitro Verfahren zum Auswählen eines Behandlungsschemas, wobei der Test umfasst

a) Bereitstellen einer Probe von Zellen von dem Subjekt, wobei die Probe Zellen umfasst, welche der Oberfläche des Ösophagus des Subjekts entnommen sind;
b) Testen der Zellen auf mindestens vier Marker, ausgewählt aus:

(i) p53;
(ii) c-Myc;
(iii) AURKA; und
(iv) Methylierung von MyoD und Runx3;

wobei, wenn abnormale Spiegel von mindestens vier Markern festgestellt werden, das Behandlungsschema Endoskopie und Biopsie ausgewählt wird.

**11.** Vorrichtung oder System, das

(a) spezifisch adaptiert ist, eine Ösophagusprobe von einem Subjekts zu untersuchen, wobei die Untersuchung umfasst
(b) Beurteilen von Zellen hinsichtlich mindestens vier Markern, ausgewählt aus

(i) p53;
(ii) c-Myc;
(iii) AURKA; und
(iv) Methylierung von MyoD und Runx3;

wobei die Vorrichtung oder das System ein Ausgangsmodul umfasst,
das, wenn abnormale Spiegel von mindestens vier Markern festgestellt werden, das Ausgangsmodul eine erhöhte Wahrscheinlichkeit für eine Oberflächenanomalie im Ösophagus für das Subjekt anzeigt, wobei die Oberflächenanomalie ausgewählt ist aus der Gruppe, bestehend aus niedrig gradiger Dysplasie (LGD), hochgradiger Dysplasie (HGD), asymptomatischem Adenokarzinom des Ösophagus (OAC) und intramukosalem Krebs (IMC).

**12.** Verwendung einer Testvorrichtung gemäß dem Verfahren nach einem der Ansprüche 1 bis 9 zur Verwendung bei der Unterstützung des Feststellens einer Oberflächenanomalie im Ösophagus eines Subjekts, wobei die Oberflächenanomalie ausgewählt ist aus der Gruppe, bestehend aus niedrig gradiger Dysplasie (LGD), hochgradiger Dysplasie (HGD), asymptomatischem Adenokarzinom des Ösophagus (OAC) und intramukosalem Krebs (IMC), die ein festes Substrat mit einer Stelle umfasst, welche ein Material enthält, das bestimmte Polypeptide oder die Methylierung bestimmter Nukleinsäuresequenzen erkennt, bindet oder eine Affinität dazu aufweist, wobei die Polypeptide und/oder Aminosäuresequenzen sind:

(i) p53;
(ii) c-Myc;
(iii) AURKA oder PLK1, vorzugsweise AURKA; und
(iv) Methylierung von MyoD und Runx3.

## Revendications

1. Procédé *in vitro* d'aide à la détection d'une anomalie de surface dans l'oesophage d'un sujet, dans lequel ladite anomalie de surface est choisie dans le groupe constitué de la dysplasie de bas grade (LGD), la dysplasie de haut grade (HGD), l'adénocarcinome oesophagien asymptomatique (OAC) et le cancer intra-muqueux (IMC), le procédé comprenant :

   a) la fourniture d'un échantillon de cellules provenant dudit sujet, dans lequel ledit échantillon comprend des cellules recueillies à partir de la surface de l'oesophage du sujet ;
   b) l'évaluation desdites cellules vis-à-vis d'au moins quatre marqueurs, au moins un marqueur de chacune des sous-sections (i) à (iv), choisi parmi

      (i) p53 ;
      (ii) c-Myc;
      (iii) AURKA ou PLK1, de préférence AURKA ; et
      (iv) la méthylation de MyoD et de Runx3 ;

   dans lequel de la détection de niveaux anormaux d'au moins quatre desdits marqueurs il est déduit que le sujet a une probabilité accrue d'une anomalie de surface dans l'oesophage.

2. Procédé *in vitro* d'aide à la détection d'une anomalie de surface dans l'oesophage d'un sujet, dans lequel ladite anomalie de surface est choisie dans le groupe constitué par la dysplasie de bas grade (LGD), la dysplasie de haut grade (HGD), l'adénocarcinome de l'oesophage asymptomatique (OAC) et le cancer intra-muqueux (IMC), le procédé comprenant :

   (a) la fourniture d'un échantillon de cellules provenant dudit sujet, dans lequel ledit échantillon comprend des cellules recueillies à partir de la surface de l'oesophage du sujet ;
   (b) l'évaluation desdites cellules vis-à-vis d'au moins quatre marqueurs choisis parmi

      (i) p53 ;
      (ii) c-Myc;
      (iii) AURKA; et
      (iv) la méthylation de MyoD et de Runx3 ;

   dans lequel de la détection de niveaux anormaux d'au moins quatre desdits marqueurs il est déduit que le sujet a une probabilité accrue d'une anomalie de surface dans l'oesophage.

3. Procédé *in vitro* selon l'une quelconque des revendications précédentes, comprenant en outre l'évaluation desdites cellules vis-à-vis de l'atypie, dans lequel l'atypie est évaluée en marquant les cellules pour leur morphologie selon l'échelle de Vienne.

4. Procédé *in vitro* selon l'une quelconque des revendications précédentes, dans lequel lesdites cellules sont recueillies par échantillonnage non biaisé de la surface de l'oesophage.

5. Procédé *in vitro* selon la revendication 4, dans lequel lesdites cellules sont recueillies en utilisant une éponge en capsule.

6. Procédé *in vitro* selon l'une quelconque des revendications précédentes, dans lequel les cellules sont préparées avant d'être mises en contact avec les réactifs pour la détection des marqueurs moléculaires par les étapes de (i) granulation des cellules par centrifugation, (ii) remise en suspension des cellules dans du plasma, et (iii) addition de thrombine et incubation jusqu'à la formation d'un caillot, incubation du caillot dans du formol, traitement dans un bloc de paraffine et découpage en sections appropriées pour un examen microscopique.

7. Procédé *in vitro* selon l'une quelconque des revendications 1 à 6, dans lequel p53 est évalué par immunohistochimie.

8. Procédé *in vitro* selon l'une quelconque des revendications 1 à 6, dans lequel p53 est évalué par détection d'une ou plusieurs mutation (s) de p53.

**9.** Procédé *in vitro* selon l'une quelconque des revendications 1 à 6, dans lequel p53 est évalué par immunohistochimie et dans lequel p53 est également évalué par détection d'une ou plusieurs mutation(s) de p53.

**10.** Evaluation *in vitro* pour sélectionner un protocole de traitement, ladite évaluation comprenant :

(a) fournir un échantillon de cellules provenant dudit sujet, dans lequel ledit échantillon comprend des cellules collectées à partir de la surface de l'oesophage du sujet ;
(b) évaluer lesdites cellules vis-à-vis d'au moins quatre marqueurs choisis parmi

(i) p53 ;
(ii) c-Myc;
(iii) AURKA ; et
(iv) la méthylation de MyoD et de Runx3 ;

dans lequel si des niveaux anormaux d'au moins quatre desdits marqueurs sont détectés, alors un protocole de traitement d'endoscopie et de biopsie est sélectionné.

**11.** Appareil ou système qui est :

(a) spécifiquement adapté à analyser un échantillon d'oesophage provenant d'un sujet, dans lequel ladite analyse comprend :
(b) l'évaluation desdites cellules vis-à-vis d'au moins quatre marqueurs choisis parmi

(i) p53 ;
(ii) c-Myc;
(iii) AURKA; et
(iv) la méthylation de MyoD et de Runx3 ;

ledit appareil ou système comprenant un module de sortie,
dans lequel si des niveaux anormaux d'au moins quatre desdits marqueurs sont détectés, alors ledit module de sortie indique une probabilité accrue d'une anomalie de surface dans l'oesophage pour ledit sujet, ladite anomalie de surface étant sélectionnée dans le groupe constitué par la dysplasie de bas grade (LGD), la dysplasie de haut grade (HGD), l'adénocarcinome de l'oesophage asymptomatique (OAC) et le cancer intra-muqueux (IMC).

**12.** Utilisation d'un dispositif d'évaluation selon l'une quelconque des revendications 1 à 9 pour aider à la détection d'une anomalie de surface dans l'oesophage d'un sujet, ladite anomalie de surface étant choisie dans le groupe constitué par le dysplasie de bas grade (LGD), la dysplasie de haut grade (HGD), l'adénocarcinome oesophagien asymptomatique (OAC) et le cancer intra-muqueux (IMC), qui comprend un substrat solide ayant une localisation contenant un matériau, qui reconnaît, se lie à ou présente une affinité pour certains polypeptides, ou la méthylation de certaines séquences d'acide nucléique, dans lequel les polypeptides et/ou les séquences d'acide nucléique sont :

(i) p53 ;
(ii) c-Myc;
(iii) AURKA ou PLK1, de préférence AURKA ; et
(iv) la méthylation de MyoD et Runx3.

FIGURE 1

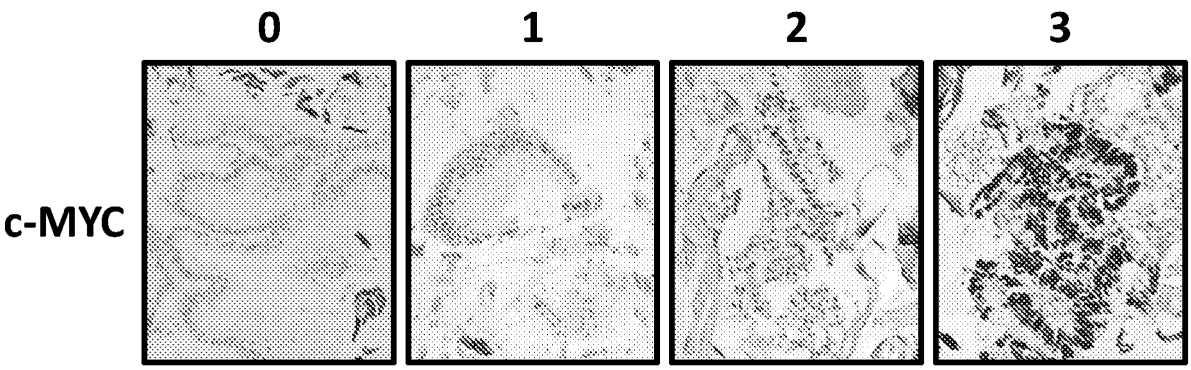


FIGURE 2

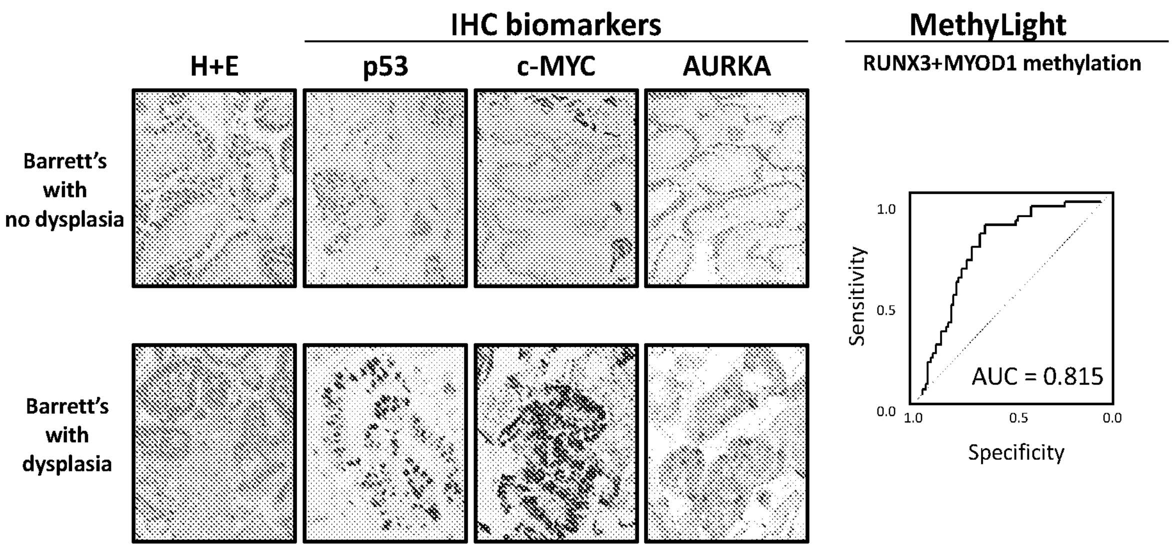

FIGURE 3

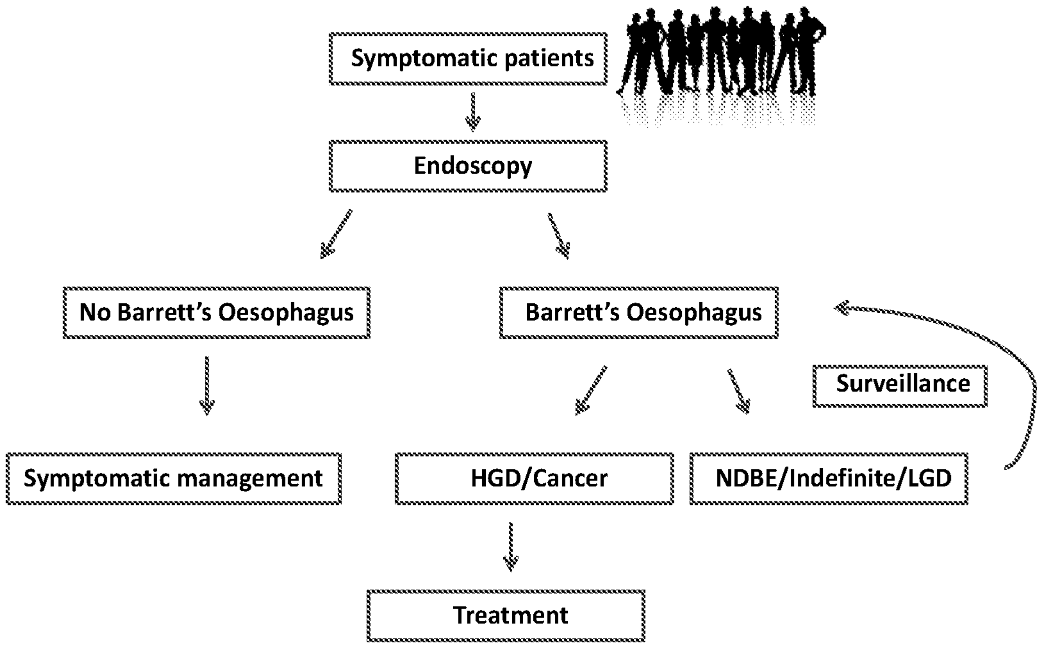


FIGURE 4

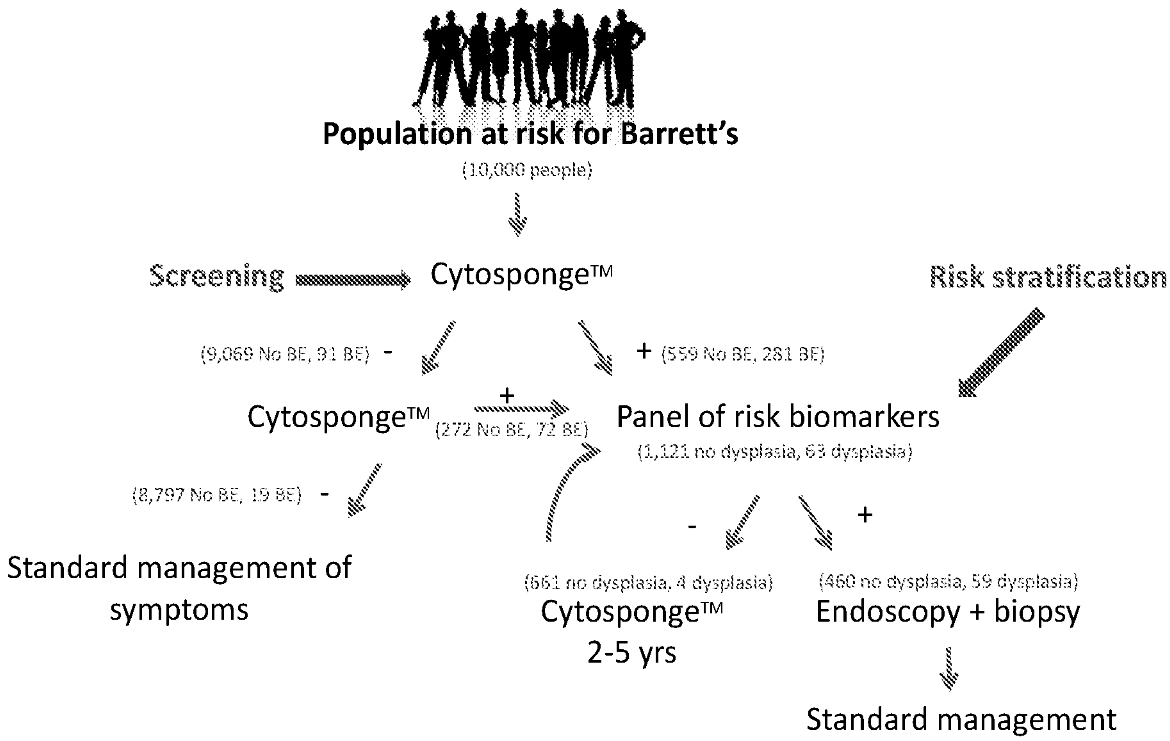

FIGURE 5

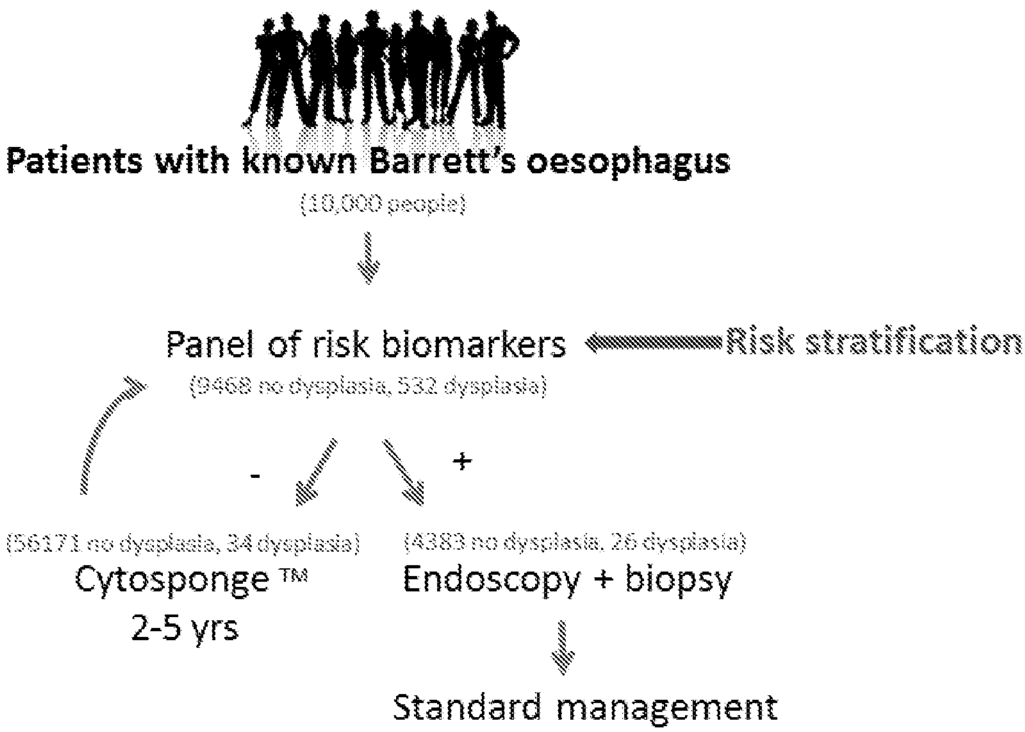
Patients with known Barrett's oesophagus
(10,000 people)
Panel of risk biomarkers
Risk stratification
(9468 no dysplasia, 532 dysplasia)
-
+
(56171 no dysplasia, 34 dysplasia)
Cytosponge ™
2-5 yrs
(4383 no dysplasia, 26 dysplasia)
Endoscopy + biopsy
Standard management

FIGURE 6

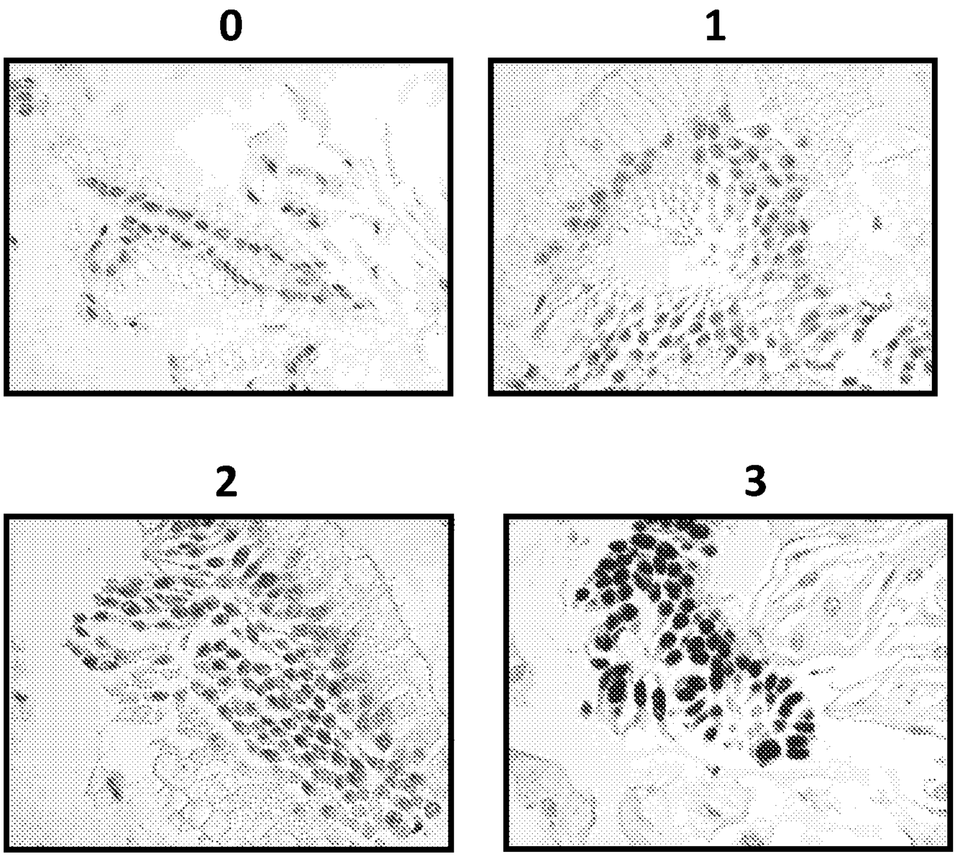

Figure 7

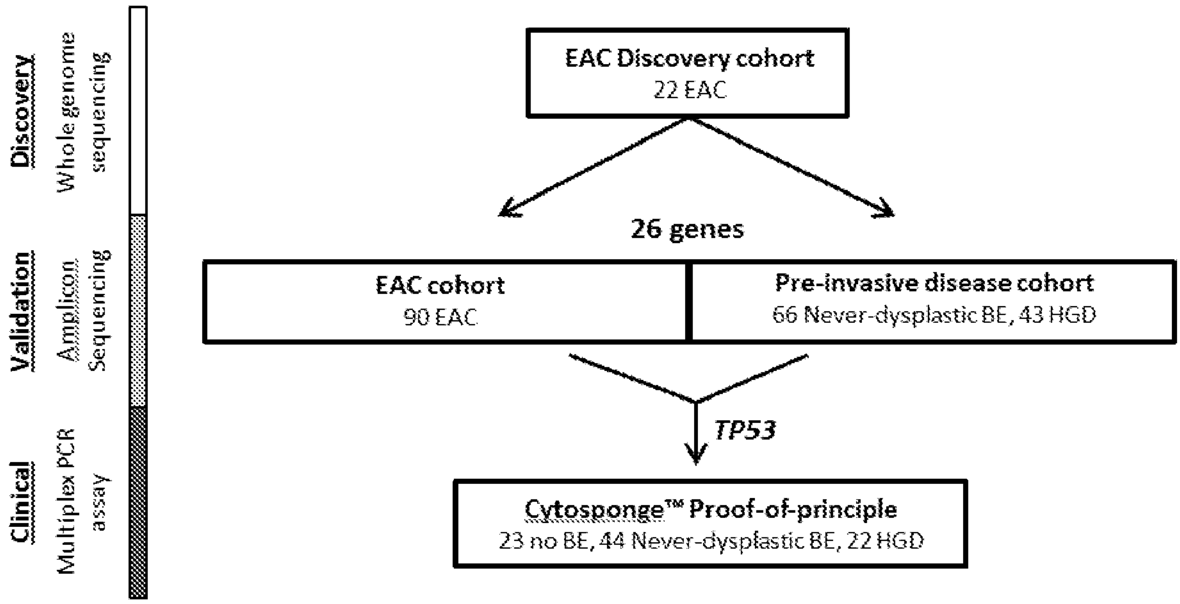
Discovery
Whole genome sequencing
Validation
Amplicon Sequencing
Clinical
Multiplex PCR assay
EAC Discovery cohort
22 EAC
26 genes
EAC cohort
90 EAC
Pre-invasive disease cohort
66 Never-dysplastic BE, 43 HGD
TP53
Cytosponge™ Proof-of-principle
23 no BE, 44 Never-dysplastic BE, 22 HGD

# Figure 8

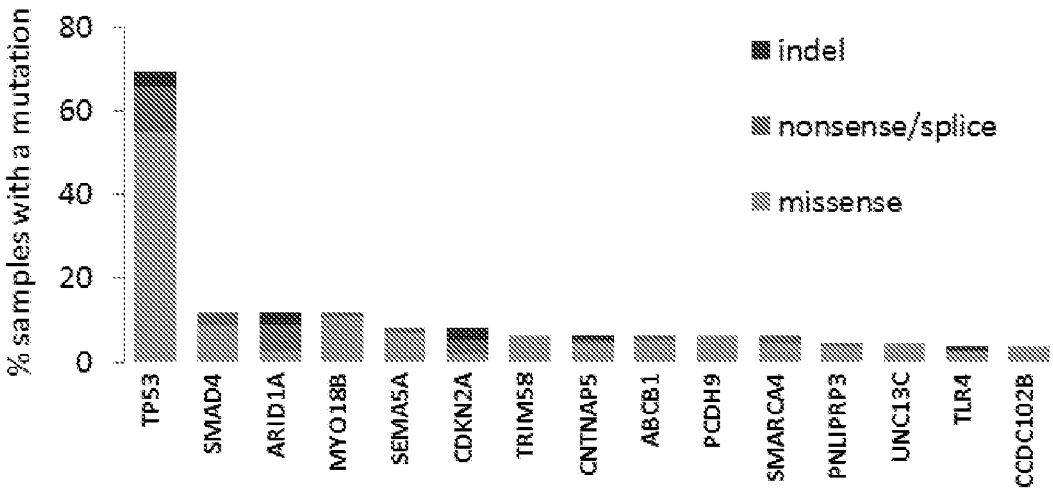


| TP53 | SMAD4 | ARID1A | MYO18B | SEMA5A | CDKN2A | TRIM58 | CNTNAP5 | ABCB1 | PCDH9 | SMARCA4 | PNLIPRP3 | UNC13C | TLR4 | CCDC102B | |
|---|---|---|---|---|---|---|---|---|---|---|---|---|---|---|---|
| **77** | 10 | 11 | 10 | 6 | 8 | 7 | 5 | 5 | 5 | 5 | 3 | 4 | 3 | 3 | **TP53** |
| | **13** | 5 | 1 | 1 | 1 | 0 | 1 | 1 | 0 | 0 | 1 | 1 | 0 | 0 | **SMAD4** |
| | | **13** | 1 | 0 | 0 | 1 | 0 | 2 | 1 | 0 | 0 | 1 | 2 | 1 | **ARID1A** |
| | | | **13** | 1 | 0 | 2 | 1 | 1 | 0 | 3 | 0 | 2 | 0 | 1 | **MYO18B** |
| | | | | **9** | 1 | 1 | 1 | 5 | 1 | 0 | 1 | 1 | 0 | 0 | **SEMA5A** |
| | | | | | **9** | 0 | 0 | 0 | 1 | 0 | 0 | 1 | 0 | 0 | **CDKN2A** |
| | | | | | | **7** | 0 | 0 | 0 | 2 | 1 | 1 | 1 | 1 | **TRIM58** |
| | | | | | | | **7** | 1 | 0 | 0 | 0 | 0 | 0 | 1 | **CNTNAP5** |
| | | | | | | | | **7** | 2 | 0 | 0 | 0 | 1 | 1 | **ABCB1** |
| | | | | | | | | | **7** | 0 | 0 | 0 | 1 | 1 | **PCDH9** |
| | | | | | | | | | | **7** | 0 | 1 | 0 | 0 | **SMARCA4** |
| | | | | | | | | | | | **5** | 0 | 0 | 0 | **PNLIPRP3** |
| | | | | | | | | | | | | **5** | 0 | 0 | **UNC13C** |
| | | | | | | | | | | | | | **4** | 1 | **TLR4** |
| | | | | | | | | | | | | | | **4** | **CCDC102B** |

Figure 9

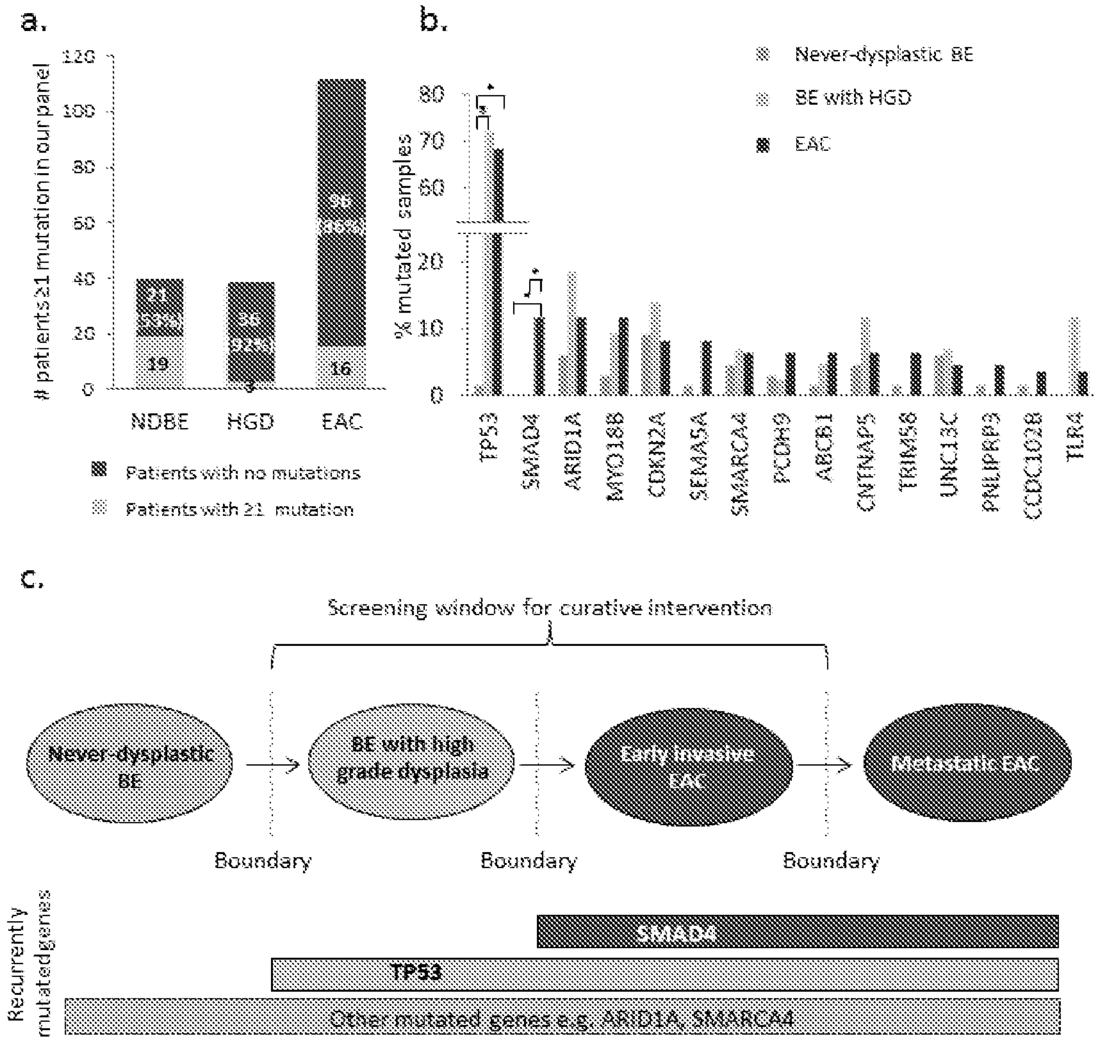
a.
# patients ≥1 mutation in our panel
120
100
80
60
40
20
0
21
(53%)
19
36
(92%)
3
96
(86%)
16
NDBE
HGD
EAC
Patients with no mutations
Patients with ≥1 mutation
b.
Never-dysplastic BE
BE with HGD
EAC
% mutated samples
80
70
60
20
10
0
TP53
SMAD4
ARID1A
MYO18B
CDKN2A
SEMA5A
SMARCA4
PCDH9
ABCB1
CNTNAP5
TRIM58
UNC13C
PNLIPRP3
CCDC102B
TLR4
c.
Screening window for curative intervention
Never-dysplastic BE
BE with high grade dysplasia
Early invasive EAC
Metastatic EAC
Boundary
Boundary
Boundary
Recurrently mutated genes
SMAD4
TP53
Other mutated genes e.g. ARID1A, SMARCA4

Figure 10

a.

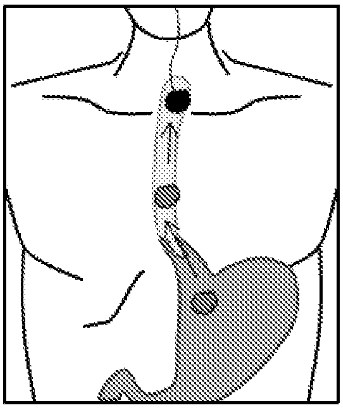

b.

| Patient | Mutation | AF on Biopsy #1 | AF on Biopsy #2 | AF on Cytosponge™ #1 | AF on Cytosponge™ #2 |
|---|---|---|---|---|---|
| HGD_01 | Chr17: 7574003 G>A | 0.35 | N/A | 0.04 | N/A |
| HGD_40 | Chr17: 7577538 C>T | 0.23 | 0.52 | 0.10 | N/A |
| HGD_03 | Chr17: 7578406 C>T | 0.51 | 0.72 | 0.06 | N/A |
| HGD_04 | Chr17: 7577551 C>T | 0.19 | N/A | 0.14 | 0.24 |

c.

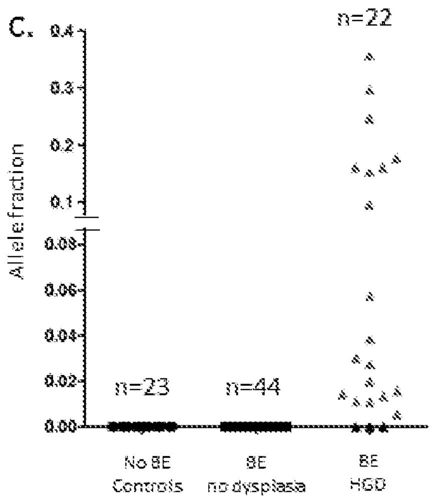


d.

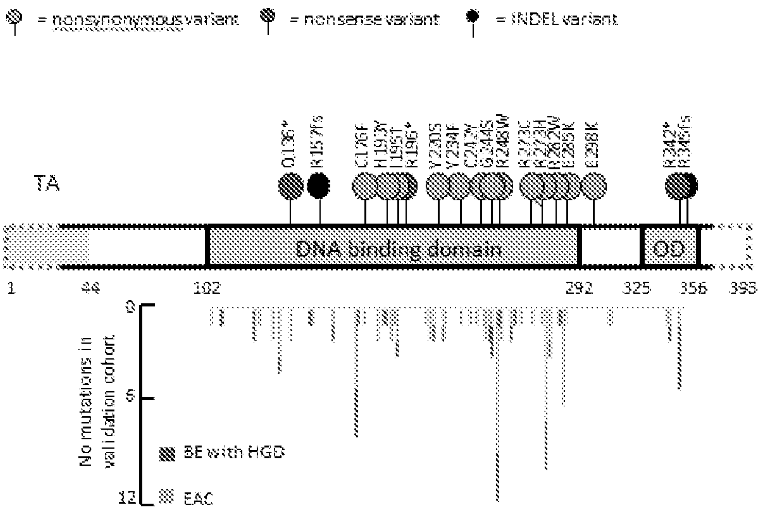

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011058316 A
- US 20120009597 A


### Non-patent literature cited in the description


- **RUGGE et al.** *Human Pathology,* 2010, vol. 41, 1380-1386
- **LIU et al.** *World Journal of Gastroenterology,* 2008, vol. 14, 7199-7207
- **AGNESE et al.** *European Society for Medical Oncology,* 2007, vol. 8 (6), vi110-vi115
- **SCHLEMPER et al.** *Gut,* 2000, vol. 47, 251-255
- **WINIFRED GRAY.** Diagnostic Cytopathology
- **CECILIA M. FENOGLIO-PREISER ; AMY E. NOFFSINGER ; GRANT N. STEMMERMANN ; PATRICK E. LANTZ ; PETER G. ISAACSON.** Gastrointestinal Pathology An Atlas and Textbook
- **DEVEREUX et al.** Nucleic Acids Research. University of Wisconsin, 1984, vol. 12, 387
- **ALTSCHUL et al.** *J. Mol. Biol.,* 1990, vol. 215, 403-410
- **LI, H. ; DURBIN, R.** Fast and accurate short read alignment with Burrows-Wheeler transform. *Bioinformatics,* 2009, vol. 25, 1754-60
- **LARSON, D.E. et al.** SomaticSniper: identification of somatic point mutations in whole genome sequencing data. *Bioinformatics,* 2012, vol. 28, 311-7
- **KOBOLDT, D.C. et al.** VarScan 2: somatic mutation and copy number alteration discovery in cancer by exome sequencing. *Genome Res,* 2012, vol. 22, 568-76
- **LI, H. et al.** The Sequence Alignment/Map format and SAMtools. *Bioinformatics,* 2009, vol. 25, 2078-9
- **YE, K. ; SCHULZ, M.H. ; LONG, Q. ; APWEILER, R. ; NING, Z.** Pindel: a pattern growth approach to detect break points of large deletions and medium sized insertions from paired-end short reads. *Bioinformatics,* 2009, vol. 25, 2865-71
- **SHAH, S.P. et al.** The clonal and mutational evolution spectrum of primary triple-negative breast cancers. *Nature,* 2012, vol. 486, 395-9
- **FORSHEW, T. et al.** Noninvasive identification and monitoring of cancer mutations by targeted deep sequencing of plasma DNA. *Sci Transl Med,* 2012, vol. 4, 136ra68
- **CHIN, L. ; ANDERSEN, J.N. ; FUTREAL, P.A.** Cancer genomics: from discovery science to personalized medicine. *Nat Med,* 2011, vol. 17, 297-303
- **GERLINGER, M. et al.** Intratumor heterogeneity and branched evolution revealed by multiregion sequencing. *N Engl J Med,* 2012, vol. 366, 883-92
- **JONES, S. et al.** Comparative lesion sequencing provides insights into tumor evolution. *Proc Natl Acad Sci U S A,* 2008, vol. 105, 4283-8
- **NIK-ZAINAL, S. et al.** The life history of 21 breast cancers. *Cell,* 2012, vol. 149, 994-1007
- **VOGELSTEIN, B. et al.** Genetic alterations during colorectal-tumor development. *N Engl J Med,* 1988, vol. 319, 525-32
- **GOH, X.Y. et al.** Integrative analysis of array-comparative genomic hybridisation and matched gene expression profiling data reveals novel genes with prognostic significance in oesophageal adenocarcinoma. *Gut,* 2011, vol. 60, 1317-26
- **QUANTE, M. et al.** Bile acid and inflammation activate gastric cardia stem cells in a mouse model of Barrett-like metaplasia. *Cancer Cell,* 2012, vol. 21, 36-51
- **GREAVES, M. ; MALEY, C.C.** Clonal evolution in cancer. *Nature,* 2012, vol. 481, 306-13
- **VARGHESE, S. ; LAO-SIRIEIX, P. ; FITZGERALD, R.C.** Identification and clinical implementation of biomarkers for Barrett's esophagus. *Gastroenterology,* 2012, vol. 142 (e2), 435-441
- **DULAK, A.M. et al.** Gastrointestinal Adenocarcinomas of the Esophagus, Stomach, and Colon Exhibit Distinct Patterns of Genome Instability and Oncogenesis. *Cancer Res,* 2012
- **DULAK, A.M. et al.** Exome and whole-genome sequencing of esophageal adenocarcinoma identifies recurrent driver events and mutational complexity. *Nat Genet,* 2013, vol. 45, 478-86
- **AGRAWAL, N. et al.** Comparative genomic analysis of esophageal adenocarcinoma and squamous cell carcinoma. *Cancer Discov,* 2012

- **CORLEY, D.A. et al.** Impact of Endoscopic Surveillance on Mortality From Barrett's Esophagus-Associated Esophageal Adenocarcinomas. *Gastroenterology,* 2013, vol. 145 (e1), 312-319
- **SHAHEEN, N.J. ; HUR, C. GARLIC.** Silver Bullets, and Surveillance Upper Endoscopy for Barrett's Esophagus. *Gastroenterology,* 2013, vol. 145, 273-6
- **HAYES, D.F. et al.** Breaking a vicious cycle. *Sci Transl Med,* 2013, vol. 5, 196cm6
- **NIK-ZAINAL, S. et al.** Mutational processes molding the genomes of 21 breast cancers. *Cell,* 2012, vol. 149, 979-93
- **FUJIMOTO, A. et al.** Whole-genome sequencing of liver cancers identifies etiological influences on mutation patterns and recurrent mutations in chromatin regulators. *Nat Genet,* 2012, vol. 44, 760-4
- **BASS, A.J. et al.** Genomic sequencing of colorectal adenocarcinomas identifies a recurrent VTI1A-TCF7L2 fusion. *Nat Genet,* 2011, vol. 43, 964-8
- **STREPPEL, M.M. et al.** Next-generation sequencing of endoscopic biopsies identifies ARID1A as a tumor-suppressor gene in Barrett's esophagus. *Oncogene,* 2013
- **CURVERS, W.L. et al.** Low-grade dysplasia in Barrett's esophagus: overdiagnosed and underestimated. *Am J Gastroenterol,* 2010, vol. 105, 1523-30
- **WANG, K. et al.** Exome sequencing identifies frequent mutation of ARID1A in molecular subtypes of gastric cancer. *Nat Genet,* 2011, vol. 43, 1219-23
- **JONES, S. et al.** Frequent mutations of chromatin remodeling gene ARID1A in ovarian clear cell carcinoma. *Science,* 2010, vol. 330, 228-31
- **REID, B.J. ; LI, X. ; GALIPEAU, P.C. ; VAUGHAN, T.L.** Barrett's oesophagus and oesophageal adenocarcinoma: time for a new synthesis. *Nat Rev Cancer,* 2010, vol. 10, 87-101
- **KADRI, S.R. et al.** Acceptability and accuracy of a non-endoscopic screening test for Barrett's oesophagus in primary care: cohort study. *BMJ,* 2010, vol. 341, c4372
- **LAO-SIRIEIX, P. et al.** Non-endoscopic screening biomarkers for Barrett's oesophagus: from microarray analysis to the clinic. *Gut,* 2009, vol. 58, 1451-9
- **DAWSON, S.J. et al.** Analysis of circulating tumor DNA to monitor metastatic breast cancer. *N Engl J Med,* 2013, vol. 368, 1199-209
- **THEISEN, J. et al.** Preoperative chemotherapy unmasks underlying Barrett's mucosa in patients with adenocarcinoma of the distal esophagus. *Surg Endosc,* 2002, vol. 16, 671-3
- **BHAT, S. et al.** Risk of malignant progression in Barrett's esophagus patients: results from a large population-based study. *J Natl Cancer Inst,* 2011, vol. 103, 1049-57
- **NOWELL, P.C.** The clonal evolution of tumor cell populations. *Science,* 1976, vol. 194, 23-8
- **MUTTER, G.L. et al.** Molecular identification of latent precancers in histologically normal endometrium. *Cancer Res,* 2001, vol. 61, 4311-4
- **KINDE, I. et al.** Evaluation of DNA from the Papanicolaou test to detect ovarian and endometrial cancers. *Sci Transl Med,* 2013, vol. 5, 167ra4
- **MALEY, C.C. et al.** Genetic clonal diversity predicts progression to esophageal adenocarcinoma. *Nat Genet,* 2006, vol. 38, 468-73
- **BIAN, Y.S. ; OSTERHELD, M.C. ; BOSMAN, F.T. ; BENHATTAR, J. ; FONTOLLIET, C.** p53 gene mutation and protein accumulation during neoplastic progression in Barrett's esophagus. *Mod Pathol,* 2001, vol. 14, 397-403
- **EADS, C.A. ; DANENBERG, K.D. ; KAWAKAMI, K. ; SALTZ, L.B. ; BLAKE, C. ; SHIBATA, D. ; DANENBERG, P.V. ; LAIRD, P.W.** MethyLight: a high-throughput assay to measure DNA methylation. *Nucleic Acids Res,* 2000, vol. 28, E32
- **EADS, C.A. ; LORD, R.V. ; WICKRAMASINGHE, K. ; LONG, T.I. ; KURUMBOOR, S.K. ; BERNSTEIN, L. ; PETERS, J.H. ; DEMEESTER, S.R. ; DEMEESTER, T.R. ; SKINNER, K.A. et al.** Epigenetic patterns in the progression of esophageal adenocarcinoma. *Cancer Res,* 2001, vol. 61, 3410-3418
- **KADRI, S.R. ; LAO-SIRIEIX, P. ; O'DONOVAN, M. ; DEBIRAM, I. ; DAS, M. ; BLAZEBY, J.M. ; EMERY, J. ; BOUSSIOUTAS, A. ; MORRIS, H. ; WALTER, F.M. et al.** Acceptability and accuracy of a non-endoscopic screening test for Barrett's oesophagus in primary care: cohort study. *BMJ,* 2010, vol. 341, c4372
- **KASTELEIN, F. ; BIERMANN, K. ; STEYERBERG, E.W. ; VERHEIJ, J. ; KALISVAART, M. ; LOOIJENGA, L.H. ; STOOP, H.A. ; WALTER, L. ; KUIPERS, E.J. ; SPAANDER, M.C. et al.** Aberrant p53 protein expression is associated with an increased risk of neoplastic progression in patients with Barrett's oesophagus. *Gut,* 2012
- **KAYE, P.V. ; HAIDER, S.A. ; ILYAS, M. ; JAMES, P.D. ; SOOMRO, I. ; FAISAL, W. ; CATTON, J. ; PARSONS, S.L. ; RAGUNATH, K.** Barrett's dysplasia and the Vienna classification: reproducibility, prediction of progression and impact of consensus reporting and p53 immunohistochemistry. *Histopathology,* 2009, vol. 54, 699-712
- **KAYE, P.V. ; HAIDER, S.A. ; JAMES, P.D. ; SOOMRO, I. ; CATTON, J. ; PARSONS, S.L. ; RAGUNATH, K. ; ILYAS, M.** Novel staining pattern of p53 in Barrett's dysplasia--the absent pattern. *Histopathology,* 2010, vol. 57, 933-935

- **LAO-SIRIEIX, P. ; BRAIS, R. ; LOVAT, L. ; COLEMAN, N. ; FITZGERALD, R.C.** Cell cycle phase abnormalities do not account for disordered proliferation in Barrett's carcinogenesis. *Neoplasia,* 2004, vol. 6, 751-760
- **LAO-SIRIEIX, P. ; LOVAT, L. ; FITZGERALD, R.C.** Cyclin A immunocytology as a risk stratification tool for Barrett's esophagus surveillance. *Clin Cancer Res,* 2007, vol. 13, 659-665
- **MILLER, C.T. ; MOY, J.R. ; LIN, L. ; SCHIPPER, M. ; NORMOLLE, D. ; BRENNER, D.E. ; IANNETTONI, M.D. ; ORRINGER, M.B. ; BEER, D.G.** Gene amplification in esophageal adenocarcinomas and Barrett's with high-grade dysplasia. *Clin Cancer Res,* 2003, vol. 9, 4819-4825
- **RUGGE, M. ; FASSAN, M. ; ZANINOTTO, G. ; PIZZI, M. ; GIACOMELLI, L. ; BATTAGLIA, G. ; RIZZETTO, C. ; PARENTE, P. ; ANCONA, E.** Aurora kinase A in Barrett's carcinogenesis. *Hum Pathol,* 2010, vol. 41, 1380-1386
- **RYGIEL, A.M. ; MILANO, F. ; TEN KATE, F.J. ; SCHAAP, A. ; WANG, K.K. ; PEPPELENBOSCH, M.P. ; BERGMAN, J.J. ; KRISHNADATH, K.K.** Gains and amplifications of c-myc, EGFR, and 20.q13 loci in the no dysplasia-dysplasia-adenocarcinoma sequence of Barrett's esophagus. *Cancer Epidemiol Biomarkers Prev,* 2008, vol. 17, 1380-1385
- **SCHULMANN, K. ; STERIAN, A. ; BERKI, A. ; YIN, J. ; SATO, F. ; XU, Y. ; OLARU, A. ; WANG, S. ; MORI, Y. ; DEACU, E. et al.** Inactivation of p16, RUNX3, and HPP1 occurs early in Barrett's-associated neoplastic progression and predicts progression risk. *Oncogene,* 2005, vol. 24, 4138-4148
- **SIKKEMA, M. ; KERKHOF, M. ; STEYERBERG, E.W. ; KUSTERS, J.G. ; VAN STRIEN, P.M. ; LOOMAN, C.W. ; VAN DEKKEN, H. ; SIERSEMA, P.D. ; KUIPERS, E.J.** Aneuploidy and overexpression of Ki67 and p53 as markers for neoplastic progression in Barrett's esophagus: a case-control study. *Am J Gastroenterol,* 2009, vol. 104, 2673-2680
- **SKACEL, M. ; PETRAS, R.E. ; RYBICKI, L.A. ; GRAMLICH, T.L. ; RICHTER, J.E. ; FALK, G.W. ; GOLDBLUM, J.R.** p53 expression in low grade dysplasia in Barrett's esophagus: correlation with interobserver agreement and disease progression. *Am J Gastroenterol,* 2002, vol. 97, 2508-2513
- **ZHOU, H. ; KUANG, J. ; ZHONG, L. ; KUO, W.L. ; GRAY, J.W. ; SAHIN, A. ; BRINKLEY, B.R. ; SEN, S.** Tumour amplified kinase STK15/BTAK induces centrosome amplification, aneuploidy and transformation. *Nat Genet,* 1998, vol. 20, 189-193